# FASCICULE DE BREVET EUROPEEN

(11) **EP 0 350 403 B1**
(45) Date de publication et mention de la délivrance du brevet: **13.01.1993**
(21) Numéro de dépôt: 89401948.8
(22) Date de dépôt: 06.07.1989
(51) Int. Cl.: C07D 275/06, C07D 417/06, C07D 417/14, A61K 31/435, A61K 31/425, C07D 513/06

(54) **Dérivés de (aza)naphtalènesultame, leurs procédés de préparation et les médicaments les contenant**
(Aza)Naphtalensultam-Derivate, Verfahren zu deren Herstellung und Arzneimittel, die sie enthalten
(Aza)naphthalenesultam derivatives, process for their preparation and drugs containing same

(30) Priorité: 07.07.1988 FR 8809218; 20.02.1989 FR 8902167
(43) Date de publication de la demande: 10.01.1990
(73) Titulaire: RHONE-POULENC SANTE, 92160 Antony (FR)
(72) Inventeur: Comte, Marie-Thérèse, F-94550 Chevilly Larue (FR); Gueremy, Claude, F-78800 Houilles (FR); Malleron, Jean-Luc, F-91460 Marcoussis (FR); Mignani, Serge, F-93190 Livry Gargan (FR); Peyronel, Jean-François, F-91120 Palaiseau (FR); Truchon, Alain, F-94000 Créteil (FR)
(74) Mandataire: Savina, Jacques

(56) Documents cités:
- EP-A- 0 200 322
- US-A- 4 110 449

## Description

La présente invention concerne des dérivés de formule : 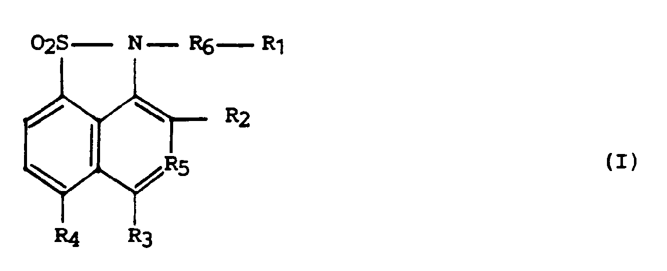 leurs sels, leurs procédés de préparation et les médicaments les contenant.

Dans la formule (I),
- R₁ représente
   . un radical tétrahydro-1,2,3,6 pyridyl-1 substitué en position
   . 4 par (a) un radical phényle, (b) un radical phényle substitué par un atome d'halogène ou un radical alkyle, hydroxy ou alcoxy, (c) un radical indolyl-3, (d) un radical indolyl-3 substitué sur l'atome d'azote par un radical alkyle ou alkylcarbonyle et/ou en position -5 par un atome de chlore ou de fluor ou (e) un radical (hydroxy-5 indolyl)-3,
   . un radical pipérazinyl-1 substitué en position -4 par (a) un radical phényle, (b) un radical phényle substitué par un radical alcoxy, alkyle, hydroxy, nitro, amino ou un atome d'halogène, (c) un radical benzisothiazol-1,2 yl-3, (d) un radical benzisoxazol-1,2 yl-3 ou (e) un radical pyridyl-2,
   . un radical pipéridino substitué en position -4 par (a) un radical phényle, (b) un radical phényle substitué par un atome d'halogène ou un radical hydroxy, alkyle ou alcoxy, (c) deux radicaux phényle, (d) un radical bis (fluoro-4 phényl) méthylène, (e) un radical fluoro-4 benzoyle, (f) un radical oxo-2 benzimidazolinyl-1, (g) un radical oxo-2 benzimidazolinyl-1 substitué en position -3 par un radical alkylcarbonyle ou benzoyle, (h) un radical hydroxy et un radical phényle éventuellement substitué par un radical alkyle, alcoxy, hydroxy ou un atome d'halogène, (i) un radical indolyl-3, (j) un radical indolyl-3 substitué sur l'atome d'azote par un radical alkyle ou alkylcarbonyle et/ou en position -5 par un atome de chlore ou de fluor ou (k) un radical (hydroxy-5 indolyl)-3,
- soit :
   . R₂ et R₃ identiques représentent un atome d'hydrogène ou d'halogène et R₄ représente un atome d'hydrogène ou
   . R₂ et R₄ représentent un atome d'hydrogène et R₃ représente un atome d'halogène ou un radical acétylamino ou
   . R₂ et R₃ représentent un atome d'hydrogène et R₄ représente un atome d'halogène
      et R₅ représente un groupe -CH=,
- soit R₂, R₃ et R₄ représentent un atome d'hydrogène et R₅ représente un atome d'azote, R₆ représente une chaîne alkylène contenant 2 à 4 atomes de carbone ou une chaîne propylène substituée en position -1 ou -3 par un radical alkyle ou en position -2 par un radical alkyle, alcoxy, hydroxy, dialkylamino, pipéridino, morpholino ou thiomorpholino, sous réserve que lorsque R₆ représente un radical propylène substitué en position -2 par un radical dialkylamino, pipéridino, morpholino ou thiomorpholino, R₁ ne peut pas être un radical comportant un radical hydroxy.

Dans les définitions qui précèdent et celles qui seront citées ci-après, les radicaux alkyle et alcoxy et les portions alkyle et alcoxy contiennent 1 à 4 atomes de carbone en chaîne droite ou ramifiée et les atomes d'halogène sont de préférence les atomes de chlore et de brome.

L'invention concerne également les sels des composés de formule (I) avec les acides minéraux ou organiques.

Les composés de formule (I) dans laquelle R₁, R₂, R₃, R₄ et R₅ ont les significations mentionnées précédemment sous réserve que R₁ ne peut pas être un radical aminophényl-4 pipérazinyl-1, et/ou R₆ ne peut pas être un radical propylène substitué en position -2 par un radical dialkylamino, pipéridino, morpholino ou thiomorpholino peuvent être préparés par action d'un dérivé halogéné de formule : 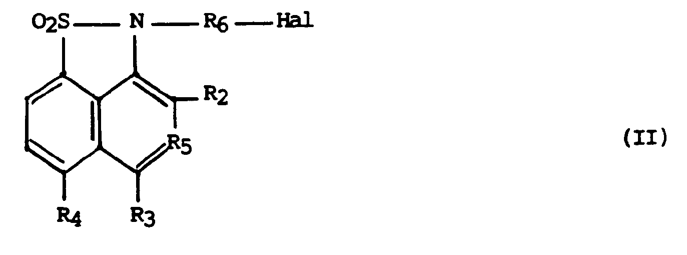 dans laquelle R₂, R₃, R₄, R₅ et R₆ ont les mêmes significations que ci-dessus et Hal représente un atome d'halogène sur un composé de formule : 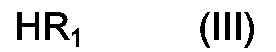 dans laquelle R₁ a les mêmes significations que dans la formule (I) sous réserve que R₁ ne peut pas être un radical aminophényl-4 pipérazinyl-1 ou un sel d'un tel composé avec un acide.

Cette réaction s'effectue généralement en présence d'une base telle qu'un carbonate de métal alcalin ou d'une base organique comme le diaza-1,8 bicyclo[5.4.0] undecène-7, le diaza-1,5 bicyclo [4.3.0] nonène-5 ou la triéthylamine éventuellement en présence d'iodure de sodium, dans un solvant organique tel que le benzène, le toluène, le diméthylformamide, l'acétonitrile, le tétrahydrofuranne ou l'acétone, à une température comprise entre 20°C et la température d'ébullition du solvant.

Les dérivés halogénés de formule (II) pour lesquels
- R₅ représente un groupe =CH- et
- soit R₂, R₃ et R₄ représentent un atome d'hydrogène,
- soit R₂ et R₃ représentent un atome d'halogène et R₄ représente un atome d'hydrogène,
- soit R₂ et R₄ représentent un atome d'hydrogène et R₃ représente un atome d'halogène,
- soit R₂ et R₃ représentent un atome d'hydrogène et R₄ représente un atome d'halogène,
- ou bien R₅ représente un atome d'azote et R₂, R₃ et R₄ représentent un atome d'hydrogène, et R₆ représente un radical alkylène (2-4 C) ou un radical propylène substitué en position -2 par un radical hydroxy, alkyle ou alcoxy peuvent être préparés par action d'un composé de formule : 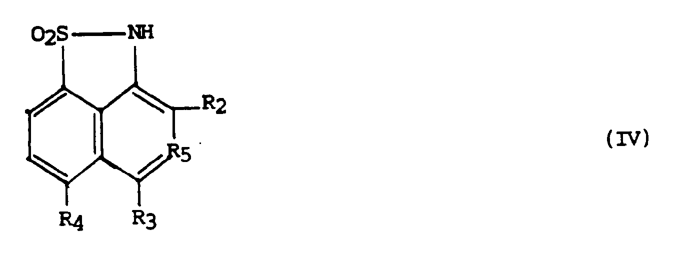 dans laquelle R₂, R₃, R₄ et R₅ ont les mêmes significations que précédemment, sur un composé de formule :
   X - Rₛ - Hal (V)
      dans laquelle R₆ représente un radical alkylène (2-4 C) ou propylène substitué en position -2 par un radical hydroxy, alkyle ou alcoxy, Hal et X représentent un atome d'halogène.

Cette réaction s'effectue, de préférence, en présence d'une base telle qu'un hydrure de métal alcalin, un hydroxyde de métal alcalin ou un carbonate de métal alcalin, dans un solvant inerte tel que le diméthylformamide ou le tétrahydrofuranne, à une température comprise entre 20°C et la température d'ébullition du solvant.

Les composés de formule (IV) pour lesquels R₅ représente un groupe =CH-, R₂ et R₄ représentent un atome d'hydrogène et R₃ représente un atome d'halogène, peuvent être préparés par application ou adaptation des méthodes décrites par A. MUSTAFA et coll., Arch. Pharm., 298, 741 (1965).

Les composés de formule (IV) pour lesquels R₅ représente un groupe =CH-, R₂ et R₃ représentent un atome d'hydrogène et R₄ représente un atome d'halogène, peuvent être préparés par cyclisation d'un composé de formule : 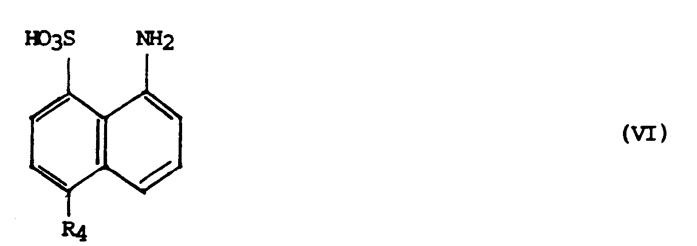 dans laquelle R₄ a les mêmes significations que précédemment.

Cette cyclisation s'effectue, par exemple, au moyen d'oxychlorure de phosphore, à la température d'ébullition du milieu réactionnel par adaptation de la méthode décrite par F. DANNERTH et coll., J. Am. Chem. Soc., 29, 1319 (1907).

Les composés de formule (VI) peuvent être obtenus par application ou adaptation de la méthode décrite par P. FRIEDLANDER et coll., Chem. Ber., 55B, 45 (1922).

Les composés de formule (IV) pour lesquels R₅ représente un groupe =CH-, R₂ et R₃ représentent un atome d'halogène et R₄ représente un atome d'hydrogène peuvent être préparés par application ou adaptation de la méthode décrite par Th. ZINCKE et coll., Liebigs Ann. Chem., 411, 195 (1916).

Le composé de formule (IV) pour lequel R₅ représente un atome d'azote peut être obtenu par cyclisation du bromo-4 isoquinolyl-5 sulfonamide.

Cette cyclisation s'effectue, de préférence, au moyen d'une base comme un hydrure de métal alcalin, dans un solvant organique inerte tel que le diméthylformamide ou le tétrahydrofuranne, à une température comprise entre 20°C et la température d'ébullition du solvant.

Le bromo-4 isoquinolyl-5 sulfonamide peut être préparé par action de l'ammoniac sur le chlorure de bromo-4 isoquinolyl-5 sulfonyle, de préférence, au sein du tétrahydrofuranne, à une température variant de -50°C à 20°C.

Le chlorure de bromo-4 isoquinolyl-5 sulfonyle peut être préparé par action de nitrite de sodium surl'amino-5 bromo-4 isoquinoléine en présence d'acide chlorhydrique à une température voisine de 0°C puis de dioxyde de soufre dans l'acide acétique en présence de chlorure cuivreux à une température voisine de 20°C.

L'amino-5 bromo-4 isoquinoléine peut être obtenue par réduction de la bromo-4 nitro-5 isoquinoléine. Cette réduction est effectuée, de préférence, au moyen de chlorure stanneux et d'acide chlorhydrique à la température d'ébullition du mélange réactionnel.

La bromo-4 nitro-5 isoquinoléine peut être préparée par application de la méthode décrite par M. D. NAIR et coll., Indian J. Chem. Soc., 5, 224 (1967).

Les dérivés de formule (V) pour lesquels Rr, représente un radical alkylène (2-4 C) ou propylène substitué en position -2 par un radical hydroxy sont commercialisés.

Les dérivés de formule (V) pour lesquels R₆ représente un radical propylène substitué en position -2 par un radical alkyle peuvent être obtenus par application ou adaptation de la méthode décrite par Y. HARAMOTO et coll., J. Chem. Soc. Chem. Comm., 75 (1983).

Les dérivés de formule (V) pour lesquels R₆ représente un radical propylène substitué en position -2 par un radical alcoxy peuvent être obtenus par application ou adaptation de la méthode décrite dans Can. J. Chem., vol 48, 975 (1970).

Les dérivés halogénés de formule (II) pour lesquels R₅ représente un groupe =CH-, R₂ et R₃ représentent un atome d'halogène et R₄ représente un atome d'hydrogène et ceux pour lesquels R₂ et R₄ représentent un atome d'hydrogène et R₃ représente un atome d'halogène et R₆ représente un radical alkylène (2-4 C), peuvent également être obtenus par halogénation d'un composé de formule (II) dans laquelle R₂, R₃ et R₄ représentent un atome d'hydrogène et R₅ représente un groupe =CH- puis séparation éventuelle du dérivé monohalogéné et du dérivé dihalogéné.

L'halogénation peut être effectuée par des méthodes connues telles celles décrites par F. DANNERTH et coll., J. Am. Chem. Soc., 29, 1319 (1907), Th. ZINCKE et coll., Ann. Chem., 411, 195 (1916), A. MUSTAFA et coll., Arch. Pharm, 298, 741 (1965).

Les dérivés halogénés de formule (II) pour lesquels R₂, R₃, R₄, et R₅ ont les mêmes significations que dans la formule (I), et R₆ représente un radical propylène substitué en position -1 ou -3 par un radical alkyle à l'exception de ceux pour lesquels R₃ représente un radical acétylamino peuvent être obtenus par halogénation d'un dérivé hydroxylé de formule : 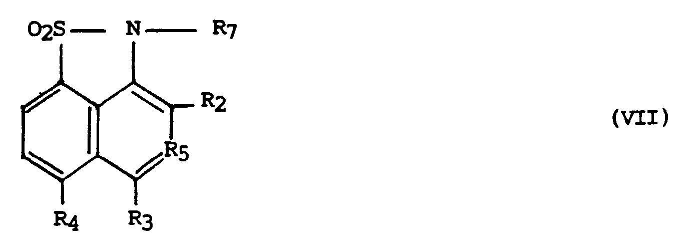 dans laquelle R₂, R₃, R₄ et R₅ ont les mêmes significations que ci-dessus et R₇ représente un radical propanol-3 substitué en position -1 ou -3 par un radical alkyle.

Cette halogénation peut être réalisée selon des procédés connus en soi tels que ceuxdécrits dans MARCH, Advanced Organic Chemistry, p. 382 (1985), Wiley Interscience.

De préférence, on effectue cette halogénation au moyen de tribromure ou trichlorure de phosphore, au sein d'un solvant inerte tel que le toluène, le benzène ou le xylène, à une température comprise entre 20°C et la température d'ébullition du solvant.

Les dérivés hydroxylés de formule (VII) peuvent être préparés par action d'un dérivé de formule (IV) dans laquelle R₂, R₃, R₄ et R₅ ont les mêmes significations que dans la formule (VII), sur un dérivé de formule :
Br - R₇ (VIII)

dans laquelle R₇ a les mêmes significations que dans la formule (VII).

Cette réaction s'effectue généralement en présence d'une base telle qu'un hydrure de métal alcalin, un hydroxyde de métal alcalin ou un carbonate de métal alcalin, dans un solvant inerte tel que l'acétonitrile, le diméthylformamide ou le tétrahydrofuranne, à une température comprise entre 20°C et la température d'ébullition du solvant.

Les dérivés de formule (VIII) pour lesquels R₇ représente un radical propanol-3 substitué en position -1 par un radical alkyle peuvent être préparés par application ou adaptation de la méthode décrite par D.A. PALAVANDISHVILI et coll., Chem. Abst., 70, 106089.

Les dérivés de formule (VIII) pour lesquels R₇ représente un radical propanol-3 substitué en position -3 par un radical alkyle peuvent être obtenus par application ou adaptation de la méthode décrite par F. SATO et coll., Chem. lett, 99 (1980).

Les dérivés halogénés de formule (II) pour lesquels R₂, R₃, R₄ et R₅ ont les mêmes significations que dans la formule (I), sous réserve que R₃ ne peut pas représenter un radical acétylamino, R₆ représente un radical propylène substitué en position -2 par un radical alcoxy peuvent également être obtenus par alkylation du dérivé de formule (II) correspondant pour lequel R₆ représente un radical propylène substitué en position -2 par un radical hydroxy.

Cette alkylation peut être réalisée selon des procédés connus en soi tels que ceux décrits dans MARCH, Advanced Organic Chemistry, 342 (1985), Wiley Interscience.

On peut, par exemple, faire réagir un halogénure d'alkyle au sein d'un solvant inerte tel que le diméthylformamide, ou le tétrahydrofuranne, en présence d'un hydrure de métal alcalin ou un carbonate de métal alcalin, à une température comprise entre 20°C et la température d'ébullition du solvant.

Les dérivés halogénés de formule (II) pour lesquels R₅ représente un groupe =CH-, R₂ et R₄ représentent un atome d'hydrogène, R₃ représente un radical acétylamino et R₆ a les mêmes significations que précédemment, peuvent être préparés par N-acétylation des amines correspondantes.

L'acétylation peut être effectuée au moyen d'anhydride acétique, en présence d'acétate de métal alcalin, à la température d'ébullition du milieu réactionnel.

Les amines correspondantes peuvent être obtenues par réduction d'un dérivé de formule : 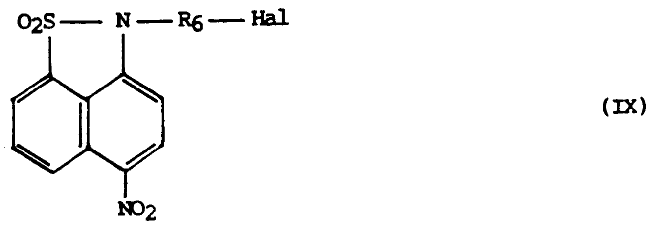 dans laquelle R₆ et Hal ont les mêmes significations que dans la formule (II).

Cette réduction s'effectue par exemple au moyen de chlorure de nickel et de borohydrure de sodium, dans un alcool tel que le méthanol ou l'éthanol, à une température voisine de 0°C.

Les dérivés nitrés de formule (IX) peuvent être obtenus par nitration d'un composé de formule (II) dans laquelle R₂, R₃ et R₄ représentent un atome d'hydrogène et R₅ représente un groupe =CH-.

Cette nitration s'effectue, généralement au moyen d'acide nitrique de préférence, à une température voisine de 0°C.

Les composés de formule (III) sont commercialisés ou peuvent être préparés par application ou adaptation des méthodes décrites par R.L. DUNCAN et coll., J. Med. Chem., 13, 1 (1970) ; D.K. YUNK et coll., J. Med. Chem., 21, 1301 (1978) ; L. NEDELEC et coll., Eur. J. Med. Chem., 22, 33 (1987) ; J.P. YEVICH et coll., J. Med. Chem., 29, 3, 359 (1986) ; L. THUNUS et coll., Ann. Pharm., 38, 353 (1980) ; L. GOOTES etcoll.,Arzneim. Forsch., 17, 1145 (1967) ; J. BERGMAN et coll., J. Het. Chem. 1071 (1970) et dans les brevets DE 2139084, BE 62630, EP 110435, US 4470989, 3575990 et des méthodes décrites dans les exemples.

Les hydroxyphényl-1 pipérazines peuvent être obtenues par déméthylation des méthoxyphényl-1 pipérazines correspondantes par toute méthode connue de l'homme de l'art et notamment au moyen d'acide bromhydrique.

Les composés de formule (I) dans laquelle
- soit R₅ représente un groupe =CH-, R₂, R₃ et R₄ représentent un atome d'hydrogène ou R₂ et R₄ représentent un atome d'hydrogène et R₃ représente un atome d'halogène ou R₂ et R₃ représentent un atome d'hydrogène et R₄ représente un atome d'halogène, ou R₂ et R₃ représentent un atome d'halogène et R₄ représente un atome d'hydrogène,
- soit R₅ représente un atome d'azote et R₂, R₃ et R₄ représentent un atome d'hydrogène,

R₆ représente un radical alkylène (2-4 C) ou propylène substitué en position -2 par un radical hydroxy, alkyle ou alcoxy, exceptés ceux pour lesquels R₁ est un radical aminophényl-4 pipérazinyl-1 peuvent également être préparés par action d'un composé de formule (IV) dans laquelle R₂, R₃, R₄ et R₅ ont les significations mentionnées précédemment sur un dérivé de formule :
Hal - Rₛ - R₁ (X)

dans laquelle R₆ et R₁ ont les mêmes significations que ci-dessus et Hal représente un atome d'halogène.

Cette réaction s'effectue généralement en présence d'une base telle qu'un hydrure de métal alcalin, un hydroxyde de métal alcalin ou un carbonate de métal alcalin, dans un solvant organique tel que le diméthylformamide ou le tétrahydrofuranne ou en présence d'un hydroxyde de métal alcalin en milieu aqueux ou hy- droorganique, à une température comprise entre 20°C et la température d'ébullition du solvant.

Les dérivés de formule (X) peuvent être préparés par action d'un dihalogénure d'alkyle de formule (V) sur un composé de formule (III).

Cette réaction s'effectue généralement en présence d'un carbonate de métal alcalin ou d'une amine tertiaire, dans un solvant organique tel que l'acétonitrile, à une température voisine de 20°C.

Les composés de formule (I) pour lesquels R₆ représente un radical propylène substitué en position -2 par un radical alcoxy, à l'exception de ceux pour lesquels R₁ représente un radical tétrahydro-1,2,3,6 pyridyl-1 substitué en position -4 par un radical indolyl-3, indolyl-3 substitué en position -5 par un radical hydroxy ou hydroxyphényle, pipérazinyl-1 substitué en position -4 par un radical amino ou hydroxyphényle ou pipéridino substitué en position -4 par un radical oxo-2 benzimidazolinyl-1, hydroxyphényle, hydroxy et phényle éventuellement substitué par un radical hydroxy ou indolyl-3 éventuellement substitué en position -5 par un radical hydroxy peuvent également être obtenus par alkylation des composés de formule (I) correspondants pour lesquels R₆ représente un radical propylène substitué en position -2 par un radical hydroxy.

Cette alkylation peut être effectuée dans les mêmes conditions que celles décrites précédemment pour l'alkylation des dérivés halogénés de formule (ll) pour lesquels R₆ représente un radical propylène substitué en position -2 par un radical hydroxy.

Les composés de formule (I) pour lesquels R₆ représente un radical propylène substitué en position -2 par un radical dialkylamino, pipéridino, morpholino ou thiomorpholino peuvent être obtenus par action d'un dérivé bromé de formule : 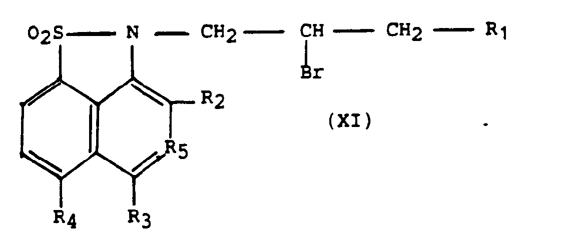 dans laquelle R₂, R₃, R₄, R₅ et R₁ ont les mêmes significations que dans la formule (I) sur un dérivé de formule :
H - R₈ (Xll)

dans laquelle Rₛ représente un radical dialkylamino, pipéridino, morpholino ou thiomorpholino.

Cette réaction s'effectue généralement dans un solvant inerte tel que le benzène, le toluène, le xylène, en présence d'une amine tertiaire comme la triéthylamine, dans un autoclave et à une température variant de 20°C à la température d'ébullition du solvant.

Les dérivés bromés de formule (XI) peuvent être obtenus par bromuration d'un dérivé de formule (I) correspondant pour lequel R₇ représente un radical propylène substitué en position -2 par un radical hydroxy.

Cette bromuration est effectuée dans les mêmes conditions que celles décrites précédemment pour la bromuration des composés de formule (VII).

Les composés de formule (I) pour lesquels R₆ représente un radical propylène substitué en position -3 par un radical méthyle, à l'exception de ceux pour lesquels R₃ représente un radical acétylamino et/ou R₁ représente un substituant comportant un radical hydroxy, peuvent également être obtenus par action d'un dérivé de formule (IV) sur un dérivé de formule : 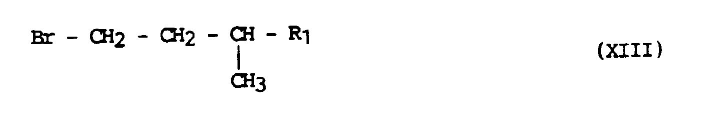 dans laquelle R₁ a les mêmes significations que ci-dessus.

Cette réaction s'effectue généralement dans les mêmes conditions que celles mentionnées précédemment pour la réaction des composés de formule (IV) et (VII).

Les dérivés de formule (XIII) peuvent être obtenus par bromuration d'un dérivé de formule : 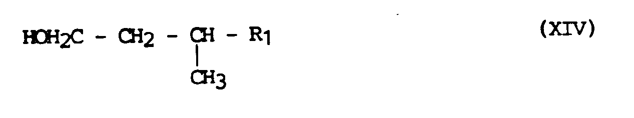 dans laquelle R₁ a les mêmes significations que ci-dessus.

Cette bromuration s'effectue, de préférence, dans les mêmes conditions que celles décrites précédemment pour la bromuration des composés de formule (VII).

Les dérivés de formule (XIV) peuvent être obtenus par action de bromo-3 butanol-1 sur un dérivé de formule (III).

Cette réaction s'effectue, généralement, dans les conditions décrites précédemment pour la réaction des dérivés de formules (II) et (III).

Les composés de formule (I) pour lesquels R₁ représente un radical tétrahydro -1,2,3,6 pyridyl-1 substitué en position -4 par un radical indolyl-3 substitué sur l'atome d'azote par un radical alkyle ou alkylcarbonyle ou un radical pipéridino substitué en position -4 par un radical oxo-2 benzimidazolinyl-1 substitué en position -3 par un radical alkylcarbonyle ou benzoyle ou un radical indolyl-3 substitué sur l'atome d'azote par un radical alkyle ou alkylcarbonyle à l'exception de ceux pour lesquels R₆ représente un radical propylène substitué en position -2 par un radical hydroxy peuvent être obtenus par action d'un dérivé de formule : 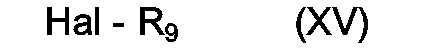 dans laquelle Hal représente un atome d'halogène et R₉ représente un radical alkyle, alkylcarbonyle ou benzoyle, sur les dérivés de formule (I) correspondants pour lesquels R₁ représente un radical tétrahydro-1,2,3,6 pyridyl-1 substitué en position -4 par un radical indolyl-3 ou un radical pipéridino substitué en position -4 par un radical oxo-2 benzimidazolinyl-1 ou indolyl-3.

Cette réaction s'effectue de préférence dans un solvant inerte tel que le diméthylformamide ou le tétrahydrofuranne, à une température comprise entre 20°C et la température d'ébullition du solvant.

Les composés de formule (I) dans laquelle R₁ représente un radical tétrahydro-1,2,3,6 pyridyl-1 substitué en position -4 par un radical phényle substitué par un atome d'halogène peuvent également être obtenus par déshydratation d'un composé de formule (I) correspondant pour lequel R₁ représente un radical pipéridino substitué en position -4 par un radical hydroxy et un radical phényle substitué par un atome d'halogène.

Cette déshydratation peut être effectuée par des méthodes connues telles celles décrites dans MARCH, Advanced organic Chemistry, 901 (1985) et notamment au moyen d'acide sulfurique, d'un mélange acide acétique - acide bromhydrique, acide acétique - acide chlorhydrique à une température comprise entre 20°C et la température d'ébullition du milieu réactionnel.

Les composés de formule (I) pour lesquels R₁ représente un radical aminophényl-4 pipérazinyl-1 peuvent être obtenus par réduction des composés de formule (I) correspondants pour lesquels R₁ représente un radical nitrophényl-4 pipérazinyl-1.

Cette réduction s'effectue généralement au moyen de chlorure stanneux et de borohydrure de sodium dans un alcool tel que le méthanol ou l'éthanol, en présence d'eau, à une température comprise entre 20°C et 70°C ou au moyen de fer et d'acide chlorhydrique dans l'eau ou un mélange eau-alcool, à une température comprise entre 20°C et la température d'ébullition du milieu réactionnel.

Les composés de formule (I) peuvent être purifiés par les méthodes connues habituelles telles que la cristallisation ou la chromatographie.

Les composés de formule (l), sous forme de base libre, peuvent être transformés en sel d'addition avec les acides par action d'un acide dans un solvant organique tel qu'un alcool, une cétone, un solvant chloré ou un éther.

Les composés de formule (I) et leurs sels présentent des propriétés intéressantes. Ces composés possèdent des propriétés antagonistes de la sérotonine (récepteurs 5HT₂) et sont donc utiles pour le traitement des affections où la sérotonine est impliquée et notamment les affections du système nerveux central, du système cardiovasculaire et les troubles gastrointestinaux.

Ces composés sont, en particulier, utiles pour le traitement de l'anxiété, des troubles du sommeil, de la dépression, des psychoses et notamment de la schizophrénie, de la migraine, de l'asthme, de l'hypertension et de l'urticaire, comme analgésiques et comme inhibiteurs de l'agrégation plaquettaire.

L'affinité des composés de formule (I) pour les sites récepteurs centraux à sérotonine (type S₂) a été déterminée selon une technique inspirée de celle de J. E. LEYSEN et al., Mol. Pharmacol., 21, 301 (1982) qui consiste à mesurer l'affinité des produits pour les sites de liaison de la kétansérine tritiée. Dans ce test, la Cl₅₀ des composés de formule (I) est généralement inférieure à 25 nM.

Les composés de formule (I) présentent une toxicité faible. Ils sont généralement atoxiques à 300 mg/kg par voie orale chez la souris en administration unique.

Sont particulièrement intéressants, les composés de formule (I) dans laquelle R₁ représente un radical tétrahydro-1,2,3,6 pyridyl-1 substitué en position -4 par un radical halogénophényle, phényle ou indolyl-3 substitué sur l'atome d'azote par un radical alkyle ou alkylcarbonyle, un radical pipérazinyl-1 substitué en position -4 par un radical pyridyl-2, benzisothiazol-1,2 yl-3 ou phényle substitué par un atome d'halogène ou un radical hydroxy, amino ou alkyle ou un radical pipéridino substitué en position -4 par un radical phényle ou N-alkylindolyl-3.

Sont d'un intérêt particulier, les produits suivants :
- [((benzisoxazol-1,2 yl-3)-4 pipérazinyl-1)-3 propyl]-2 naphto [1,8 - cd] isothiazole dioxyde-1,1
- [((amino-4 phényl)-4 pipérazinyl-1)-3 propyl]-2 naphto [1,8 - cd] isothiazole dioxyde-1,1
- [((fluoro-3 phényl)-4 pipérazinyl-1)-3 propyl]-2 naphto [1,8 - cd] isothiazole dioxyde-1,1
- [((méthyl-4 phényl)-4 pipérazinyl-1)-3 propyl]-2 naphto [1,8 - cd] isothiazole dioxyde-1,1
- [((hydroxy-3 phényl)-4 pipérazinyl-1)-3 propyl]-2 naphto [1,8 -cd] isothiazole dioxyde-1,1
- [((méthyl-1 indolyl-3)-4 tétrahydro-1,2,3,6 pyridyl-1)-3 propyl]-2 naphto [1,8- cd] isothiazole dioxyde-1,1
- [((acétyl-1 indolyl-3)-4 tétrahydro-1,2,3,6 pyridyl-1)-3 propyl]-2 naphto [1,8 - cd] isothiazole dioxyde-1,1
- [((hydroxy-4 phényl)-4 pipérazinyl-1)-2 éthyl]-2 naphto [1,8 - cd] isothiazole dioxyde-1,1
- [((fluoro-4 phényl)-4 pipérazinyl-1)-3 propyl]-2 naphto[1,8-cd] isothiazole dioxyde-1,1
- [(phényl-4 tétrahydro-1,2,3,6 pyridyl-1)-3 propyl]-2 naphto [1,8-cd] isothiazole dioxyde-1,1
- [((fluoro-4 phényl)-4 tétrahydro-1,2,3,6 pyridyl-1)-3 propyl]-2 naphto[1,8-cd] isothiazole dioxyde-1,1
- [((pyridyl-2)-4 pipérazinyl-1)-3 propyl]-2 naphto[1,8-cd] isothiazole dioxyde-1,1
- [(phényl-4 pipéridino-1)-3 propyl]-2 naphto[1,8-cd] isothiazole dioxyde-1,1
- [((hydroxy-4 phényl)-4 pipérazinyl-1)-3 propyl]-2 naphto[1,8-cd] isothiazole dioxyde-1,1
- [((fluoro-4 phényl)-4 pipérazinyl-1)-3 hydroxy-2 propyl]-2 naphto [1,8-cd] isothiazole dioxyde-1,1
- [((fluoro-4 phényl)-4 pipérazinyl-1)-3 méthoxy-2 propyl]-2 naphto [1,8-cd] isothiazole dioxyde-1,1
- [diméthylamino-2 ((fluoro-4 phényl)-4 pipérazinyl-1)-3 propyl]-2 naphto [1,8-cd] isothiazole dioxyde-1,1
- [((fluoro-4 phényl)-4 pipérazinyl-1)-3 méthyl-2 propyl]-2 naphto [1,8-cd] isothiazole dioxyde-1,1
- [((fluoro-4 phényl)-4 pipérazinyl-1)-3 butyl]-2 naphto [1,8-cd] isothiazole dioxyde-1,1
- [((fluoro-4 phényl)-4 pipérazinyl-1)-4 butyl-2]-2 naphto [1,8-cd] isothiazole dioxyde-1,1.

Pour l'emploi thérapeutique, il peut être fait usage des composés de formule (I) tels quels ou à l'état de sels pharmaceutiquement acceptables.

Comme sels pharmaceutiquement acceptables, peuvent être notamment cités les sels d'addition avec les acides minéraux tels que chlorhydrates, sulfates, nitrates, phosphates, ou organiques tels que acétates, pro- pionates, succinates, benzoates, fumarates, maléates, méthanesulfonates, isothionates, théophillineacétates, salicylates, phénolphtalinates, méthylène-bis-β-oxynaphtoates ou des dérivés de substitution de ces dérivés.

Les exemples suivants donnés à titre non limitatif, montrent comment l'invention peut être mise en pratique.

### EXEMPLE 1

28,1 g de (chloro-3 propyl)-2 naphto[1,8-cd] isothiazole dioxyde-1,1, 14 cm3 de triéthylamine et 18,8 g de (fluoro-4 phényl)-1 pipérazine dans 300 cm3 de toluène sont chauffés pendant 8 heures à ébullition. Le mélange est ensuite refroidi à une température voisine de 20°C et l'agitation est maintenue durant 15 heures à cette température. Le précipité formé est séparé par filtration et lavé par 2 fois 50 cm3 de toluène. Le filtrat est évaporé à sec à 40°C sous pression réduite (20 mm de mercure ; 2,7 kPa). Le résidu obtenu est purifié par flash-chromatographie sur colonne de silice, sous courant d'argon à moyenne pression (0,1-1,5 bar) avec de l'acétate d'éthyle comme éluant et recristallisé dans 150 cm3 d'acétonitrile bouillant. On obtient 37,8 g de [((fluoro-4 phényl)-4 pipérazinyl-1)-3 propyl]-2 naphto [1,8-cd] isothiazole dioxyde-1,1 fondant à 95-97°C .

Le (chloro-3 propyl)-2 naphto[1,8-cd] isothiazole dioxyde-1,1 peut être préparé de la manière suivante : à 40,2 g d'hydrure de sodium en dispersion à 50 % dans de l'huile de vaseline, sous courant d'argon, on coule, en 3 heures et 30 minutes, une solution de 175 g de naphtosultam-1,8 dans 2100 cm3 de diméthylformamide, en maintenant la température entre 20°C et 30°C. Le milieu réactionnel est agité 1 heure à une température voisine de 20°C puis on ajoute, en 10 minutes, 83 cm3 de bromo-1 chloro-3 propane. L'agitation est maintenue durant 15 heures à une température voisine de 20°C. Le mélange réactionnel est concentré à sec à 50°C sous pression réduite (0,5 mm de mercure ; 0,07 kPa). Le résidu est purifié par chromatographie sur colonne de silice avec un mélange de dichlorométhane et de cyclohexane (60-40 en volumes) comme éluant. On obtient 165,7g de (chloro-3-propyl)-2 naphto [1,8-cd] isothiazole dioxyde-1,1 fondant à 78°C.

### EXEMPLE 2

On opère comme à l'exemple 1, à partir de 2,8 g de (chloro-3 propyl)-2 naphto[1,8-cd] isothiazole dioxyde-1,1, de 1,4 cm3 de triéthylamine et de 1,6 g de phényl-4 tétrahydro-1,2,3,6 pyridine dans 30 cm3 de toluène. Le mélange est chauffé 8 heures à ébullition puis refroidi à une température voisine de 20°C. L'agitation est maintenue durant 15 heures à cette température. Après purification parflash-chromatographie sur colonne de silice, sous courant d'argon à moyenne pression (0,5-1,5 bar) avec de l'acétate d'éthyle comme éluant et recristallisation dans 20 cm3 d'isopranol bouillant, on obtient 1,5 g de [(phényl-4 tétrahydro-1,2,3,6 pyridyl-1)-3 propyl]-2 naphto[1,8-cd] isothiazole dioxyde-1,1 fondant à 88°C.

### EXEMPLE 3

On opère comme à l'exemple 1, à partir de 100 g de (chloro-3 propyl)-2 naphto[1,8-cd] isothiazole dioxyde-1,1, de 50 cm3 de triéthylamine et de 77,9 g de (fluoro-4 phényl)-4 tétrahydro-1,2,3,6 pyridine dans 950 cm3 de toluène. Le mélange est chauffé 7 heures à ébullition puis refroidi à une température voisine de 20°C. L'agitation est maintenue durant 8 heures à cette température. Après purification parflash-chromatographie sur colonne de silice, sous courant d'argon à moyenne pression (0,5-1,5 bar) avec de l'acétate d'éthyle comme éluant, on obtient 105,4 g de [((fluoro-4 phényl)-4 tétrahydro-1,2,3,6 pyridyl-1)-3 propyl]-2 naphto[1,8-cd] isothiazole dioxyde-1,1 sous forme d'une huile brune [Rf=0,8; chromatographie sur couche mince de gel de silice ; éluant : acétate d'éthyle]. Cette huile est transformée en chlorhydrate fondant à 224°C.

### EXEMPLE 4

On opère comme à l'exemple 1, à partir de 8,5 g de (chloro-3 propyl)-2 naphto[1,8-cd] isothiazole dioxyde-1,1, de 4,2 cm3 de triéthylamine et de 5,7 g de (chloro-4 phényl)-4 tétrahydro-1,2,3,6 pyridine dans 80 cm3 de toluène. Le mélange est chauffé 8 heures à ébullition puis refroidi à une température voisine de 20°C. L'agitation est maintenue durant 8 heures à cette température. Après purification parflash-chromatographie surco- lonne de silice, sous courant d'argon à moyenne pression (0,5-1,5 bar) avec de l'acétate d'éthyle comme éluant et recristallisation dans 50 cm3 d'acétonitrile bouillant, on obtient 4,9 g de [((chloro-4 phényl)-4 tétrahydro-1,2,3,6 pyridyl-1)-3 propyl]-2 naphto[1,8-cd] isothiazole dioxyde-1,1 fondant à 111°C.

### EXEMPLE 5

On opère comme à l'exemple 1, à partir de 8,5 g de (chloro-3 propyl)-2 naphto[1,8-cd] isothiazole dioxyde-1,1, de 4,2 cm3 de triéthylamine et de 4,7 cm3 de (pyridyl-2)-1 pipérazine dans 80 cm3 de toluène. Le mélange est chauffé 2 heures à ébullition puis refroidi à une température voisine de 20°C. Après purification parflash-chromatographie sur colonne de silice, sous courant d'argon à moyenne pression (0,5-1,5 bar) avec de l'acétate d'éthyle comme éluant et recristallisation dans 30 cm3 d'acétonitrile bouillant, on obtient4,2 g de [((pyridyl-2)-4 pipérazinyl-1)-3 propyl]-2 naphto[1,8-cd] isothiazole dioxyde-1,1 fondant à 101°C.

### EXEMPLE 6

On opère comme à l'exemple 1, à partir de 8,5 g de (chloro-3 propyl)-2 naphto[1,8-cd] isothiazole dioxyde-1,1, de 4,2 cm3 de triéthylamine et de 4,8 cm3 de N-phényl pipérazine dans 80 cm3 de toluène. Le mélange est chauffé 8 heures à ébullition puis refroidi à une température voisine de 20°C. Après purification parflash-chromatographie sur colonne de silice, sous courant d'argon à moyenne pression (0,5-1,5 bar) avec de l'acétate d'éthyle comme éluant et recristallisation dans 30 cm3 d'acétonitrile bouillant, on obtient 5,7 g de [(phényl-4 pipérazinyl-1)-3 propyl]-2 naphto[1,8-cd] isothiazole dioxyde-1,1 fondant à 126°C .

### EXEMPLE 7

On opère comme à l'exemple 1, à partir de 8,5 g de (chloro-3 propyl)-2 naphto[1,8-cd] isothiazole dioxyde-1,1, de 4,2 cm3 de triéthylamine et de 4,9 g de phényl-4 pipéridine dans 80 cm3 de toluène. Le mélange est chauffé 8 heures à ébullition puis refroidi à une température voisine de 20°C. L'agitation est maintenue durant 15 heures à cette température. Après purification parflash-chromatographie sur colonne de silice, sous courant d'argon à moyenne pression (0,5-1,5 bar) avec de l'acétate d'éthyle comme éluant et recristallisation dans 30 cm3 d'acétonitrile bouillant, on obtient 7,1 g de [(phényl-4 pipéridino-1)-3 propyl]-2 naphto[1,8-cd] isothiazole dioxyde-1,1 fondant à 125°C.

### EXEMPLE 8

On opère comme à l'exemple 1, à partir de 70,3 g de (chloro-3 propyl)-2 naphto[1,8-cd] isothiazole dioxyde-1,1, de 105 cm3 de triéthylamine et de 85,2 g de dibromhydrate d'(hydroxy-4 phényl)-1 pipérazine dans 2100 cm3 de diméthylformamide. Le mélange est chauffé 8 heures à ébullition puis refroidi à une température voisine de 20°C. L'agitation est maintenue durant 6 heures à cette température. Après purification par flash-chromatographie sur colonne de silice, sous courant d'argon à moyenne pression (0,5-1,5 bar) avec du dichlorométhane puis un mélange de dichlorométhane et d'acétate d'éthyle (50-50 en volumes) comme éluant et recristallisation dans 1150 cm3 d'acétonitrile bouillant, on obtient 20,5 g d'[((hydroxy-4 phényl)-4 pipérazinyl-1)-3 propyl]-2 naphto(1,8-cd] isothiazole dioxyde-1,1 fondant à 185°C.

Le dibromhydrate d'(hydroxy-4 phényl)-1 pipérazine peut être préparé de la manière suivante : à 70 g de dichlorhydrate de (méthoxy-4 phényl)-4 pipérazine sont ajoutés, durant 30 minutes et à une température voisine de 20°C, 720 cm3 d'une solution aqueuse d'acide bromhydrique à 47 %. Le mélange est chauffé à ébullition pendant 4 heures puis refroidi à une température voisine de 20°C. L'agitation est maintenue durant 15 heures à cette température puis le mélange est concentré à 40°C sous pression réduite (20 mm de mercure ; 2,7 kPa). L'huile résiduelle est reprise par 300 cm3 d'acétonitrile. Le précipité formé est séparé par filtration, lavé par 2 fois 50 cm3 d'acétonitrile et 2 fois 100 cm3 d'éther de diisopropyle. On obtient 85,2 g de dibromhydrate d'(hydroxy-4 phényl)-4 pipérazine (point de fusion supérieur à 260°C) utilisé à l'état brut dans les synthèses ultérieures.

### EXEMPLE 9

On opère comme à l'exemple 1, à partir de 4,2 g de (chloro-3 propyl)-2 naphto[1,8-cd] isothiazole dioxyde-1,1, de 2,1 cm3 de triéthylamine, de 2,2 g d'iodure de sodium et de 3,1 g de (fluoro-4 benzoyl)-4 pipéridine dans 50 cm3 de diméthylformamide. Le mélange est chauffé 8 heures à ébullition puis refroidi à une température voisine de 20°C. L'agitation est maintenue durant 7 heures à cette température. Après purification par flash-chromatographie sur colonne de silice, sous courant d'argon à moyenne pression (0,5-1,5 bar) avec de l'acétate d'éthyle comme éluant et recristallisation dans 15 cm3 d'acétonitrile bouillant, on obtient 1,65 g de [((fluoro-4 benzoyl)-4 pipéridino)-3 propyl]-2 naphto[1,8-cd] isothiazole dioxyde-1,1 fondant à 132°C.

La (fluoro-4 benzoyl)-4 pipéridine peut être préparée selon la méthode décrite par R. L. DUNCAN et coll., J. Med. Chem., 13, 1 (1970).

### EXEMPLE 10

On opère comme à l'exemple 1, à partir de 5,3 g de (chloro-3 propyl)-2 naphto[1,8-cd] isothiazole dioxyde-1,1, de 2,6 cm3 de triéthylamine, de 5 g de (chloro-4 phényl)-4 pipérazine et de 2,8 g d'iodure de sodium dans 50 cm3 de toluène. Le mélange est chauffé 6 heures à ébullition puis refroidi à une température voisine de 20°C. Après recristallisation une première fois dans 25 cm3 d'acétonitrile bouillant et une seconde fois dans 15 cm3 de méthyléthylcétone bouillante, on obtient 3 g de [((chloro-4 phényl)-4 pipérazinyl-1 )-3 propyl]-2 naphto[1,8-cd] isothiazole dioxyde-1,1 fondant à 120°C.

### EXEMPLE 11

On opère comme à l'exemple 1, à partir de 6,5 g de (chloro-2 éthyl)-2 naphto[1,8-cd] isothiazole dioxyde-1,1, de 3,4 cm3 de triéthylamine et de 3,9 g de phényl-4 tétrahydro-1,2,3,6 pyridine dans 150 cm3 de toluène. Le mélange est chauffé 9 heures à ébullition puis refroidi à une température voisine de 20°C. Après purification par flash-chromatographie sur colonne de silice, sous courant d'argon à moyenne pression (0,5-1,5 bar) avec du dichlorométhane comme éluant et recristallisation dans 10 cm3 d'acétonitrile bouillant, on obtient 2,5 g de [(phényl-4 tétrahydro-1,2,3,6 pyridyl-1)-2 éthyl]-2 naphto[1,8-cd] isothiazole dioxyde-1,1 fondant à 106°C.

Le (chloro-2 éthyl)-2 naphto[1,8-cd] isothiazole dioxyde-1,1 peut être préparé de la manière suivante : on opère comme à l'exemple 1 pour la préparation du (chloro-3 propyl)-2 naphto [1,8-cd] isothiazole dioxyde-1,1, à partir de 4,8 g d'hydrure de sodium à 50 % en dispersion dans l'huile de vaseline, de 8,8 cm3 de bromo-1 chloro-2 éthane et de 20,5 g de naphtosultam-1,8 dans 250 cm3 de diméthylformamide. Après purification par flash-chromatographie sur colonne de silice, sous courant d'argon à moyenne pression (0,5-1,5 bar) avec un mélange de dichlorométhane et de cyclohexane (70-30 en volumes) comme éluant, on obtient 20,7 g de (chloro-2 éthyl)-2 naphto[1,8-cd] isothiazole dioxyde-1,1 fondant à 96°C.

### EXEMPLE 12

On opère comme à l'exemple 1, à partir de 2,8 g de (chloro-3 propyl)-2 naphto[1,8-cd] isothiazole dioxyde-1,1, de 2,8 cm3 de triéthylamine et de 2,7 g de chlorhydrate de diphényl-4,4 pipéridine dans 30 cm3 de toluène. Le mélange est chauffé 8 heures à ébullition puis refroidi à une température voisine de 20°C. L'agitation est maintenue durant 7 heures à cette température. Après purification par flash-chromatographie sur colonne de silice, sous courant d'argon à moyenne pression (0,5-1,5 bar) avec de l'acétate d'éthyle comme éluant et recristallisation dans 10 cm3 d'acétonitrile bouillant, on obtient 1,6 g de [(diphényl-4,4 pipéridino)-3 propyl]-2 naphto [1,8-cd] isothiazole dioxyde-1,1 fondant à 168°C.

Le chlorhydrate de diphényl-4,4 pipéridine peut être préparé selon la méthode décrite dans le brevet allemand 2139084.

### EXEMPLE 13

On opère comme à l'exemple 1, à partir de 5,6 g de (chloro-3 propyl)-2 naphto[1,8-cd] isothiazole dioxyde-1,1, de 2,8 cm3 de triéthylamine et de 4,8 g de (bromo-4 phényl)-4 pipérazine dans 60 cm3 de toluène. Le mélange est chauffé 8 heures à ébullition puis refroidi à une température voisine de 20°C. L'agitation est maintenue durant 8 heures à cette température. Après purification par flash-chromatographie sur colonne de silice, sous courant d'argon à moyenne pression (0,5-1,5 bar) avec de l'acétate d'éthyle comme éluant et recristallisation dans 40 cm3 d'acétonitrile bouillant, on obtient 4,3 g de [((bromo-4 phényl)-4 pipérazinyl-1)-3 propyl]-2 naphto[1,8-cd] isothiazole dioxyde-1,1 fondant à 149°C.

Le dichlorhydrate de (bromo-4 phényl)-4 pipérazine peut être préparé selon la méthode décrite par D. K. YUNG, J. Med. Chem., 21, 1301 (1978).

### EXEMPLE 14

On opère comme à l'exemple 1, à partir de 6,7 g de (chloro-2 éthyl)-2 naphto[1,8-cd] isothiazole dioxyde-1,1, de 3,5 cm3 de triéthylamine, de 5,2 g de (fluoro-4 benzoyl)-4 pipéridine et de 3,7 g d'iodure de sodium dans 100 cm3 de diméthylformamide. Le mélange est chauffé 6 heures à ébullition puis refroidi à une température voisine de 20°C. Après purification par flash-chromatographie sur colonne de silice, sous courant d'argon à moyenne pression (0,5-1,5 bar) avec du dichlorométhane comme éluant et recristallisation dans 8 cm3 d'acétonitrile bouillant, on obtient 2,9 g de [((fluoro-4 benzoyl)-4 pipéridino)-2 éthyl]-2 naphto[1,8-cd] isothiazole dioxyde-1,1 fondant à 145°C.

### EXEMPLE 15

On opère comme à l'exemple 1, à partir de 9,3 g de (chloro-3 propyl)-2 naphto[1,8-cd] isothiazole dioxyde-1,1, de 4,6 cm3 de triéthylamine et de 6,5 g d'(indolyl-3)-4 tétrahydro-1,2,3,6 pyridine dans 100 cm3 de diméthylformamide. Le mélange est chauffé 8 heures à ébullition puis refroidi à une température voisine de 20°C. L'agitation est maintenue durant 7 heures à cette température. Après purification par flash-chromatographie sur colonne de silice, sous courant d'argon à moyenne pression (0,5-1,5 bar) avec de l'acétate d'éthyle comme éluant, on obtient 2,9 g d'un solide jaune qui est purifié par flash-chromatographie sur colonne de silice, sous courant d'argon à moyenne pression (0,5-1,5 bar) avec un mélange de dichlorométhane et de méthanol (90-10 en volumes) comme éluant et recristallisation dans 40 cm3 d'acétonitrile bouillant. On obtient 2,1 g d'[((indolyl-3)-4 tétrahydro-1,2,3,6 pyridyl-1)-3 propyl]-2 naphto [1,8-cd] isothiazole dioxyde-1,1 fondant à 226°C.

L'(indolyl-3)-4 tétrahydro-1,2,3,6 pyridine peut être préparée selon la méthode décrite par L. NEDELEC et coll., Eur. J. Med. Chem., 22, 33 (1987).

### EXEMPLE 16

On opère comme à l'exemple 1, à partir de 10 g de (chloro-4 butyl)-2 naphto[1,8-cd] isothiazole dioxyde-1,1, de 4,8 cm3 de triéthylamine et de 5,4 g de phényl-4 tétrahydro-1,2,3,6 pyridine dans 300 cm3 de toluène. Le mélange est chauffé 9 heures à ébullition puis refroidi à une température voisine de 20°C. Après purification par flash-chromatographie sur colonne de silice, sous courant d'argon à moyenne pression (0,5-1,5 bar) avec de l'acétate d'éthyle comme éluant et recristallisation dans 10 cm3 d'acétonitrile bouillant, on obtient 2,6 g de [(phényl-4 tétrahydro-1,2,3,6 pyridyl-1)-4 butyl]-2 naphto[1,8-cd] isothiazole dioxyde-1,1 fondant à 101°C.

Le (chloro-4 butyl)-2 naphto[1,8-cd] isothiazole dioxyde-1,1 peut être préparé de la manière suivante : on opère comme à l'exemple 1 pour la préparation du (chloro-3 propyl)-2 naphto [1,8-cd] isothiazole dioxyde-1,1, à partir de 4,8 g d'hydrure de sodium à 50 % en dispersion dans l'huile de vaseline, de 11,9 cm3 de bromo-1 chloro-4 butane et de 20,5 g de naphtosultam-1,8 dans 250 cm3 de diméthylformamide. Après purification par flash-chromatographie sur colonne de silice, sous courant d'argon à moyenne pression (0,5-1,5 bar) avec un mélange de dichlorométhane et de cyclohexane (30-70 en volumes) comme éluant, on obtient 24,6 g de (chloro-4 butyl)-2 naphto[1,8-cd] isothiazole dioxyde-1,1 sous forme d'une huile jaune utilisée à l'état brut dans les synthèses ultérieures.

### EXEMPLE 17

On opère comme à l'exemple 1, partir de 3,9 g de (chloro-3 propyl)-2 naphto[1,8-cd] isothiazole dioxyde-1,1, de 1,9 cm3 de triéthylamine, de 3,7 g de [bis(fluoro-4 phényl) méthylène]-4 pipéridine et de 2,1 g d'iodure de sodium dans 150 cm3 de toluène. Le mélange est chauffé 6 heures à ébullition puis refroidi à une température voisine de 20°C. Après purification par flash-chromatographie sur colonne de silice, sous courant d'argon à moyenne pression (0,5-1,5 bar) avec du dichlorométhane comme éluant, on obtient 4,6 g de [(bis(fluoro-4 phényl) méthylène-4 pipéridino)-3 propyl]-2 naphto [1,8-cd] isothiazole dioxyde-1,1 sous forme d'une huile brune que l'on transforme en chlorhydrate fondant à 137°C.

La (bis(fluoro-4 phényl) méthylène)-4 pipéridine peut être préparé selon la méthode décrite dans le brevet EP 110 435.

### EXEMPLE 18

A 0,6 g d'hydrure de sodium en dispersion à 80 % dans l'huile de vaseline, sous courant d'argon, on ajoute 20 cm3 de diméthylformamide. La suspension obtenue est agitée, puis une solution de 5,7 g de bromo-5 naphto[1,8-cd] isothiazole dioxyde-1,1 dans 30 cm3 de diméthylformamide est coulée goutte à goutte, pendant 15 minutes, en maintenant la température entre 20°C et 30°C. Le milieu réactionnel est agitée 15 minutes à une température voisine de 20°C, puis une solution de 4,7 g de (chloro-3 propyl)-1 phényl-4 tétrahydro-1,2,3,6 pyridine dans 20 cm3 de diméthylformamide est coulée goutte à goutte, en 10 minutes, à une température voisine de 20°C. Le milieu réactionnel est chauffé 1 heure à 100°C, refroidi à une température voisine de 20°C. Il est repris par 300 cm3 d'eau distillée et extrait par 200 cm3 d'acétate d'éthyle. Après lavage par trois fois 50 cm3 d'eau distillée, l'extrait organique est séché sur sulfate de magnésium et concentré à sec à 40°C sous pression réduite (20 mm de mercure ; 2,7 kPa). Après recristallisation dans 110 cm3 d'acétonitrile bouillant, on obtient 5,3 g de bromo-5 [(phényl-4 tétrahydro-1,2,3,6 pyridyl-l)-3 propyl]-2 naphto[1,8-cd] isothiazole dioxyde-1,1 fondant à 138°C.

La (chloro-3 propyl)-1 phényl-4 tétrahydro-1,2,3,6 pyridine peut être préparée de la façon suivante : 41 g de phényl-4 tétrahydro-1,2,3,6 pyridine, 100 cm3 de bromo-1 chloro-3 propane, 140 g de carbonate de potassium et 600 cm3 d'acétonitrile sont agités 12 heures à une température voisine de 20°C. Après filtration, la solution est concentrée à sec à 40°C sous pression réduite (20 mm de mercure ; 2,7 kPa). Le résidu est purifié par chromatographie sur colonne de silice avec un mélange de cyclohexane et d'acétate d'éthyle (60-40 en volumes) comme éluant. On obtient48 g de (chloro-3 propyl)-1 phényl-4 tétrahydro-1,2,3,6 pyridine sous forme d'huile.

Le bromo-5 naphto[1,8-cd] isothiazole dioxyde-1, peut être obtenu selon le procédé décrit par A. MUSTAFA et coll., Arch. Pharm., 298, 741, (1965).

### EXEMPLE 19

0,8 g d'acétylamino-5 (chloro-3 propyl)-2 naphto[1,8-cd] isothiazole dioxyde-1,1, 0,4 g de phényl-4 tétrahydro-1,2,3,6 pyridine, 4 g de carbonate de potassium et 25 cm3 d'acétonitrile sont chauffés 20 heures à l'ébullition. Le mélange réactionnel est refroidi à une température voisine de 20°C. La solution est filtrée puis concentrée à sec à 40°C sous pression réduite (20 mm de mercure ; 2,7 kPa). Le résidu est purifié par chromatographie sur colonne de silice avec un mélange d'acétate d'éthyle et de chlorure de méthylène (70-30 en volumes) comme éluant, puis recristallisé dans 20 cm3 d'éthanol bouillant. On obtient 0,35 g d'acétylamino-5 [(phényl-4 tétrahydro-1,2,3,6 pyridyl-1)-3 propyl]-2 naphto[1,8-cd] isothiazole dioxyde-1,1 fondant à 178°C.

L'acétylamino-5 (chloro-3 propyl)-2 naphto[1,8-cd] isothiazole dioxyde-1,1 peut être préparé de la façon suivante : 2,1 g d'amino-5 (chloro-3 propyl)-2 naphto[1,8-cd] isothiazole dioxyde-1,1, 0,2 g d'acétate de sodium et 15 cm3 d'anhydride acétique sont chauffés pendant 30 minutes à l'ébullition. Le mélange réactionnel est refroidi à une température voisine de 20°C, traité par 5 cm3 d'eau distillée et chauffé 15 minutes à l'ébullition. Le mélange est refroidi à une température voisine de 20°C puis concentré à sec à 50°C sous pression réduite (0,5 mm de mercure ; 0,07 kPa). Le résidu est repris par 100 cm3 de dichlorométhane et la solution est lavée par 40 cm3 d'eau distillée, séchée sur sulfate de magnésium, puis concentrée à sec à 20°C sous pression réduite (20 mm de mercure ; 2,7 kPa). Après recristallisation dans 15 cm3 de chloroforme bouillant, on obtient 0,6 g d'acétylamino-5 (chloro-3 propyl)-2 naphto[1,8-cd] isothiazole dioxyde-1,1 fondant à 205°C.

L'amino-5 (chloro-3 propyl)-2 naphto[1,8-cd] isothiazole dioxyde-1,1 peut être préparé de la façon suivante : à une solution de 12,2 g de nitro-5 (chloro-3 propyl)-2 naphto[1,8-cd] isothiazole dioxyde-1,1 et de 18 g de chlorure de nickel à 6 molécules d'eau dans 600 cm3 de méthanol, on ajoute, à une température voisine de 0°C, par petites portions, en une heure, 3,8 g de borohydrure de sodium. Le mélange réactionnel est agité une heure à cette température puis concentré à sec à 40°C sous pression réduite (20 mm de mercure; 2,7 kPa). Le résidu est repris par 50 cm3 d'acide chlorhydrique 1 H, puis traité par 100 cm3 d'une solution d'ammoniaque 3N. Le mélange est extrait par 500 cm3 de dichlorométhane. L'extrait organique est séché sur sulfate de magnésium et concentré à sec à 20°C sous pression réduite (20 mm de mercure ; 2,7 kPa). Le résidu est purifié par chromatographie sur colonne de silice avec du dichlorométhane comme éluant. On obtient 3,4 g d'amino-5 (chloro-3 propyl)-2 naphto[1,8-cd] isothiazole dioxyde-1,1 sous forme d'huile.

Le nitro-5 (chloro-3 propyl)-2 naphto[1,8-cd] isothiazole dioxyde-1,1 peut être préparé de la façon suivante : à 50 cm3 d'une solution à 66,97 % d'acide nitrique (d=1,4), on ajoute, en 5 minutes, à une température voisine de 0°C, 12 g de (chloro-3 propyl)-2 naphto[1,8-cd] isothiazole dioxyde-1,1, obtenu selon l'exemple 1. Le mélange réactionnel est agité une heure à cette température puis traité par 150 cm3 d'eau distillée et encore agité 15 minutes. Le précipité est filtré, lavé par trois fois 50 cm3 d'eau distillé puis séché à l'air. On obtient 12,2 g de nitro-5 (chloro-3 propyl)-2 naphto[1,8-cd] isothiazole dioxyde-1,1 utilisé à l'état brut dans les synthèses ultérieures.

### EXEMPLE 20

On opère comme à l'exemple 18, à partir de 1,2 g de chloro-5 naphto[1,8-cd] isothiazole dioxyde-1,1, de 0,15 g d'hydrure de sodium en dispersion à 80 % dans l'huile de vaseline, de 1,4 g de (chloro-3 propyl)-1 phényl-4 tétrahydro-1,2,3,6 pyridine, obtenue selon l'exemple 18 et de 30 cm3 de diméthylformamide. Le mélange réactionnel est chauffé une heure à 70°C, refroidi à une température voisine de 20°C puis concentré à sec à 20°C sous pression réduite (0,5 mm de mercure ; 0,07 kPa). Le résidu est repris par 100 cm3 de dichlorométhane et la solution est lavée par 50 cm3 d'eau distillée, séchée sur sulfate de magnésium et concentrée à sec à 20°C sous pression réduite (20 mm de mercure ; 2,7 kPa). Le résidu est purifié par chromatographie sur colonne de silice avec un mélange de dichlorométhane et d'acétate d'éthyle (90-10 en volumes) comme éluant et recristallisé dans 30 cm3 d'acétonitrile bouillant. On obtient 1 g de chloro-5 [(phényl-4 tétrahydro-1,2,3,6 pyridyi-1)-3 propyl]-2 naphto[1,8-cd] isothiazole dioxyde-1,1 fondant à 135°C.

Le chloro-5 naphto[1,8-cd] isothiazole dioxyde-1,1 peut être préparé selon le procédé décrit par A. MUSTAFA et coll., Arch. Pharm., 298, 741, (1965).

Le chloro-5 [(phényl-4tétrahydro-1,2,3,6 pyridyi-1 )-3 propyl]-2 naphto[1,8-cd] isothiazole dioxyde-1,1 peut être également préparé de la façon suivante : on opère comme à l'exemple 19, à partir de 3,2 g de chloro-5 (chloro-3 propyl)-2 naphto[1,8-cd] isothiazole dioxyde-1,1, de 1,8 g de phényl-4 tétrahydro-1,2,3,6 pyridine, de 12 g de carbonate de potassium et de 125 cm3 d'acétonitrile. Le mélange réactionnel est chauffé 5 heures à l'ébullition puis refroidi à une température voisine de 20°C. Après filtration et concentration à sec à 40°C sous pression réduite (20 mm de mercure ; 2,7 kPa), le résidu est purifié par chromatographie sur colonne de silice avec un mélange de dichlorométhane et d'acétate d'éthyle (85-15 en volumes) comme éluant et recristallisé dans 40 cm3 d'acétonitrile bouillant. On obtient 2,8 g de chloro-5 [(phényl-4 tétrahydro-1,2,3,6 pyridyl-1)-3 pro- pyl]-2 naphto[1,8-cd] isothiazole dioxyde-1,1 fondant à 135°C.

Le chloro-5 (chloro-3 propyl)-2 naphto[1,8-cd] isothiazole dioxyde-1,1 peut être préparé de la façon suivante : une solution de 5,4 g de chlore dans 100 cm3 d'acide acétique est coulée goutte à goutte, pendant 15 minutes, à une température voisine de 0°C, à une solution de 18 g de (chloro-3 propyl)-2 naphto[1,8-cd] isothiazole dioxyde-1,1, obtenu selon l'exemple 1, dans 200 cm3 d'acide acétique. Le mélange réactionnel est agité 24 heures à une température voisine de 20°C puis concentré à sec à 60°C sous pression réduite (20 mm de mercure ; 2,7 kPa). Le résidu est purifié par chromatographie sur colonne de silice avec un mélange de dichlorométhane et de cyclohexane (50-50 en volumes) comme éluant. On obtient 1,7 g de dichloro-3,5 (chloro-3 propyl)-2 naphto[1,8-cd] isothiazole dioxyde-1,1 fondant à 164°C et 3,2 g de chloro-5 (chloro-3 propyl)-2 naphto[1,8-cd] isothiazole dioxyde-1,1 fondant à 80°C.

### EXEMPLE 21

On opère comme à l'exemple 19, à partir de 1,7 g de dichloro-3,5 (chloro-3 propyl)-2 naphto[1,8-cd] isothiazole dioxyde-1,1 obtenu selon l'exemple 20, de 0,85 g de phényl-4 tétrahydro-1,2,3,6 pyridine, de 7 g de carbonate de potassium et de 90 cm3 d'acétonitrile. Le mélange réactionnel est chauffé 20 heures à l'ébullition puis refroidi à une température voisine de 20°C. La solution est filtrée puis concentrée à sec à 40°C sous pression réduite (20 mm de mercure ; 2,7 kPa). Le résidu est recristallisé dans 25 cm3 d'acétonitrile bouillant. On obtient 1 g de dichloro-3,5 [(phényl-4 tétrahydro-1,2,3,6 pyridyl-1)-3 propyl]-2 naphto [1,8-cd] isothiazole dioxyde-1,1 fondant à 126°C.

### EXEMPLE 22

On opère comme à l'exemple 18, à partir de 1,4 g de chloro-6 naphto[1,8-cd] isothiazole dioxyde-1,1, de 1,5 g de (chloro-3 propyl)-1 (fluoro-4 phényl)-4 pipérazine, de 0,18 g d'hydrure de sodium en dispersion à 80 % dans l'huile de vaseline et de 15 cm3 de diméthylformamide. Le mélange réactionnel est chauffé 1 heure 30 minutes à 90°C, refroidi à une température voisine de 20°C et ensuite concentré à sec à 60°C sous pression réduite (0,5 mm de mercure ; 0,07 kPa). Le résidu est repris par 50 cm3 de dichlorométhane et la solution est lavée par 20 cm3 d'eau distillée, séchée sur sulfate de magnésium et concentrée à sec à 20°C sous pression réduite (20 mm de mercure ; 2,7 kPa). Le résidu est purifié par chromatographie sur colonne de silice avec un mélange de dichlorométhane et d'acétate d'éthyle (95-5 en volumes) comme éluant puis recristallisé dans 10 cm3 d'acétonitrile bouillant. On obtient 1,2 g de chloro-6 [((fluoro-4 phényl)-4 pipérazinyl-1)-3 propyl]-2 naphto [1,8-cd] isothiazole dioxyde-1,1 fondant à 125°C.

Le chloro-6 naphto[1,8-cd] isothiazole dioxyde-1,1 peut être préparé de la façon suivante : 13,3 g d'acide amino-8 chloro-4 naphtalènesulfonique-1 et 30 cm3 d'oxychlorure de phosphore sont chauffés 3 heures à l'ébullition. Le mélange réactionnel est ensuite refroidi à une température voisine de 0°C puis traité par 100 cm3 d'eau distillée. Le précipité est isolé par filtration, lavé par trois fois 20 cm3 d'eau distillée et extrait par trois fois 150 cm3 de chloroforme. Les extraits organiques sont séchés sur sulfate de magnésium et concentrés à sec à 40°C sous pression réduite (20 mm de mercure ; 2,7 kPa). On obtient 1,4 g de chloro-6 naphto[1,8- cd] isothiazole dioxyde-1,1 brut utilisé tel quel dans les synthèses ultérieures.

L'acide amino-8 chloro-4 naphtalènesulfonique-1 peut être préparé selon le procédé décrit par P. FRIEDLANDER et coll., Chem. Ber., 55B, 45, (1922).

La (chloro-3 propyl)-1 (fluoro-4 phényl)-4 pipérazine peut être préparée de la façon suivante : on opère comme à l'exemple 18 pour la (chloro-3 propyl)-1 phényl-4 tétrahydro-1,2,3,6 pyridine, à partir de 50 g de (fluoro-4 phényl)-1 pipérazine, de 68 cm3 de bromo-1 chloro-3 propane, de 97 g de carbonate de sodium et de 400 cm3 d'acétonitrile. Le mélange réactionnel est agité 12 heures à une température voisine de 20°C et la solution est filtrée puis concentrée à sec à 40°C sous pression réduite (20 mm de mercure ; 2,7 kPa). Le résidu est purifié par chromatographie sur colonne de silice avec de l'acétate d'éthyle comme éluant. On obtient 44,4 g de (chloro-3 propyl)-1 (fluoro-4 phényl)-4 pipérazine sous forme d'huile.

### EXEMPLE 23

Aune solution du sel de sodium du 2H-isothiazolo [3,4,5-de] isoquinoléine dioxyde-1,1 (obtenue par chauffage, après cessation du dégagement gazeux, d'une solution de 4,1 g de bromo-4 isoquinolyl-5 sulfonamide dans 25 cm3 de diméthylformamide et d'une suspension de 0,85 g d'hydrure de sodium en dispersion à 80 % dans l'huile de vaseline dans 50 cm3 de diméthylformamide, pendant 2 heures et 30 minutes à 110°C) refroidie à 20°C, on ajoute une solution de 4,9 g de (chloro-3 propyl)-1 phényl-4 tétrahydro-1,2,3,6 pyridine dans 20 cm3 de diméthylformamide. Le milieu réactionnel est chauffé 2 heures à 100°C puis versé dans un mélange de 300 cm3 d'eau et de 300 cm3 d'acétate d'éthyle. La phase organique est lavée 3 fois par 300 cm3 d'eau, séchée sur sulfate de magnésium et concentrée à sec. Le résidu est chromatographié sur une colonne de silice sous une légère surpression d'azote en éluant par de l'acétate d'éthyle puis, par un mélange d'acétate d'éthyle et d'éthanol (95-5 en volumes). On obtient 1,4 g de [(phényl-4 tétrahydro-1,2,3,6 pyridyl)-3 propyl]-2 2H-isothiazolo[3,4,5-de] isoquinoléine dioxyde-1,1 sous la forme d'une huile jaune. 1,8 g de ce produit sont purifiés par une deuxième chromatographie semblable pour donner 1,6 g de [(phényl-4 tétrahydro-1,2,3,6 pyridyl)-3 pro- pyl]-2 2H-isothiazolo[3,4,5-de] isoquinoléine dioxyde-1,1 (Rf=0,27 ; support : gel de silice; éluant : acétate d'éthyle-éthanol (9-1 en volumes)). Ce produit est dissous dans 100 cm3 d'acétate d'éthyle avant d'ajouter 5 cm3 d'une solution 3N d'acide chlorhydrique dans l'oxyde d'isopropyle. Le précipité est essoré, lavé par de l'acétate d'éthyle et séché sous vide (0,1 mm de mercure) à 35°C. On obtient 1,6 g de dichlorhydrate de [(phényl-4 tétrahydro-1,2,3,6 pyridyl)-3 propyl]-2 2H-isothiazolo [3,4,5-de] isoquinoléine dioxyde-1,1 sous la forme d'un solide jaune (amorphe).

Le bromo-4 isoquinolyl-5 sulfonamide peut être obtenu de la manière suivante : on fait barbotter jusqu'à saturation de l'ammoniac dans du tétrahydrofuranne sec à -50°C. Cette solution est traitée par une suspension de 8 g de chlorure de bromo-4 isoquinolyl-5 sulfonyle dans 50 cm3 de tétrahydrofuranne. On laisse revenir le mélange réactionnel progressivement à 20°C. Le précipité formé est essoré, lavé 3 fois par 10 cm3 de tétrahydrofuranne, 3 fois par 30 cm3 d'eau et 2 fois par 10 cm3 d'acétate d'éthyle puis essoré et séché. On obtient 4,5 g de bromo-4 isoquinolyl-5 sulfonamide sous la forme d'un solide beige dont le point de fusion est supérieur à 270°C.

Le chlorure de bromo-4 isoquinolyl-5 sulfonyle peut être obtenu par le procédé suivant : une solution de 4,46 g d'amino-5 bromo-4 isoquinoléine dans 44 cm3 d'acide chlorhydrique concentré (d=1,19) est refroidie à -5°C puis est traitée par une solution de 1,93 g de nitrite de sodium dans 10 cm3 d'eau. Le mélange réactionnel est agité 1 heure à 0°C. La solution obtenue est versée sur une solution saturée de dioxyde de soufre dans 48 ml d'acide acétique à laquelle a été ajoutée une solution de 0,65 g de chlorure cuivreux dans 5,6 cm3 d'eau. Le mélange réactionnel est agité jusqu'à la fin du dégagement gazeux puis est extrait 2 fois par 100 cm3 de dichlorométhane. Les phases organiques sont réunies, lavées à l'eau, séchées sur sulfate de magnésium et concentrées. On obtient 5,6 g de chlorure de bromo-4 isoquinolyl-5 sulfonyle sous la forme d'un solide jaune utilisé tel quel dans les synthèses ultérieures.

L'amino-5 bromo-4 isoquinoléine peut être préparée de la manière suivante : à une solution de 90 g de chlorure stanneux dans 100 cm3 d'acide chlorhydrique concentré (d=1,19), on ajoute progressivement une suspension de 25,3 g de bromo-4 nitro-5 isoquinoléine dans 180 cm3 d'acide chlorhydrique 2N. Le mélange réactionnel est chauffé au reflux pendant 90 minutes puis est refroidi et versé dans 2 litres de solution de soude 2N en agitant et refroidissant vers 0°C. Le précipité est lavé à l'eau, essoré et séché. On obtient 21 ,6 g d'amino-5 bromo-4 isoquinoléine sous la forme de cristaux jaunes fondant à 155°C.

La bromo-4 nitro-5 isoquinoléine peut être obtenue par le procédé décrit par M. D. NAIR et coll., Indian J. Chem. Soc., 5, 224 (1967).

### EXEMPLE 24

On opère comme à l'exemple 1, à partir de 12 g de (chloro-3 propyl)-2 naphto [1,8-cd] isothiazole dioxyde-1,1, de 6 cm3 de triéthylamine et de 7,5 g d'(hydroxy-4 phényl)-4 tétrahydro-1,2,3,6 pyridine dans 120 cm3 de diméthylformamide. Le mélange est chauffé 8 heures à ébullition puis refroidi à une température voisine de 20°C. L'agitation est maintenue durant 12 heures à cette température. Après purification par flash-chromatographie sur colonne de silice, sous courant d'argon à moyenne pression (0,5-1,5 bar) avec de l'acétate d'éthyle comme éluant et recristallisation dans 80 cm3 d'acétonitrile bouillant, on obtient 3,3 g d'[((hydroxy-4 phényl)-4 tétrahydro-1,2,3,6 pyridyi-1)-3 propyl]-2 naphto [1,8-cd] isothiazole dioxyde-1,1 fondant à 198°C.

L'(hydroxy-4 phényl)-4 tétrahydro-1,2,3,6 pyridine peut être préparée de la manière suivante : à 21 g de N-benzyloxycarbonyl hydroxy-4 (méthoxy-4 phényl)-4 pipéridine sont ajoutés, durant 1 heure et à une température voisine de 5°C, 250 cm3 d'une solution d'acide bromhydrique à 35% dans l'acide acétique. Le mélange est agité 1 heure à une température voisine de 20°C puis la phase aqueuse est lavée par 3 fois 500 cm3 d'éther diéthylique. Après décantation, la phase aqueuse est reprise par 250 cm3 d'eau et alcalinisée par de la soude 10N jusqu'à pH 10. Après extraction par 4 fois 500 cm3 de dichlorométhane, le précipité formé est séparé par filtration. On obtient ainsi 7,5 g d'(hydroxy-4 phényl)-4 tétrahydro-1,2,3,6 pyridine fondant à 245°C.

La N-benzyloxycarbonyl hydroxy-4 (méthoxy-4 phényl)-4 pipéridine peut être préparée de la manière suivante : à 9,3 g de magnésium en tournures dans 150 cm3 d'éther diéthylique, sous courant d'argon, on ajoute à une température voisine de 20°C, 1 cm3 de p-bromoanisole et quelques cristaux d'iode. Après 5 minutes, le milieu réactionnel est au reflux du solvant et on ajoute en 1 heure une solution de 24,7 cm3 de p-bromoanisole dans 300 cm3 d'éther diéthylique de telle façon que le reflux du solvant soit maintenu. L'agitation est poursuivie 30 minutes à cette température puis, après avoir refroidi le mélange à une température voisine de 20°C, on coule une solution de 48,2 g de N-benzyloxycarbonyl pipéridone-4 dans 450 cm3 d'éther éthylique. L'agitation est maintenue 15 heures à cette température. Le milieu réactionnel est ensuite hydrolysé par une solution saturée de chlorure d'ammonium jusqu'à pH 6-7, puis extrait par 3 fois 500 cm3 d'éther diéthylique. La phase organique est séchée sur du sulfate de magnésium anhydre et concentrée à sec sous pression réduite (20 mm de mercure ; 2,7 kPa). L'huile résiduelle est purifiée parflash-chromatographie sur colonne de silice, sous courant d'argon à moyenne pression (0,5-1,5 bar) avec du dichlorométhane puis un mélange de dichlorométhane et de méthanol (90-10 en volumes) comme éluant. On obtient ainsi 21,2 g de N-benzyloxycarbonyl hydroxy-4 (méthoxy-4 phényl)-4 pipéridine fondant à 98°C, utilisée à l'état brut dans les synthèses ultérieures.

La N-benzyloxycarbonyl pipéridone-4 peut être préparée selon la méthode décrite par H. STETTER et coll., Chem. Ber. 105, 2773 (1972).

### EXEMPLE 25

On opère comme à l'exemple 1, à partir de 6,74 g de (chloro-3 propyl)-2 naphto [1,8-cd] isothiazole dioxyde-1,1, de 3,4 cm3 de triéthylamine et de 4,5 g de (méthoxy-4 phényl)-4 tétrahydro-1,2,3,6 pyridine dans 70 cm3 de diméthylformamide. Le mélange est chauffé 8 heures à ébullition puis refroidi à une température voisine de 20°C. L'agitation est maintenue durant 12 heures à cette température. Après purification par flash-chromatographie sur gel de silice, sous courant d'argon à moyenne pression (0,5-1,5 bar) avec de l'acétate d'éthyle comme éluant et recristallisation dans 60 cm3 d'acétonitrile bouillant, on obtient 2 g de [((méthoxy-4 phényl)-4 tétrahydro-1,2,3,6 pyridyl-l)-3 propyl]-2 naphto [1,8-cd] isothiazole dioxyde-1,1 fondant à 178°C.

### EXEMPLE 26

On opère comme à 1 exemple 1, à partir de 75 g de (chloro-3 propyl)-2 naphto [1,8-cd] isothiazole dioxyde-1,1, de 37,5 cm3 de triéthylamine et de 55,3 g de (nitro-4 phényl)-4 pipérazine dans 600cm3 de toluène. Le mélange est chauffé 10 heures à ébullition puis refroidi à une température voisine de 20°C. Après purification par flash-chromatographie sur colonne de silice, sous courant d'argon à moyenne pression (0,5-1,5 bar) avec un mélange de dichlorométhane et d'acétate d'éthyle comme éluant (80-20 en volumes) et recristallisation dans 1100 cm3 de méthyl éthyl cétone bouillante, on obtient41,8 g de [((nitro-4 phényl)-4 pipérazinyl-1)-3 propyl]-2 naphto [1,8-cd] isothiazole dioxyde-1,1 fondant à 186°C.

### EXEMPLE 27

30g de [((nitro-4 phényl)-4 pipérazinyl-1)-3 propyl]-2 naphto[1,8-cd] isothiazole dioxyde-1,1, 74,8 g de chlorure stanneux dihydrate et 500 cm3 d'éthanol sont chauffés une heure à 60°C sous courant d'argon. On ajoute ensuite en une heure et par petites portions 1,3 g de borohydrure de sodium. L'agitation est maintenue une heure à 60°C puis 15 heures à une température voisine de 20°C. Le mélange est versé sur 500 cm3 d'eau et de glace puis alcalinisé par de la soude 4N jusqu'à pH 9. La phase organique est extraite par 4 fois 200 cm3 d'éther diéthylique, reprise par 3 g de noir 3S, séchée sur du sulfate de magnésium anhydre, filtrée et concentrée à sec sous pression réduite (20 mm de mercure ; 2,7 kPa). Le résidu obtenu est purifié par recristallisation, une première fois dans 250 cm3 de propanol-1 bouillant puis deux fois dans 20 cm3 d'éthanol bouillant. On obtient ainsi 2,4 g d'[«amino-4 phényl)-4 pipérazinyl-1 )-3 propyl]-2 naphto [1,8-cd] isothiazole dioxyde-1, 1 fondant à 113°C.

### EXEMPLE 28

On opère comme à l'exemple 1, à partir de 8,4 g de (chloro-3 propyl)-2 naphto [1,8-cd] isothiazole dioxyde-1,1,4,2 cm3 de triéthylamine et 5,3 g de (fluoro-3 phényl)-4 pipérazine dans 100 cm3 de toluène. Le mélange est chauffé 8 heures à ébullition puis refroidi à une température voisine de 20°C. Après purification parflash-chromatographie sur colonne de silice, sous courant d'argon à moyenne pression (0,5-1,5 bar) avec du dichlorométhane puis un mélange de dichlorométhane et d'acétate d'éthyle comme éluant (50-50 en volumes) et recristallisation dans 20 cm3 d'acétonitrile bouillant, on obtient 4,7 g de [((fluoro-3 phényl)-4 pipérazinyl-1)-3 pro- pyl]-2 naphto [1,8-cd] isothiazole dioxyde-1,1 fondant à 103°C.

La (fluoro-3 phényl)-4 pipérazine peut être préparée selon la méthode décrite par L. THUNUS et coll., Ann. Pharm., 38, 353 (1980).

### EXEMPLE 29

On opère comme à l'exemple 1, à partir de 7 g de (chloro-3 propyl)-2 naphto [1,8-cd] isothiazole dioxyde-1,1, de 3,5 cm3 de triéthylamine et de 4,4 g de (fluoro-2 phényl)-4 pipérazine dans 150 cm3 de toluène. Le mélange est chauffé 8 heures à ébullition puis refroidi à une température voisine de 20°C. Après purification par flash-chromatographie sur colonne de silice, sous courant d'argon à moyenne pression (0,5-1,5 bar) avec du dichlorométhane comme éluant et recristallisation dans 15 cm3 d'acétonitrile bouillant, on obtient 4,8 g de [((fluoro-2 phényl)-4 pipérazinyl-l)-3 propyl]-2 naphto [1,8-cd] isothiazole dioxyde-1,1 fondant à 112°C.

La (fluoro-2 phényl)-4 pipérazine peut être préparée selon la méthode décrite par L. THUNUS et coll., Ann. Pharm., 38, 353 (1980).

### EXEMPLE 30

On opère comme à l'exemple 1, à partir de 7 g de (chloro-3 propyl)-2 naphto [1,8-cd] isothiazole dioxyde-1,1, de 3,5 cm3 de triéthylamine et de 4,4 g de (méthyl-4 phényl)-4 pipérazine dans 100 cm3 de toluène. Le mélange est chauffé 6 heures à ébullition puis refroidi à une température voisine de 20°C. Après purification par flash-chromatographie sur colonne de silice, sous courant d'argon à moyenne pression (0,5-1,5 bar) avec un mélange de dichlorométhane et d'acétate d'éthyle comme éluant (80-20 en volumes) et recristallisation dans 40 cm3 d'acétonitrile bouillant, on obtient 4,5 g de [((méthyl-4 phényl)-4 pipérazinyl-1)-3 propyl]-2 naphto [1,8- cd] isothiazole dioxyde-1,1 fondant à 136°C.

La (méthyl-4 phényl)-4 pipérazine peut être préparée selon la méthode décrite par L. THUNUS etcoll., Ann. Pharm., 38, 353 (1980).

### EXEMPLE 31

On opère comme à l'exemple 1, à partir de 7,2 g de (chloro-3 propyl)-2 naphto [1,8-cd] isothiazole dioxyde-1,1, de 3,6 cm3 de triéthylamine et de 5,6 g de (pipéridinyl-4)-1 benzimidazolinone-2 dans 100 cm3 de diméthylformamide. Le mélange est chauffé 8 heures à ébullition puis refroidi à une température voisine de 20°C. Après purification par flash-chromatographie sur colonne de silice, sous courant d'argon à moyenne pression (0,5-1,5 bar) avec un mélange de dichlorométhane et d'éthanol comme éluant (95-5 en volumes) et recristallisation dans 200 cm3 d'acétonitrile bouillant, on obtient 1,6 g d'[((oxo-2 benzimida - zolinyl-1)-4 pipéridino)-3 propyl]-2 naphto [1,8-cd] isothiazole dioxyde-1,1 fondant à 253°C.

La (pipéridinyl-4)-1 benzimidazolinone-2 peut être préparée selon la méthode décrite dans le brevet US 4 470 989.

### EXEMPLE 32

On opère comme à l'exemple 1, à partir de 3,6 g de (chloro-3 propyl)-2 naphto [1,8-cd] isothiazole dioxyde-1,1, de 1,8 cm3 de triéthylamine et de 2,6 g de (benzisoxazol-1,2 yl-3)-4 pipérazine dans 200 cm3 de diméthylformamide. Le mélange est chauffé 8 heures à ébullition puis refroidi à une température voisine de 20°C. Après purification par flash-chromatographie sur gel de silice, sous courant d'argon à moyenne pression (0,5-1,5 bar) avec du dichlorométhane comme éluant et recristallisation dans 30 cm3 d'acétonitrile bouillant, on obtient 1,4 g de [((benzisoxazol-1,2 yl-3)-4 pipérazinyl-1)-3 propyl]-2 naphto [1,8-cd] isothiazole dioxyde-1,1 fondant à 263°C.

La (benzisoxazol-1,2 yl-3)-4 pipérazine peut être préparée selon la méthode décrite par J.P.YEVICH et coll., J. Med. Chem., 29, 359 (1986).

### EXEMPLE 33

On opère comme à l'exemple 1, à partir de 5,3 g de (chloro-2 éthyl)-2 naphto [1,8-cd] isothiazole dioxyde-1,1, de 3 cm3 de triéthylamine, de 4 g de (benzisoxazol-1,2 yl-3)-4 pipérazine et de 3 g d'iodure de sodium dans 60 cm3 de diméthylformamide. Le mélange est chauffé 8 heures à ébullition puis refroidi à une température voisine de 20°C. Après purification par flash-chromatographie sur gel de silice, sous courant d'argon à moyenne pression (0,5-1,5 bar) avec de l'acétate d'éthyle comme éluant et recristallisation dans 22 cm3 d'acé- tonitrile bouillant, on obtient2,3 g de [((benzisoxazol-1,2 yl-3)-4 pipérazinyl-1)-2 éthyl]-2 naphto [1,8-cd] isothiazole dioxyde-1,1 fondant à 148°.

### EXEMPLE 34

On opère comme à l'exemple 1, à partir de 5,8 g de (chloro-4 butyl)-2 naphto [1,8-cd] isothiazole dioxyde-1,1, de 3 cm3 de triéthylamine et de 4 g de (benzisoxazol-1,2 yl-3)-4 pipérazine dans 55 cm3 de diméthylformamide. Le mélange est chauffé 8 heures à ébullition puis refroidi à une température voisine de 20°C. Après purification par flash-chromatographie sur colonne de silice, sous courant d'argon à moyenne pression (0,5-1,5 bar) avec de l'acétate d'éthyle comme éluant et recristallisation dans 15 cm3 d'acétonitrile bouillant, on obtient 2,2 g de [((benzisoxazol-1,2 yl-3)-4 pipérazinyl-1)-4 butyl]-2 naphto [1,8-cd] isothiazole dioxyde-1,1 fondant à 138°C.

### EXEMPLE 35

On opère comme à l'exemple 1, à partir de 11 g de (chloro-3 propyl)-2 naphto [1,8-cd] isothiazole dioxyde-1,1, de 11 cm3 de triéthylamine et de 8,6 g de (benzisothiazol-1,2 yl-3)-4 pipérazine dans 120 cm3 de diméthylformamide. Le mélange est chauffé 8 heures à ébullition puis refroidi à une température voisine de 20°C. Après purification par flash-chromatographie sur colonne de silice, sous courant d'argon à moyenne pression avec de l'acétate d'éthyle comme éluant et recristallisation dans 160 cm3 de méthyléthylcétone bouillante, on obtient 4,4 g de [((benzisothiazol-1,2 yl-3)-4 pipérazinyl-l)-3 propyl]-2 naphto [1,8-cd] isothiazole dioxyde-1,1 fondant à 172°-173°C.

### EXEMPLE 36

On opère comme à l'exemple 1, à partir de 8,9 g de (chloro-2 éthyl)-2 naphto [1,8-cd] isothiazole dioxyde-1,1, de 4,5 cm3 de triéthylamine et de 6,6 g de (benzisothiazol-1,2 yl-3)-4 pipérazine dans 95 cm3 de diméthylformamide. Le mélange est chauffé 8 heures à ébullition puis refroidi à une température voisine de 20°C. Après purification par flash-chromatographie sur colonne de silice, sous courant d'argon à moyenne pression (0,5-1,5 bar) avec de l'acétate d'éthyle comme éluant et recristallisation dans 90 cm3 d'acétonitrile bouillant, on obtient 4,6 g de [((benziso- thiazol-1,2 yl-3)-4 pipérazinyl-l)-2 éthyl]-2 naphto [1,8-cd] isothiazole dioxyde-1,1 fondant à 162°C.

### EXEMPLE 37

On opère comme à l'exemple 1, à partir de 8,9 g de (chloro-4 butyl)-2 naphto [1,8-cd] isothiazole dioxyde-1,1, de 4,5 cm3 de triéthylamine et de 6,6 g de (benzisothiazol-1,2 yl-3)-4 pipérazine dans 95 cm3 de diméthylformamide. Le mélange est chauffé 8 heures à ébullition puis refroidi à une température voisine de 20°C. Après purification par flash-chromatographie sur colonne de silice, sous courant d'argon à moyenne pression (0,5-1,5 bar) avec de l'acétate d'éthyle comme éluant et recristallisation dans 30 cm3 d'acétonitrile bouillant, on obtient 6,3 g de [((benzisothiazoi-1,2 yl-3)-4 pipérazinyi-1)-4 butyl]-2 naphto [1,8-cd] isothiazole dioxyde-1,1 fondant à 123°C.

### EXEMPLE 38

On opère comme à l'exemple 1, à partir de 8,9 g de (chloro-4 butyl)-2 naphto [1,8-cd] isothiazole dioxyde-1,1, de 4,3 cm3 de triéthylamine et de 5,8 g de (chloro-4 phényl)-4 tétrahydro-1,2,3,6 pyridine dans 80 cm3 de toluène. Le mélange est chauffé 8 heures à ébullition puis refroidi à une température voisine de 20°C. L'agitation est maintenue durant 8 heures à cette température. Après purification parflash-chromatographie sur colonne de silice, sous courant d'argon à moyenne pression (0,5-1,5 bar) avec de l'acétate d'éthyle comme éluant et recristallisation dans 25 cm3 d'acétonitrile bouillant, on obtient 4,1 g de [((chloro-4 phényl)-4 tétrahydro-1,2,3,6 pyridyl-1)-4 butyl]-2 naphto[1,8-cd] isothiazole dioxyde-1,1 fondant à 123°C.

### EXEMPLE 39

On opère comme à l'exemple 1, à partir de 11,2g de (chloro-3 propyl)-2 naphto [1,8-cd] isothiazole dioxyde-1,1, de 16,8 cm3 de triéthylamine et 13,6 g de dibromhydrate d'(hydroxy-3 phényl)-4 pipérazine dans 250 cm3 de diméthylformamide. Le mélange est chauffé 6 heures à ébullition puis refroidi à une température voisine de 20°C. L'agitation est maintenue durant 6 heures à cette température. Après purification par cristallisation dans 30 cm3 d'oxyde d'isopropyle puis recristallisation dans 125 cm3 d'acétonitrile bouillant, on obtient 4,6 g d'[((hydroxy-3 phényl)-4 pipérazinyl-1)-3 propyl]-2 naphto [1,8-cd] isothiazole dioxyde-1,1 fondant à 175°C.

Le dibromhydrate d'(hydroxy-3 phényl)-4 pipérazine peut être préparé de la manière suivante : on opère comme à l'exemple 8 pour la préparation du dibromhydrate d'(hydroxy-4 phényl)-4 pipérazine à partir de 25 g de (méthoxy-3 phényl)-4 pipérazine et de 360 cm3 d'une solution aqueuse d'acide bromhydrique à 47 %. Le mélange est chauffé à ébullition pendant 2 heures puis refroidi à une température voisine de 20°C. L'agitation est maintenue durant 15 heures à cette température puis le mélange est concentré à 40°C sous pression réduite (20 mm de mercure ; 2,7 kPa). L'huile résiduelle est reprise par 100 cm3 d'acétonitrile ; le précipité formé est séparé par f iltration, lavé par 2 fois 50 cm3 d'acétonitrile et 2 fois 50 cm3 d'oxyde d'isopropyle. On obtient 42,5 g de dibromhydrate d'(hydroxy-3 phényl)-4 pipérazine (point de fusion : 240°C) utilisé à l'état brut dans les synthèses ultérieures.

### EXEMPLE 40

On opère comme à l'exemple 1, à partir de 7,02 g de (chloro-3 propyl)-2 naphto [1,8-cd] isothiazole dioxyde-1,1, de 10,5 cm3 de triéthylamine et de 8,5 g de dibromhydrate d'(hydroxy-2 phényl)-4 pipérazine dans 150 cm3 de diméthylformamide. Le mélange est chauffé 6 heures à ébullition puis refroidi à une température voisine de 20°C. L'agitation est maintenue durant 15 heures à cette température. Après purification par flash-chromatographie sur colonne de silice, sous courant d'argon à moyenne pression (0,5-1,5 bar) avec un mélange de dichlorométhane et d'acétate d'éthyle (50-50 en volumes) comme éluant et recristallisation dans 25 cm3 d'acétonitrile, on obtient 5,8 g d'[((hydroxy-2 phényl)-4 pipérazinyl-1)-3 propyl]-2 naphto [1,8-cd] isothiazole dioxyde-1,1 fondant à 128°C.

Le dibromhydrate d'(hydroxy-2 phényl)-4 pipérazine peut être préparé de la manière suivante : on opère comme à l'exemple 8 pour la préparation du dibromhydrate d'(hydroxy-4 phényl)-4 pipérazine à partir de 25 g de (méthoxy-2 phényl)-4 pipérazine et de 360 cm3 d'une solution aqueuse d'acide bromhydrique à 47 %. Le mélange est chauffé à ébullition pendant 9 heures puis refroidi à une température proche de 20°C. L'agitation est maintenue durant 15 heures à cette température puis le mélange est concentré à 40°C sous pression réduite (20 mm de mercure ; 2,7 kPa). L'huile résiduelle est reprise par 40 cm3 d'acétonitrile et 40 cm3 d'oxyde d'iso- propyle; le précipité formé est séparé par filtration, lavé par 2 fois 50 cm3 d'oxyde d'isopropyle. On obtient 18 g de dibromhydrate d'(hydroxy-2 phényl)-4 pipérazine (point de fusion : 235°C) utilisé à l'état brut dans les synthèses ultérieures.

### EXEMPLE 41

On opère comme à l'exemple 1, à partir de 10,68 g de (chloro-2 éthyl)-2 naphto [1,8-cd] isothiazole dioxyde-1,1, de 5,6 cm3 de triéthylamine, de 6 g d'iodure de sodium et de 8,0 g d'(indolyl-3)-4 tétrahydro-1,2,3,6 pyridine dans 200 cm3 de diméthylformamide. Le mélange est chauffé 8 heures à ébullition puis refroidi à une température voisine de 20°C. L'agitation est maintenue durant 2 heures à cette température. Après purification par cristallisation dans 150 cm3 d'acétonitrile et recristallisation dans 150 cm3 de méthyléthylcétone bouillante, on obtient 0,8 g d'[((indolyl-3)-4 tétrahydro-1,2,3,6 pyridyl-1)-2 éthyl]-2 naphto [1,8-cd] isothiazole dioxyde-1,1 fondant à 208°C.

### EXEMPLE 42

On opère comme à l'exemple 1, à partir de 2,95 g de (chloro-4 butyl)-2 naphto [1,8-cd] isothiazole dioxyde-1,1, de 0,84 g de bicarbonate de sodium et de 1,98 g d'(indolyl-3)-4 tétrahydro-1,2,3,6 pyridine dans un mélange de 50 cm3 de diméthylformamide et de 50 cm3 de tétrahydrofuranne. Le mélange est chauffé 4 heures à ébullition puis refroidi à une température voisine de 20°C. L'agitation est maintenue durant 2 heures à cette température. Après purification par cristallisation dans 15 cm3 de méthyléthylcétone, flash-chromatographie sur colonne de silice, sous courant d'argon à moyenne pression (0,5-1,5 bar) avec du dichlorométhane puis de l'acétate d'éthyle comme éluant, recristallisation dans 65cm3 de méthyléthylcétone bouillante, on obtient 1,5 g d' [((indolyl-3)-4 tétrahydro-1,2,3,6 pyridyl-1)-4 butyl]-2 naphto [1,8-cd] isothiazole dioxyde-1,1 fondant à 203°C.

### EXEMPLE 43

On opère comme à l'exemple 1, à partir de 2,81 g de (chloro-3 propyl)-2 naphto [1,8-cd] isothiazole dioxyde-1,1, de 1,4 cm3 de triéthylamine et de 1,96 g de (chloro-4 phényl)-4 pipéridine dans 30 cm3 de toluène. Le mélange est chauffé 8 heures à ébullition puis refroidi à une température voisine de 20°C. L'agitation est maintenue durant 8 heures à cette température. Après purification par flash-chromatographie sur colonne de silice, sous courant d'argon à moyenne pression (0,5-1,5 bar) avec de l'acétate d'éthyle comme éluant et recristallisation une première fois dans 12 cm3 d'acétonitrile bouillant puis une seconde fois dans 17 cm3 d'acétonitrile bouillant, on obtient 1,4 g de [((chloro-4 phényl)-4 pipéridino)-3 propyl]-2 naphto [1,8-cd] isothiazole dioxyde-1,1 fondant à 111 °C.

La (chloro-4 phényl)-4 pipéridine peut être préparée selon la méthode décrite par L.GOOTJES et coll, Arzneim. Forsch., 17, 1145 (1967).

### EXEMPLE 44

A 1,3 g d'hydrure de sodium en dispersion à 50 % dans de l'huile de vaseline, sous courant d'argon, on ajoute, en 30 minutes, un mélange de 11 g d'[((indolyl-3)-4 tétrahydro-1,2,3,6 pyridyl-1)-3 propyl]-2 naphto[1,8- cd] isothiazole dioxyde-1,1 dans 150 cm3 de diméthylformamide, en maintenant la température voisine de 20°C. Le milieu réactionnel est agité 2 heures à une température voisine de 20°C puis on ajoute, en 15 minutes, une solution de 3,9 g d'iodure de méthyle dans 50 cm3 de dioxanne. L'agitation est maintenue durant 15 heures à une température voisine de 20°C. Le mélange réactionnel est repris par 250 cm3 d'eau distillée et la phase organique est extraite par 4 fois 100 cm3 de dichlorométhane puis lavée par 400 cm3 d'eau distillée, séchée sur du sulfate de magnésium anhydre, filtrée et concentrée à sec à 40°C sous pression réduite (20 mm de mercure ; 2,7 kPa). Le résidu obtenu est purifié parflash-chromatographie sur colonne de silice, sous courant d'argon, à moyenne pression (0,5-1,5 bar) avec du dichlorométhane comme éluant et recristallisé dans 70 cm3 de méthyléthylcétone bouillante. On obtient 3,3 g de [((méthyl-1 indolyl-3)-4 tétrahydro-1,2,3,6 pyridyl-1 )-3 pro- pyl]-2 naphto[1,8-cd] isothiazole dioxyde-1,1 1 fondant à 161°C.

### EXEMPLE 45

On opère comme à l'exemple 44, à partir de 1,3 g d'hydrure de sodium à 50 % en dispersion dans l'huile de vaseline, de 11 g d'[((indolyl-3)-4 tétrahydro-1,2,3,6 pyridyl-1)-3 propyl]-2 naphto[1,8-cd] isothiazole dioxyde-1,1 dans 150 cm3 de diméthylformamide et de 1,95 cm3 de chlorure d'acétyle dans 50 cm3 de dioxanne. Le mélange est agité 15 heures à une température voisine de 20°C. Après purification par flash-chromatographie sur colonne de silice, sous courant d'argon à moyenne pression (0,5-1,5 bar) une première fois avec du dichlorométhane comme éluant et une seconde fois avec de l'acétate d'éthyle comme éluant puis recristallisation dans 45 cm3 d'acétonitrile bouillant, on obtient 1,3 g d'[«acétyl-1 indolyl-3)-4 tétrahydro-1,2,3,6 pyridyl-1)-3 propyl]-2 naphto[1,8-cd] isothiazole dioxyde-1,1 fondant à 163°C.

### EXEMPLE 46

On opère comme à l'exemple 1, à partir de 2,8 g de (chloro-3 propyl)-2 naphto [1,8-cd] isothiazole dioxyde-1,1, de 2,1 g d'hydroxy-4 (méthyl-4 phényl)-4 pipéridine et de 0,94 g de bicarbonate de sodium dans un mélange de 60 cm3 de diméthylformamide et de 50 cm3 de tétrahydrofuranne. Le mélange est chauffé 5 heures à une température voisine de 100°C puis refroidi à une température voisine de 20°C. L'agitation est maintenue durant 15 heures à cette température. Après purification parflash-chromatographie sur colonne de silice, sous courant d'argon à moyenne pression (0,5-1,5 bar) avec un mélange de dichlorométhane et de méthanol (95-5 en volumes) comme éluant, on obtient 1 g de [((méthyl-4 phényl)-4 hydroxy-4 pipéridino)-3 propyl]-2 naphto [1,8- cd] isothiazole dioxyde-1,1 sous forme de chlorhydrate dont le point de fusion est de 218°C.

L'hydroxy-4 (méthyl-4 phényl)-4 pipéridine peut être préparé de la manière suivante : 5 g de benzyl-1 hydroxy-4 (méthyl-4 phényl)-4 pipéridine, 1 g de palladium à 5 % sur charbon et 100 cm3 de méthanol sont chargés dans un autoclave de 250 cm3. Le milieu réactionnel est hydrogéné sous pression (52 bars) à 50°C pendant 16 heures. Après refroidissement à une température proche de 20°C et purge à l'azote, le mélange est filtré sur verre fritté et lavé par 3 fois 50 cm3 de méthanol. Le filtrat est concentré à sec à 40°C sous pression réduite (20 mm de mercure ; 2,7 kPa). On obtient 3,4 g d'hydroxy-4 (méthyl-4 phényl)-4 pipéridine sous forme d'une huile brune, utilisée à l'état brut dans les synthèses ultérieures.

La benzyl-1 hydroxy-4 (méthyl-4 phényl)-4 pipéridine peut être préparée de la façon suivante : à 1,7 g de magnésium en tournures dans 25 cm3 d'éther diéthylique, sous courant d'argon, on ajoute quelques gouttes de p-bromotoluène et un cristal d'iode. Le mélange est chauffé à ébullition puis on coule une solution de 12,1 g de p-bromotoluène dans 70 cm3 d'éther diéthylique de telle façon que le reflux soit maintenu. Le milieu réactionnel est agité 30 minutes à l'ébullition du solvant puis refroidi à une température voisine de 20°C. On ajoute une solution de 6,7 g de benzyl-1 pipéridone dans 60 cm3 d'éther diéthylique et maintient l'agitation 15 heures à la même température. 150 cm3 d'une solution saturée de chlorure d'ammonium sont ajoutés au mélange ; la phase aqueuse est décantée et la phase organique est extraite par 2 fois 150 cm3 d'éther éthylique, séchée sur du sulfate de magnésium anhydre, filtrée et concentrée à sec à 40°C sous pression réduite (20 mm de mercure ; 2,7 kPa). Le résidu obtenu est purifié parflash-chromatographie sur colonne de silice, sous courant d'argon à moyenne pression (0,5-1,5 bar) avec un mélange de dichlorométhane et de méthanol (95-5 en volumes) comme éluant. On obtient 5 g de benzyl-1 hydroxy-4 (méthyl-4 phényl)-4 pipéridone sous forme d'une huile, utilisée à l'état brut dans les synthèses ultérieures.

### EXEMPLE 47

On opère comme à l'exemple 1, à partir de 6,3 g de (chloro-3 propyl)-2 naphto [1,8-cd] isothiazole dioxyde-1,1, de 4,5 g d'(indolyl-3)-4 pipéridine et de 1,9 g de bicarbonate de sodium dans un mélange de 100 cm3 de diméthylformamide et de 100 cm3 de tétrahydrofuranne. Le mélange est chauffé 5 heures à ébullition puis refroidi à une température voisine de 20°C. L'agitation est maintenue durant 15 heures à cette température. Après purification par flash-chromatographie sur colonne de silice, sous courant d'argon à moyenne pression (0,5-1,5 bar) avec de l'acétate d'éthyle comme éluant et recristallisation dans 60 cm3 de méthyléthylcétone bouillante, on obtient 2 g d'[((indolyl-3)-4 pipéridino)-3 propyl]-2 naphto [1,8-cd] isothiazole dioxyde-1,1 fondant à 162°C.

L'(indolyl-3)-4 pipéridine peut être préparée selon la méthode décrite par J.BERGMAN et coll., J. Het. Chem., 1071 (1970).

### EXEMPLE 48

On opère comme à l'exemple 1, à partir de 1,2 g de (chloro-2 éthyl)-2 naphto [1,8-cd] isothiazole dioxyde-1,1, de 3,4 g de dibromhydrate d'(hydroxy-4 phényl)-4 pipérazine et de 1,3 g de bicarbonate de sodium dans 30 cm3 de diméthylformamide. Le mélange est chauffé 4 heures à ébullition puis refroidi à une température voisine de 20°C. Après purification par flash-chromatographie sur colonne de silice, sous courant d'argon à moyenne pression (0,5-1,5 bar) avec de l'acétate d'éthyle comme éluant, et recristallisation dans 90cm3 d'acé- tonitrile bouillante, on obtient 1,6 g d'[((hydroxy-4 phényl)-4 pipérazinyl-1)-2 éthyl]-2 naphto [1,8-cd] isothiazole dioxyde-1,1 fondant à 235°C.

### EXEMPLE 49

On opère comme à l'exemple 1, à partir de 3 g de (chloro-4 butyl)-2 naphto [1,8-cd] isothiazole dioxyde-1,1, de 3,8 g de dibromhydrate d'(hydroxy-4 phényl)-4 pipérazine et de 2,8 g de bicarbonate de sodium dans un mélange de 30 cm3 de diméthylformamide et de 20 cm3 de tétrahydrofuranne. Le mélange est chauffé 5 heures à ébullition puis refroidi à une température voisine de 20°C. Après purification par recristallisation dans 215 cm3 d'acétone bouillante, on obtient 3,1 g d'[((hydroxy-4 phényl)-4 pipérazinyi-1)-4 butyl]-2 naphto [1,8- cd] isothiazole dioxyde-1,1 fondant à 195°C.

### EXEMPLE 50

On opère comme à l'exemple 1, à partir de 2,1 g de (chloro-3 propyl)-2 naphto [1,8-cd] isothiazole dioxyde-1,1, de 0,6 g de bicarbonate de sodium et 1,6 g de (fluoro-5 indolyl-3)-4 tétrahydro-1,2,3,6 pyridine dans 15cm3 de diméthylformamide et 15 cm3 de tétrahydrofuranne. Le mélange est chauffé 5 heures à ébullition puis refroidi à une température voisine de 20°C. L'agitation est maintenue durant 7 heures à cette température. Après purification par cristallisation avec 50 cm3 d'eau puis recristallisation dans 50 cm3 d'un mélange acétone-éthanol bouillant (50-50 en volumes),on obtient 1,8 g de [((fluoro-5 indolyl-3)-4 tétrahydro-1,2,3,6 pyridyl-l)-3 pro- pyl]-2 naphto [1,8-cd] isothiazole dioxyde-1,1 fondant à 224°C.

La (fluoro-5 indolyl-3)-4 tétrahydro-1,2,3,6 pyridine peut être préparée selon la méthode décrite par L.NEDELEC et coll., Eur. J. Med. Chem., 22, 33 (1987). Elle présente un point de fusion de 175°C.

### EXEMPLE 51

On opère comme à l'exemple 1, à partir de 2,4 g de (chloro-3 propyl)-2 naphto [1,8-cd] isothiazole dioxyde-1,1, de 0,7g de bicarbonate de sodium et de 2 g de (chloro-5 indolyl-3)-4 tétrahydro-1,2,3,6 pyridine dans 15 cm3 de diméthylformamide et 15 cm3 de tétrahydrofuranne. Le mélange est chauffé 5 heures à ébullition puis refroidi à une température voisine de 20°C. L'agitation est maintenue durant 7 heures à cette température. Après purification par cristall isation avec 50 cm3 d'eau puis recristallisation dans 50 cm3 d'acétonitrile bouillant, on obtient 2,1 g de[((chloro-5 indolyl-3)-4 tétrahydro-1,2,3,6 pyridyl-1)-3 propyl]-2 naphto [1,8-cd] isothiazole dioxyde-1,1 fondant à 189°C.

La (chloro-5 indolyl-3)-4 tétrahydro-1,2,3,6 pyridine peut être préparée selon la méthode décrite par L. NEDELEC et coll, Eur.J.Med.Chem., 22,33 (1987). Elle présente un point de fusion de 220°C.

### EXEMPLE 52

On opère comme à l'exemple 1, à partir de 7 g de (chloro-3 propyl)-2 naphto [1,8-cd] isothiazole dioxyde-1,1, de 4,2 g de bicarbonate de sodium et de 5,3g d'(hydroxy-5 indolyl-3)-4 tétrahydro-1,2,3,6 pyridine dans 50 cm3 de diméthylformamide et 50 cm3 de tétrahydrofuranne. Le mélange est chauffé 5 heures à ébullition puis refroidi à une température voisine de 20°C. L'agitation est maintenue durant 7 heures à cette température. Après purification par cristallisation avec 100cm3 d'eau puis recristallisation par 80 cm3 d'un mélange acétone-éthanol bouillant (50-50 en volumes), on obtient 5,1 g d'[(hydroxy-5 indolyl-3)-4 tétrahydro-1,2 ,3,6 pyridyl-1)-3 propyl]-2 naphto [1,8-cd] isothiazole dioxyde-1,1 fondant à 214°C.

L'(hydroxy-5 indolyl-3)-4 tétrahydro-1,2,3,6 pyridine peut être préparée selon la méthode décrite par L.NEDELEC et coll., Eur.J.Med.Chem., 22,33 (1987). Elle présente un point de fusion de 185°C.

### EXEMPLE 53

On opère comme à l'exemple 44, à partir de 0,48 g d'hydrure de sodium à 50 % en dispersion dans l'huile de vaseline, de 4,6 g d'[(oxo-2 benzimidazolinyl-1)-4 pipéridino)-3 propyl]-2 naphto[1,8-cd] isothiazole dioxyde-1,1 dans 20 cm3 de diméthylformamide et de 1,4 g de chlorure de benzoyle dans 20 cm3 de tétrahydrofuranne. Le mélange est agité 15 heures à une température voisine de 20°C. Après purification par flash-chromatographie sur colonne de silice, sous courant d'argon à moyenne pression (0,5-1,5 bar) avec comme éluant un mélange dichlorométhane-méthanol (90-10 en volumes) puis recristallisation par 60 cm3 d'un mélange acétone-éthanol bouillant (90-10 en volumes), on obtient 2,9 g de [((benzoyl-3 oxo-2 benzimidazolinyl-1) -4 pipéridino)-3 propyl]-2 naphto[1,8-cd] isothiazole dioxyde-1,1 fondant à 133°C.

### EXEMPLE 54

On opère comme à l'exemple 1, à partir de 2,8 g de (chloro-3 propyl)-2 naphto [1,8-cd] isothiazole dioxyde-1,1, de 2,4 g d'hydroxy-4 (bromo-4 phényl)-4 pipéridine et de 0,94 g de bicarbonate de sodium dans un mélange de 80 cm3 de diméthylformamide et de 50 cm3 de tétrahydrofuranne. Le mélange est chauffé 5 heures à une température voisine de 100°C puis refroidi à une température voisine de 20°C. L'agitation est maintenue durant 15 heures à cette température. Après purification parflash-chromatographie sur colonne de silice, sous courant d'argon (0,5-1,5 bar) avec un mélange de dichlorométhane et de méthanol (95-5 en volumes) comme éluant et recristallisation dans 50 cm3 d'acétate d'éthyle bouillant, on obtient 1,5 g de [(bromo-4 phényl)-4 hydroxy-4 pipéridino)-3 propyl]-2 naphto [1,8-cd] isothiazole dioxyde-1,1 sous forme de chlorhydrate dont le point de fusion est de 110°C.

L'hydroxy-4 (bromo-4 phényl)-4 pipéridine peut être préparé selon la méthode décrite dans le brevet américain 3,575,990.

### EXEMPLE 55

Un mélange de 3,7 g de [((bromo-4 phényl)-4 hydroxy-4 pipéridino)-3 propyl]-2 naphto[1,8-cd] isothiazole dioxyde-1,1, de 25 cm3 d'acide chlorhydrique 12 N et de 50 cm3 d'acide acétique est porté au reflux sous agitation pendant 16 heures. La solution est ensuite concentrée à sec à 60°C sous pression réduite (0,5 mm de mercure ; 0,07 kPa). Le brut réactionnel est repris par 25 cm3 d'eau et neutralisé par du bicarbonate de sodium jusqu'à pH 7. L'extraction de cette phase aqueuse par 3 fois 50 cm3 de dichlorométhane puis séchage au sulfate de magnésium conduit à une huile brune qui est purifiée par flash-chromatographie sur colonne de silice, sous courant d'argon (0,5-1,5 bar) avec un mélange de dichlorométhane et de méthanol (98-2 en volumes) comme éluant. Après recristallisation dans 30 cm3 d'éthanol bouillant on obtient 1,5g de [((bromo-4 phényl)-4 tétrahydro-1,2,3,6 pyridyl-1)-3 propyl]-2 naphto[1,8-cd] isothiazole dioxyde-1,1 fondant à 105°C.

### EXEMPLE 56

26,8 g de (bromo-3 hydroxy-2 propyl)-2 naphto [1,8-cd] isothiazole dioxyde-1,1, 11 cm3 de triéthylamine et 14,1 g de (fluoro-4 phényl)-4 pipérazine dans 280 cm3 de toluène, sont chauffés pendant 8 heures à ébullition. Le mélange est ensuite refroidi à une température voisine de 20°C et l'agitation est maintenue durant 15 heures à cette température. Le précipité formé est séparé par filtration, lavé par 2 fois 50 cm3 de toluène. Le filtrat est évaporé à sec à 40°C sous pression réduite (20 mm de mercure ; 2,7 kPa). Le résidu obtenu est purifié par chromatographie sur colonne de silice, avec de l'acétate d'éthyle comme éluant et recristallisé dans 120 cm3 d'acétonitrile bouillant. On obtient 16,2 g de [((fluoro-4 phényl)-4 pipérazinyl-1)-3 hydroxy-2 propyl]-2 naphto [1,8-cd] isothiazole dioxyde-1,1 fondant à 130°C.

Le (bromo-3 hydroxy-2 propyl)-2 naphto [1,8-cd] isothiazole dioxyde-1,1 peut être préparé de la manière suivante : à une suspension de 9,6 g d'hydrure de sodium en dispersion à 50 % dans de l'huile de vaseline dans 100 cm3 de diméthylformamide, sous courant d'argon, on ajoute, en 2 heures, en maintenant la température entre 20 et 35°C, une solution de 41 g de naphto [1,8-cd] isothiazole dioxyde-1,1 dans 1000 cm3 de diméthylformamide. Le milieu réactionnel est agité 30 minutes à une température voisine de 20°C puis, on ajoute, en 10 minutes, 20,5 cm3 de dibromo-1,3 hydroxy-2 propane dans 500 cm3 de diméthylformamide. L'agitation est maintenue durant 15 heures à une température voisine de 20°C. Le mélange réactionnel est concentré à sec à 70°C sous pression réduite (0,5 mm de mercure ; 0,07 kPa). Le résidu est purifié par chromatographie sur colonne de silice, sous courant d'argon avec un mélange de dichlorométhane et de méthanol (95-5 en volumes) comme éluant. On obtient 62,1 g de (bromo-3 hydroxy-2 propyl)-2 naphto [1,8-cd] isothiazole dioxyde-1,1, sous forme d'une huile verte, utilisé à l'état brut dans les synthèses ultérieures.

### EXEMPLE 57

A un mélange de 0,53 g d'hydrure de sodium en dispersion à 50 % dans l'huile de vaseline et de 10cm3 de diméthylformamide, sous courant d'argon, on coule, en 30 minutes, une solution de 4,41 g de [((fluoro-4 phényl)-4 pipérazinyl-1)-3 hydroxy-2 propyl]-2 naphto [1,8-cd] isothiazole dioxyde-1,1 dans 50 cm3 de diméthylformamide, en maintenant la température entre 20 et 30°C. Le milieu réactionnel est agité 1 heure à une température voisine de 20°C puis on ajoute, en 10 minutes, 0,7 cm3 d'iodure de méthyle. L'agitation est maintenue durant 15 heures à une température voisine de 20°C. Le mélange est repris par 200 cm3 d'eau distillée et la phase organique est extraite par 4 fois 100 cm3 de dichlorométhane, séchée sur du sulfate de magnésium anhydre, filtrée et concentrée à sec à 40°C sous pression réduite (20 mm de mercure ; 2,7 kPa). Le résidu est purifié parflash-chromatographie sur colonne de silice, sous courant d'argon à moyenne pression (0,5-1,5 bar) avec de l'acétate d'éthyle comme éluant et recristallisé dans 10 cm3 d'acétonitrile bouillant. On obtient 2,1 g de [((fluoro-4 phényl)-4 pipérazinyl-1)-3 méthoxy-2 propyl]-2 naphto [1,8-cd] isothiazole dioxyde-1,1 fondant à 128°C.

### EXEMPLE 58

4,2 g de [bromo-2 ((fluoro-4 phényl)-4 pipérazinyl-1)-3 propyl]-2 naphto [1,8-cd] isothiazole dioxyde-1,1, 1,1 cm3 de triéthylamine et 0,5 cm3 de diméthylamine dans 50 cm3 de toluène sont introduits dans un autoclave de 250 cm3 refroidi à une température voisine de 0°C. Le mélange est agité et chauffé 8 heures à une température proche de 120°C puis refroidi à une température de 20°C environ. L'agitation est maintenue 15 heures à cette température puis le mélange réactionnel est concentré à sec à 40°C sous pression réduite (20 mm de mercure ; 2,7 kPa). L'huile résiduelle est purifiée parflash-chromatographie sur colonne de silice, sous courant d'argon à moyenne pression (0,5-1,5 bar)avec de l'acétate d'éthyle comme éluant. Après recristallisation dans 15 cm3 d'acétonitrile bouillant, on obtient 1,9 g de [diméthylamino-2 ((fluoro-4 phényl)-4 pipérazinyl-1)-3 pro- pyl]-2 naphto (1,8-cd] isothiazole dioxyde-1,1 fondant à 118°C.

Le [bromo-2 ((fluoro-4 phényl)-4 pipérazinyl-1)-3 propyl]-2 naphto [1,8-cd] isothiazole dioxyde-1,1 peut être préparé de la manière suivante : à 11,5 g de [((fluoro-4 phényl)-4 pipérazinyl-1)-3 hydroxy-2 propyl]-2 naphto [1,8-cd] isothiazole dioxyde-1,1 dans 150 cm3 de toluène, on coule 1 cm3 de tribromure de phosphore. Le mélange est chauffé 4 heures à ébullition puis est refroidi à une température voisine de 20°C. Le mélange réactionnel est repris par 300 cm3 d'eau distillée et alcalinisé à pH 11 par 20 cm3 de soude N. La phase organique est extraite par 4 fois 300 cm3 de dichlorométhane, séchée sur du sulfate de magnésium anhydre, filtrée et concentrée à sec à 40°C sous pression réduite (20 mm de mercure ; 2,7 kPa). L'huile résiduelle est purifiée par flash-chromatographie sur colonne de silice, sous courant d'argon à moyenne pression (0,5-1,5 bar) avec de l'acétate d'éthyle comme éluant. On obtient 5,7 g de [bromo-2 ((fluoro-4 phényl)-4 pipérazinyl-1)-3 propyl]-2 naphto [1,8-cd] isothiazole dioxyde-1,1 fondant à 102°C, utilisé à l'état brut dans les synthèses ultérieures.

### EXEMPLE 59

On opère comme à l'exemple 56, à partir de 10,4 g de (chloro-3 méthyl-2 propyl)-2 naphto [1,8-cd] isothiazole dioxyde-1,1, de 5,1 cm3 de triéthylamine, de 5,4 g d'iodure de sodium et de 6,5 g de (fluoro-4 phényl)-4 pipérazine dans 130 cm3 de toluène. Le mélange est chauffé 16 heures à ébullition puis refroidi à une température voisine de 20°C. Après purification par flash-chromatographie sur colonne de silice, sous courant d'argon à moyenne pression (0,5-1,5 bar) avec du dichlorométhane puis de l'acétate d'éthyle comme éluants et recristallisation dans 20 cm3 d'acétonitrile bouillant, on obtient 3,1 g de [((fluoro-4 phényl)-4 pipérazinyl-1)-3 méthyl-2 propy]-2 naphto [1,8-cd] isothiazole dioxyde-1,1 fondant à 133°C.

Le (chloro-3 méthyl-2 propyl)-2 naphto [1,8-cd] isothiazole dioxyde-1,1 peut être préparé de la manière suivante : on opère comme à l'exemple 56 pour la préparation du (bromo-3 hydroxy-2 propyl)-2 naphto [1,8- cd] isothiazole dioxyde-1,1, à partir de 2,4 g d'hydrure de sodium en dispersion à 50 % dans l'huile de vaseline, de 5,9 cm3 de bromo-1 chloro-3 méthyl-2 propane et de 10,3 g de naphto [1,8-cd] isothiazole dioxyde-1,1 dans 125 cm3 de diméthylformamide. Après purification parflash-chromatographie sur colonne de silice, sous courant d'argon à moyenne pression (0,5-1,5 bar) avec un mélange de dichlorométhane et de cyclohexane (80-20 en volumes) comme éluant, on obtient 10,4 g de (chloro-3 méthyl-2 propyl)-2 naphto [1,8-cd] isothiazole dioxyde-1,1, sous forme d'une huile verte, utilisé à l'état brut dans les synthèses ultérieures.

### EXEMPLE 60

A un mélange de 0,76 g d'hydrure de sodium en dispersion à 50 % dans l'huile de vaseline et de 10 cm3 de diméthylformamide , sous courant d'argon, on coule, en 30 minutes, une solution de 3,25 g de naphto [1,8- cd] isothiazole dioxyde-1,1 dans 50 cm3 de diméthylformamide. Le milieu réactionnel est agité 2 heures à une température voisine de 20°C puis on ajoute, en 10 minutes, 5 g de (bromo-4 butyl-2)-1 (fluoro-4 phényl)-4 pipérazine dans 100 cm3 de diméthylformamide. Le mélange réactionnel est agité 15 heures à une température proche de 20°C puis concentré à sec à 40°C sous pression réduite (20 mm de mercure ; 2,7 kPa). L'huile résiduelle est purifiée parflash-chromatographie sur colonne de silice, sous courant d'argon à moyenne pression (0,5-1,5 bar) avec un mélange de dichlorométhane et d'acétate d'éthyle (80-20 en volumes) comme éluant. Après recristallisation dans 13 cm3 d'acétonitrile bouillant, on obtient 1,8 g de [((fluoro-4 phényl)-4 pipérazinyl-1)-3 butyl]-2 naphto [1,8-cd] isothiazole dioxyde-1,1 fondant à 122°C.

Le (bromo-4 butyl-2)-1 (fluoro-4 phényl)-4 pipérazine peut être préparé de la manière suivante : à 12,7 g d'(hydroxy-4 butyl-2)-1 (fluoro-4 phényl)-4 pipérazine dans 200 cm3 de toluène, on coule 1,6 cm3 de tribromure de phosphore. Le mélange est chauffé 1 heure à une température voisine de 60°C puis est refroidi à une température proche de 20°C. Le mélange réactionnel est repris par 200 cm3 d'eau distillée et alcalinisé à pH 11 par 30 cm3 de soude N. La phase organique est extraite par 2 fois 100 cm3 de dichlorométhane, séchée sur du sulfate de magnésium anhydre, filtrée et concentrée à sec à 40°C sous pression réduite (20 mm de mercure ; 2,7 kPa). L'huile résiduelle est purifiée par flash-chromatographie sur colonne de silice, sous courant d'argon à moyenne pression (0,5-1,5 bar) avec un mélange d'acétate d'éthyle et de méthanol (50-50 en volumes) comme éluant. On obtient 5 g de (bromo-4 butyl-2)-1 (fluoro-4 phényl)-4 pipérazine, sous forme d'une huile jaune pâle, utilisé à l'état brut dans les synthèses ultérieures.

L'(hydroxy-4 butyl-2)-1 (fluoro-4 phényl)-4 pipérazine peut être préparé de la manière suivante : 29 g de bromo-3 butanol, 26,6 cm3 de triéthylamine et 34 g de (fluoro-4 phényl)-4 pipérazine dans 800 cm3 de toluène sont chauffés à ébullition pendant 8 heures. Le mélange est ensuite refroidi à une température voisine de 20°C et l'agitation est maintenue durant 15 heures à cette température. Le précipité formé est séparé par filtration et lavé par 2 fois 50 cm3 de toluène. Le filtrat est repris par 400 cm3 d'eau distillée, la phase organique est décantée, séchée sur du sulfate de magnésium anhydre et concentrée à sec à 40°C sous pression réduite (20 mm de mercure ; 2,7 kPa). L'huile résiduelle est purifiée par flash-chromatographie sur colonne de silice, sous courant d'argon à moyenne pression (0,5-1,5 bar) avec de l'acétate d'éthyle puis un mélange d'acétate d'éthyle et de méthanol (95-5 en volumes) comme éluant. On obtient 12,7 g d'(hydroxy-4 butyl-2)-1 (fluoro-4 phényl)-4 pipérazine, sous forme d'une huile jaune, utilisé à l'état brut dans les synthèses ultérieures.

Le bromo-3 butanol peut être préparé selon la méthode décrite par D.A. PALAVANDISHVILI et coll., Chem.abstr., 70, 106089.

### EXEMPLE 61

On opère comme à l'exemple 56, à partir de 5,2 g de (bromo-4 butyl-2)-2 naphto [1,8-cd] isothiazole dioxyde-1,1, de 2,1 cm3 de triéthylamine et de 2,8 g de (fluoro-4 phényl)-4 pipérazine dans 200 cm3 de toluène. Le mélange est chauffé 8 heures à ébullition puis refroidi à une température voisine de 20°C. Après purification par flash-chromatographie sur colonne de silice, sous courant d'argon à moyenne pression (0,5-1,5 bar) avec un mélange de dichlorométhane et d'acétate d'éthyle (90-10 en volumes) comme éluant, cristallisation dans 30 cm3 d'oxyde d'isopropyle et recristallisation dans 10 cm3 d'acétonitrile bouillant, on obtient 1,2 g de [((fluoro-4 phényl)-4 pipérazinyl-1)-4 butyl-2]-2 naphto [1,8-cd] isothiazole dioxyde-1,1 fondant à 116°C.

Le (bromo-4 butyl-2)-2 naphto [1,8-cd] isothiazole dioxyde-1,1 peut être préparé de la manière suivante : à 16,1 g d'(hydroxy-4 butyl-2)-2 naphto [1,8-cd] isothiazole dioxyde-1,1 dans 250 cm3 de toluène, on coule 1,8 cm3 de tribromure de phosphore. Le mélange est chauffé 2 heures à une température voisine de 60°C puis refroidi à une température proche de 20°C. Le mélange réactionnel est repris par 200 cm3 d'eau distillée, la phase organique est extraite par 3 fois 50 cm3 de toluène, séchée sur du sulfate de magnésium anhydre, filtrée et concentrée à sec à 40°C sous pression réduite (20 mm de mercure ; 2,7 kPa). On obtient 5,2 g de (bromo-4 butyl-2)-2 naphto [1,8-cd] isothiazole dioxyde-1,1, sous forme d'une huile brune, utilisé à l'état brut dans les synthèses ultérieures.

L'(hydroxy-4 butyl-2)-2 naphto [1,8-cd] isothiazole dioxyde-1,1 peut être préparé de la manière suivante : on opère comme à l'exemple 56 pour la préparation du (bromo-3 hydroxy-2 propyl-1)-2 naphto [1,8-cd] isothiazole dioxyde-1,1, à partir de 11,6 g d'hydrure de sodium à 50 % en dispersion dans de l'huile de vaseline, de 37,1 g de bromo-2 butanol et de 49,6 g de naphto [1,8-cd] isothiazole dioxyde-1,1 dans 350 cm3 de diméthylformamide. Après purification parflash-chromatographie sur colonne de silice, sous courant d'argon à moyenne pression (0,5-1,5 bar) avec un mélange de dichlorométhane et d'acétate d'éthyle (50-50 en volumes) comme éluant, on obtient 16,1 g d'(hydroxy-4 butyl-2)-2 naphto [1,8-cd] isothiazole dioxyde-1,1, sous forme d'une huile brune, utilisé à l'état brut dans les synthèses ultérieures.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles pour le traitement des affections où la sérotonine est impliquée et notamment les affections du système nerveux central, du système cardiovasculaire et les troubles intestinaux. Ils sont, en particulier, utiles pour le traitement de l'anxiété, des troubles du sommeil, de la dépression, des psychoses et notamment de la schizophrénie, de la migraine, de l'asthme, de l'hypertension et de l'urticaire, comme analgésiques et comme inhibiteurs de l'agrégation plaquettaire.

Les doses dépendent de l'effet recherché, de la durée du traitement et de la voie d'administration utilisée; elles sont généralement comprises entre 10 et 300 mg par jour par voie orale pour un adulte avec des doses unitaires allant de 2 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### Exemple A

On prépare, selon la technique habituelle, des gélules dosées à 50 mg de produit actif ayant la composition suivante :

- [((fluoro-4 phényl)-4 pipérazinyl-1)-3 hydroxy-2

propyl]-2 naphto [1,8-cd] isothiazole dioxyde-1,1 .... 50 mg
- cellulose ............................................ 18 mg
- lactose .............................................. 55 mg
- silice, sous courant d'argon colloïdale .............. 1 mg
- carboxyméthylamidon sodique .......................... 10 mg
- talc ................................................. 10 mg
- stéarate de magnésium ................................ 1 mg

### Exemple B

On prépare, selon la technique habituelle, des comprimés dosés à 50 mg de produit actif ayant la composition suivante :
- [((fluoro-4 phényl)-4 pipérazinyl-1)-3 méthoxy-2

propyl]-2 naphto [1,8-cd] isothiazole dioxyde-1,1 .... 50 mg
- lactose .............................................. 104 mg
- cellulose ............................................ 40 mg
- polyvidone ........................................... 10 mg
- carboxyméthylamidon sodique .......................... 22 mg
- talc ................................................. 10 mg
- stéarate de magnésium ................................ 2 mg
- silice, sous courant d'argon colloïdale ............. 2 mg
- mélange d'hydroxyméthylcellulose, glycérine, oxyde de titane (72-3,5-24,5) .................... q. s. p. 1 comprimé pelliculé terminé à 245 mg

### Exemple C

On prépare une solution injectable contenant 10 mg de produit actif ayant la composition suivante :
- [diméthylamino-2 ((fluoro-4 phényl)-4 pipérazinyl-1)-3

propyl]-2 naphto [1,8-cd] isothiazole dioxyde-1,1 .... 10 mg
- acide benzoïque ...................................... 80 mg
- alcool benzylique .................................... 0,06 cm3
- benzoate de sodium ................................... 8O mg
- éthanol à 95 % ....................................... 0,4 cm3
- hydroxyde de sodium .................................. 24 mg
- propylène glycol ..................................... 1,6 cm3
- eau ........................................ q. s. p. 4 cm3

## Revendications (Revendications pour l'(les) Etat(s) contractant(s) suivant(s) : suivants : AT, BE, CH, DE, FR, GB, IT, LI, LU, NL, SE)

1. Composés de formule : 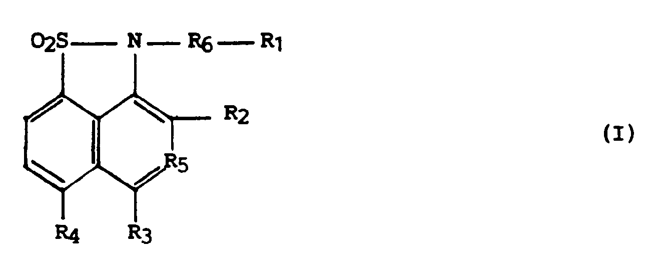
- R₁ représente
. un radical tétrahydro-1,2,3,6 pyridyl-1 substitué en position -4 par (a) un radical phényle, (b) un radical phényle substitué par un atome d'halogène ou un radical alkyle, hydroxy ou alcoxy, (c) un radical indolyl-3, (d) un radical indolyl-3 substitué sur l'atome d'azote par un radical alkyle ou alkylcarbonyle et/ou en position -5 par un atome de chlore ou de fluor ou (e) un radical (hydroxy-5 indolyl)-3,
. un radical pipérazinyl-1 substitué en position -4 par (a) un radical phényle, (b) un radical phényle substitué par un radical alcoxy, alkyle, hydroxy, nitro, amino ou un atome d'halogène, (c) un radical benzisothiazol-1,2 yl-3, (d) un radical benzisoxazol-1,2 yl-3 ou (e) un radical pyridyl-2,
. un radical pipéridino substitué en position -4 par (a) un radical phényle, (b) un radical phényle substitué par un atome d'halogène ou un radical hydroxy, alkyle ou alcoxy, (c) deux radicaux phényle, (d) un radical bis (fluoro-4 phényl) méthylène, (e) un radical fluoro-4 benzoyle, (f) un radical oxo-2 benzimidazolinyl-1, (g) un radical oxo-2 benzimidazolinyl -1 substitué en position -3 par un radical alkylcarbonyle ou benzoyle, (h) un radical hydroxy et un radical phényle éventuellement substitué par un radical alkyle, alcoxy, hydroxy ou un atome d'halogène, (i) un radical indolyl-3, (j) un radical indolyl-3 substitué sur l'atome d'azote par un radical alkyle ou alkylcarbonyle et/ou en position -5 par un atome de chlore ou de fluor ou (k) un radical (hydroxy-5 indolyl)-3,
- soit :
. R₂ et R₃ identiques représentent un atome d'hydrogène ou d'halogène et R₄ représente un atome d'hydrogène ou
. R₂ et R₄ représentent un atome d'hydrogène et R₃ représente un atome d'halogène ou un radical acétylamino ou
. R₂ et R₃ représentent un atome d'hydrogène et R₄ représente un atome d'halogène et R₅ représente un groupe -CH=,
- soit R₂, R₃ et R₄ représentent un atome d'hydrogène et R₅ représente un atome d'azote,
R₆ représente une chaîne alkylène contenant 2 à 4 atomes de carbone ou une chaîne propylène substituée en position -1 ou -3 par un radical alkyle ou en position -2 par un radical alkyle, alcoxy, hydroxy, dialkylamino, pipéridino, morpholino ou thiomorpholino, sous réserve que lorsque R₆ représente un radical propylène substitué en position -2 par un radical dialkylamino, pipéridino, morpholino ou thiomorpholino, R₁ ne peut pas être un radical comportant un radical hydroxy et étant entendu que les radicaux alkyle et alcoxy et les portions alkyle et alcoxy contiennent 1 à 4 atomes de carbone en chaîne droite ou ramifiée ainsi que leurs sels avec les acides minéraux ou organiques.

2. Composés de formule (I) selon la revendication 1 pour lesquels les atomes d'halogène sont les atomes de chlore ou de brome.

3. Composés de formule (I) selon la revendication 1 pour lesquels R₁ représente un radical tétrahydro-1,2,3,6 pyridyl-1 substitué en position -4 par un radical halogénophényle, phényle ou indolyl-3 substitué sur l'atome d'azote par un radical alkyle ou alkylcarbonyle, un radical pipérazinyl-1 substitué en position -4 par un radical pyridyl-2, benzisothiazol-1,2 yl-3 ou phényle substitué par un atome d'halogène ou un radical hydroxy, amino ou alkyle ou un radical pipéridino substitué en position -4 par un radical phényle ou N-alkylindolyl-3.

4. Procédé de préparation des composés de formule (I) selon la revendication 1 à l'exception de ceux pour lesquels R₆ représente un radical propylène substitué en position -2 par un radical dialkylamino, pipéridino, morpholino ou thiomorpholino et/ou R₁ représente un radical aminophényl-4 pipérazinyl-1 caractérisé en ce que l'on fait réagir un dérivé halogéné de formule : 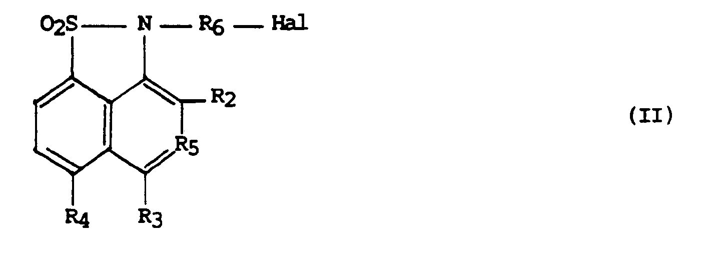 dans laquelle Hal représente un atome d'halogène et R₂, R₃, R₄, R₅ et R₆ ont les mêmes significations que dans la revendication 1 excepté que R₆ ne peut pas représenter un radical propylène substitué en position -2 par dialkylamino, pipéridino, morpholino ou thiomorpholino, sur un dérivé de formule : 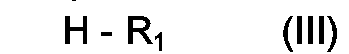 dans laquelle R₁ a les mêmes signification que dans la revendication 1 à l'exception d'être un radical aminophényl-4 pipérazinyl-1, isole le produit et le transforme éventuellement en sel avec un acide minéral ou organique.

5. Procédé de préparation des composés de formule (I) selon la revendication 1 pour lesquels
- soit R₅ représente un groupe =CH-, R₂, R₃ et R₄ représentent un atome d'hydrogène ou R₂ et R₄ représentent un atome d'hydrogène et R₃ représente un atome d'halogène ou R₂ et R₃ représentent un atome d'hydrogène et R₄ représente un atome d'halogène ou R₂ et R₃ représentent un atome d'halogène et R₄ représente un atome d'hydrogène,
- soit R₅ représente un atome d'azote et R₂, R₃ et R₄ représentent un atome d'hydrogène, R₆ représente un radical alkylène (2-4 C) ou propylène substitué en position -2 par un radical hydroxy, alkyle ou alcoxy exceptés ceux pour lesquels R₁ est un radical aminophényl-4 pipérazinyl-1 caractérisé en ce que l'on fait réagir un composé de formule : 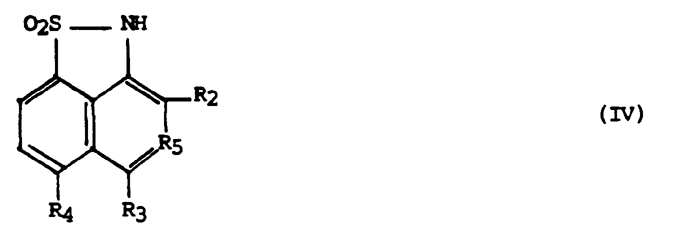 dans laquelle R₂, R₃, R₄ et R₅ ont les significations mentionnées ci-dessus sur un dérivé de formule : 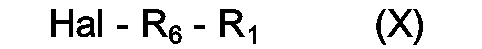 dans laquelle R₆ et R₁ ont les mêmes significations que ci-dessus et Hal représente un atome d'halogène, isole le produit et le transforme éventuellement en sel avec un acide minéral ou organique.

6. Procédé de préparation des composés de formule (I) selon la revendication 1 pour lesquels R₆ représente un radical propylène substitué en position -2 par un radical alcoxy, à l'exception de ceux pour lesquels R₁ représente un radical tétrahydro-1,2, 3,6 pyridyl-1 substitué en position -4 par un radical indolyl-3, indolyl-3 substitué en position -5 par un radical hydroxy ou hydroxyphényle, pipérazinyl-1 substitué en position -4 par un radical amino ou hydroxyphényle ou pipéridino substitué en position -4 par un radical oxo-2 benzimidazolinyl-1, hydroxyphényle, hydroxy et phényle éventuellement substitué par un radical hydroxy ou indolyl-3 éventuellement substitué en position -5 par un radical hydroxy, caractérisé en ce que l'on alkyle un composé de formule (I) correspondant pour lesquels R₆ représente un radical propylène substitué en position -2 par un radical hydroxy, isole le produit et le transforme éventuellement en sel avec un acide minéral ou organique.

7. Procédé de préparation des composés de formule (I) selon la revendication 1 pour lesquels R₆ représente un radical propylène substitué en position -2 par un radical dialkylamino, pipéridino, morpholino ou thiomorpholino caractérisé en ce que l'on fait réagir un dérivé de formule : 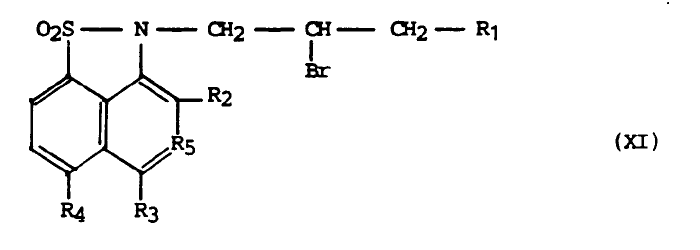 dans laquelle R₁, R₂, R₃, R₄ et R₅ ont les mêmes significations que dans la revendication 1 sur un dérivé de formule : 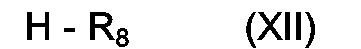 dans laquelle R₈ représente un radical dialkylamino, pipéridino, morpholino ou thiomorpholino, isole le produit et le transforme éventuellement en sel avec un acide minéral ou organique.

8. Procédé de préparation des composés de formule (I) selon la revendication 1 pour lesquels R₆ représente un radical propylène substitué en position -3 par un radical méthyle à l'exception des composés pour lesquels R₃ représente un radical acétylamino et/ou R₁ représente un substituant comportant un radical hydroxy caractérisé en ce que l'on fait réagir un dérivé de formule: 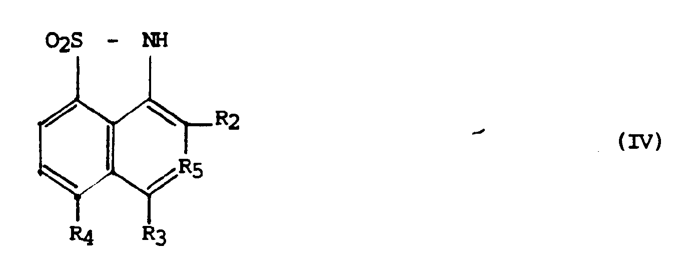 dans laquelle R₂, R₃, R₄ et R₅ ont les mêmes significations que dans la revendication 1, excepté que R₃ n'est pas un radical acétylamino, sur un dérivé de formule : 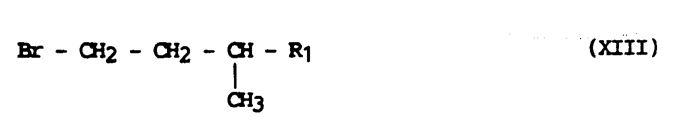 dans laquelle R₁ a les mêmes significations que dans la revendication 1, sous réserve de ne pas comporter un radical hydroxy, isole le produit et le transforme éventuellement en sel avec un acide minéral ou organique.

9. Procédé de préparation des composés de formule (I) selon la revendication 1 pour lesquels R₁ représente un radical tétrahydro-1,2,3,6 pyridyl-1 substitué en position -4 par un radical indolyl-3 substitué sur l'atome d'azote par un radical alkyle ou alkylcarbonyle ou un radical pipéridino substitué en position -4 par un radical oxo-2 benzimidazolinyl-1 substitué en position -3 par un radical alkylcarbonyle ou benzoyle ou un radical indolyl-3 substitué sur l'atome d'azote par un radical alkyle ou alkylcarbonyle, à l'exception de ceux pour lesquels R₆ représente un radical propylène substitué en position -2 par un radical hydroxy caractérisé en ce que l'on fait réagir un dérivé de formule : 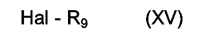 dans laquelle Hal représente un atome d'halogène et Rg représente un radical alkyle, alkylcarbonyle ou benzoyle, sur un dérivé de formule (I) correspondant pour lequel R₁ représente un radical tétrahydro-1, 2,3,6 pyridyl-1 substitué en position -4 par un radical indolyl-3 ou un radical pipéridino substitué en position -4 par un radical oxo-2 benzimidazolinyl-1 ou indolyl-3, isole le produit et le transforme éventuellement en sel avec un acide minéral ou organique.

10. Procédé de préparation des composés de formule (I) selon la revendication 1 pour lesquels R₁ représente un radical tétrahydro-1,2,3,6 pyridyl-1 substitué en position -4 par un radical halogénophényle caractérisé en ce que l'on déshydrate un composé de formule (I) correspondant pour lequel R₁ représente un radical pipéridino substitué en position -4 par un radical hydroxy et un radical phényle substitué par un atome d'halogène, isole le produit et le transforme éventuellement en sel avec un acide minéral ou organique.

11. Procédé de préparation des composés de formule (I) selon la revendication 1 pour lesquels R₁ représente un radical aminophényl-4 pipérazinyl-1 caractérisé en ce que l'on réduit un composé de formule (I) correspondant pour lequel R₁ représente un radical nitrophényl-4 pipérazinyl-1, isole le produit et le transforme éventuellement en sel avec un acide minéral ou organique.

12. Médicaments caractérisés en ce qu'ils contiennent comme principe actif au moins un composé selon la revendication 1 ou un sel de ce composé avec un acide pharmaceutiquement acceptable.

13. Médicaments caractérisés en ce qu'ils contiennent comme principe actif au moins un composé selon la revendication 2 ou un sel de ce composé avec un acide pharmaceutiquement acceptable.

14. Le [((fluoro-4 phényl)-4 pipérazinyl-1 )-3 propyl]-2 naphto [1,8-cd] isothiazole dioxyde-1,1 et ses sels avec un acide pharmaceutiquement acceptable.

15. Médicaments caractérisés en ce qu'ils contiennent comme principe actif au moins un composé selon la revendication 14 ou un sel de ce composé avec un acide pharmaceutiquement acceptable.

## Revendications (Revendications pour l'(les) Etat(s) contractant(s) suivant(s) : suivants: ES, GR)

1. Procédé de préparation d'un composé de formule : 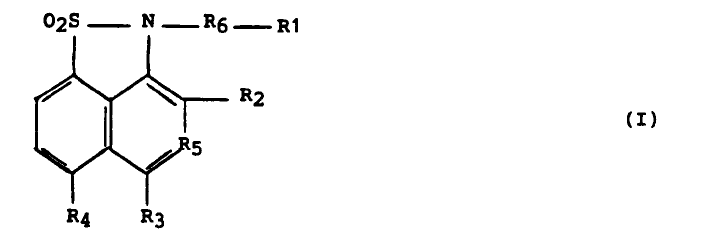 dans laquelle
- R₁ représente
. un radical tétrahydro-1,2,3,6 pyridyl-1 substitué en position -4 par (a) un radical phényle, (b) un radical phényle substitué par un atome d'halogène ou un radical alkyle, hydroxy ou alcoxy, (c) un radical indolyl-3, (d) un radical indolyl-3 substitué sur l'atome d'azote par un radical alkyle ou alkylcarbonyle et/ou en position -5 par un atome de chlore ou de fluor ou (e) un radical (hydroxy-5 indolyl)-3,
. un radical pipérazinyl-1 substitué en position -4 par (a) un radical phényle, (b) un radical phényle substitué par un radical alcoxy, alkyle, hydroxy, nitro, amino ou un atome d'halogène, (c) un radical benzisothiazol-1,2 yl-3, (d) un radical benzisoxazol-1,2 yl-3 ou (e) un radical pyridyl-2,
. un radical pipéridino substitué en position -4 par (a) un radical phényle, (b) un radical phényle substitué par un atome d'halogène ou un radical hydroxy, alkyle ou alcoxy, (c) deux radicaux phényle, (d) un radical bis (fluoro-4 phényl) méthylène, (e) un radical fluoro-4 benzoyle, (f) un radical oxo-2 benzimidazolinyl-1, (g) un radical oxo-2 benzimidazolinyl-1 substitué en position -3 par un radical alkylcarbonyle ou benzoyle, (h) un radical hydroxy et un radical phényle éventuellement substitué par un radical alkyle, alcoxy, hydroxy ou un atome d'halogène, (i) un radical indolyl-3, (j) un radical indolyl-3 substitué sur l'atome d'azote par un radical alkyle ou alkylcarbonyle et/ou en position -5 par un atome de chlore ou de brome ou (k) un radical (hydroxy-5 indolyl)-3,
- soit :
. R₂ et R₃ identiques représentent un atome d'hydrogène ou d'halogène et R₄ représente un atome d'hydrogène ou
. R₂ et R₄ représentent un atome d'hydrogène et R₃ représente un atome d'halogène ou un radical acétylamino ou
. R₂ et R₃ représentent un atome d'hydrogène et R₄ représente un atome d'halogène et R₅ représente un groupe -CH=,
- soit R₂, R₃ et R₄ représentent un atome d'hydrogène et R₅ représente un atome d'azote,
R₆ représente une chaîne alkylène contenant 2 à 4 atomes de carbone ou une chaîne propylène substituée en position -1 ou -3 par un radical alkyle ou en position -2 par un radical alkyle, alcoxy, hydroxy, dialkylamino, pipéridino, morpholino ou thiomorpholino, sous réserve que lorsque R₆ représente un radical propylène substitué en position -2 par un radical dialkylamino, pipéridino, morpholino ou thiomorpholino, R₁ ne peut pas être un radical comportant un radical hydroxy et étant entendu que les radicaux alkyle et alcoxy et les portions alkyle et alcoxy contiennent 1 à 4 atomes de carbone en chaîne droite ou ramifiée, ainsi que leurs sels avec les autres minéraux ou organiques,
aractérisé en ce que :
A- pour la préparation des composés de formule (I) à l'exception de ceux pour lesquels R₆ représente un radical propylène substitué en position-2 par un radical dialkylamino, pipéridino, morpholino ou thiomorpholino et/ou R₁ représente un radical aminophényl-4 pipérazinyl-1 on fait réagir un dérivé halogéné de formule : 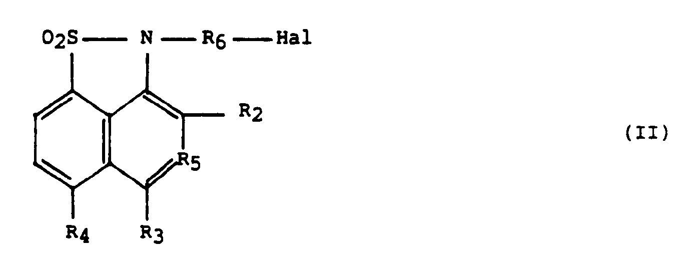 dans laquelle Hal représetne un atome d'halogène et R₂, R₃, R₄, R₅, et Rₛ, ont les mêmes significations que précédemment excepté que R₆ n'est pas un radical dialkylamino, pipéridino, morpholino ou thiomorpholino, sur un dérivé de formule : 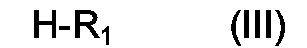 dans laquelle R₁ a les mêmes significations que précédemment à l'exception d'être un radical aminophényl-4 pipérazinyl-1, isole le produit et le transforme éventuellement en sel avec un acide minéral ou organique,
B - Pour la préparation des composés de formule (I) pour lesquels
. soit R₅ représente un groupe = CH-, R₂, R₃ et R₄ représentent un atome d'hydrogène ou R₂ et R₄ représentent un atome d'hydrogène et R₃ représente un atome d'halogène ou R₂ et R₃ représentent un atome d'hydrogène et R₄ représente un atome d'halogène ou R₂ et R₃ représentent un atome d'halogène et R₄ représente un atome d'hydrogène
. soit R₅ représente un atome d'azote et R₂, R₃ et R₄ représentent un atome d'hydrogène,
R₆ représente un radical alkylène (2-4C) ou propylène substitué en position-2 par un radical hydroxy, alkyle ou alcoxy exceptés ceux pour lesquels R₁ représente un radical aminophényl-4 pipérazinyl-1, on fait réagir un composé de formule : 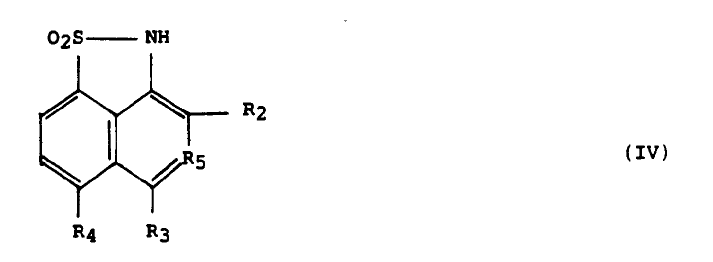 dans laquelle R₂, R₃, R₄, et R₅ ont les significations mentionnées ci-dessus avec un dérivé de formule : Hal-R₆-R₁ (X) dans laquelle R₁ et R₆ ont les mêmes significations que ci-dessus, isole le produit et le transforme éventuellement en sel avec un acide minéral ou organique, C - Pour la préparation des composés de formule (I) pour lesquels R₆ représente un radical propylène substitué en position-2 par un radical alcoxy à l'exception de ceux pour lesquels R₁ représente un radical tétrahydro-1, 2, 3, 6 pyridyl-1 substitué en position-4 par un radical hydroxyphényle, indolyl-3 substitué en position-5 par un radical hydroxy, pipérazinyl-1 substitué en position-4 par un radical amino ou hydroxyphényle ou pipéridino substitué en position-4 par un radical oxo-2 benzimidazolinyl-1, hydroxyphényle, hydroxy et phényle éventuellement substitué par un radical hydroxy ou indolyl-3 éventuellement substitué en position-5 par un radical hydroxy, on alkyle un composé de formule (I) correspondant pour lequel R₆ représente un radical propylène substitué en position-2 par un radical hydroxy, isole le produit et le transforme éventuellement en sel avec un acide minéral ou organique, D - Pour la préparation des composés de formule (I) pour lesquels R₆ représente un radical propylène substitué en position-2 par un radical dialkylamino, pipéridino, morpholino ou thiomorpholino, on fait réagir un dérivé de formule : 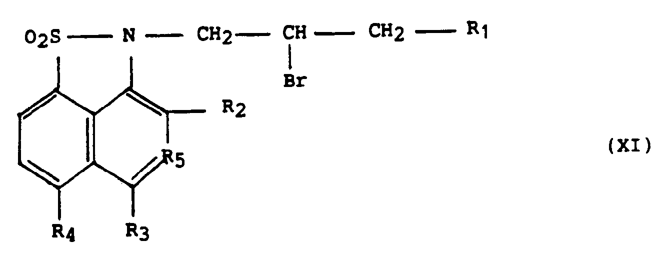 dans laquelle R₁, R₂, R₃, R₄, et R₅ ont les mêmes significations que dans la formule (l), sur un dérivé de formule: 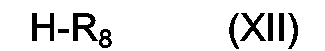 dans laquelle R₈ représente un radical dialkylamino, pipéridino, morpholino ou thiomorpholino, isole le produit et le transforme éventuellement en sel avec un acide minéral ou organique, E - Pour la préparation des composés de formule (l) pour lesquels R₆ représente un radical propylène substitué en position-3 par un radical méthyle, à l'exception des composés pour lesquels R₃ représente un radical acétylamino et/ou R₁ représente un substituant comportant un radical hydroxy, on fait réagir un dérivé de formule : 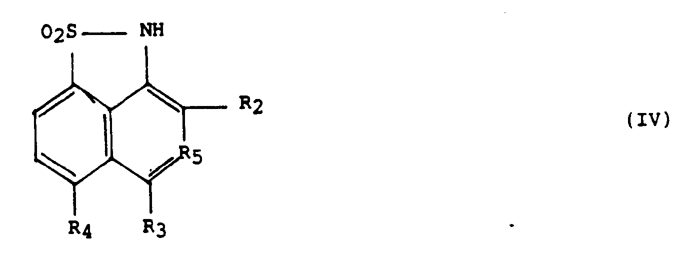 dans laquelle R₂, R₃, R₄, et R₅ ont les mêmes significations que dans la formule (I) excepté que R₃ ne peut pas être un radical acétylamino, sur un dérivé de formule : 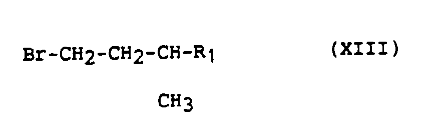 dans laquelle R₁ à les mêmes significations que dans la formule (I) sous réserve de ne pas comporter un radical hydroxy, isole le produit et le transforme éventuellement en sel avec un acide minéral ou organique, F - Pour la préparation des composés de formule (I) pour lesquels R₁ représente un radical tétrahydro-1, 2, 3, 6 pyridyl-1 substitué en position-4 par un radical indolyl-3 substitué sur l'atome d'azote par un radical alkyle ou alkylcarbonyle ou un radical pipéridino substitué en position-4 par un radical oxo-2 benzimidazolinyl-1 substitué en position-3 par un radical alkylcarbonyle ou benzoyle ou un radical indolyl-3 substitué sur l'atome d'azote par un radical alkyle ou alkylcarbonyle à l'exception de ceux pour lesquels R₆ représente un radical propylène substitué en position-2 par un radical hydroxy, on fait réagir un dérivé de formule : 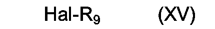 dans laquelle Hal représente un atome d'halogène et R₉ représente un radical alkyle, alkylcarbonyle ou benzoyle, sur un dérivé de formule (1) correspondant pour lequel R₁ représente un radical tetrahydro-1, 2, 3, 6 pyridyl-1 subtitué en position-4 par un radical indolyl-3 ou un radical pipéridino substitué en position-4 par un radical oxo-2 benzimidazolinyl-1 ou indolyl-3, isole le produit et le transforme éventuellement en sel avec un acide minéral ou organique, G - Pour la préparation des composés de formule (I) pour lesquels R₁ représente un radical tétrahydro-1, 2, 3, 6 pyridyl-1 substitué en position-4 par un radical halogénophényle, on deshydrate un composé de formule (I) correspondant pour lequel R₁ représente un radical pipéridino substitué en position-4 par un radical hydroxy et un radical phényle substitué par un atome d'halogène, isole le produit et le transforme éventuellement en sel avec un acide minéral ou organique, H - Pour la préparation des composés de formule (I) pour lesquels R₁ représente un radical aminophényl-4 pipérazinyl-1 on réduit un composé de formule (I) correspondant pour lequel R₁ représente un radical nitrophényl-4 pipérazinyl-1, isole le produit et le transforme éventuellement en sel avec un acide minéral ou organique.

2. Procédé selon la revendication 1 dans lequel les atomes d'halogène sont les atomes de chlore ou de brome.

3. Procédé selon la revendication 1 pour la préparation des composés de formule (I) pour lesquels R₁ représente un radical tétrahydro-1, 2, 3, 6 pyridyl-1 substitué en position-4 par un radical halogénophényle, un radical pipérazinyl-1 substitué en position-4 par un radical pyridyl-2, benzisothiazol-1, 2 yl-3 ou phényle substitué par un atome d'halogène ou un radical hydroxy, amino ou alkyle ou un radical pipéridino substitué en position-4 par un radical phényle ou N-alkylindolyl-3.

4. Procédé selon la revendication 1 pour la préparation du [((fluoro-4 phényl)-4 pipérazinyl-1)-3 propyl]-2 naphto [1,8-cd] isothiazole dioxyde-1,1 et de ses sels avec un acide pharmaceutiquement acceptable.

## Patentansprüche (Patentansprüche für folgende(n) Vertragsstaat(en) : AT, BE, CH, DE, FR, GB, IT, LI, LU, NL, SE)

1. Verbindungen der Formel 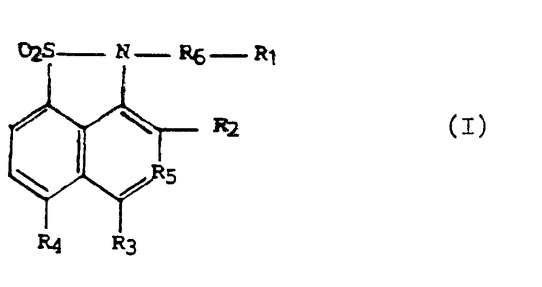 worin
- R₁ bedeutet
einen 1,2,3,6-Tetrahydro-pyridyl-1-Rest, der in 4-Stellung substituiert ist durch (a) einen Phenylrest, (b) einen durch ein Halogenatom oder einen Alkyl-, Hydroxy- oder Alkoxyrest substituierten Phenylrest, (c) einen Indolyl-3-Rest, (d) einen an dem Stickstoffatom durch einen Alkyl- oderAlkylcarbonylrest und/oder in 5-Stellung durch ein Chlor- oder Fluoratom substituierten Indolyl-3 -Rest oder (e) einen (5-Hydroxyindolyl)-3-Rest,
einen Piperazinyl-1-Rest, der in 4-Stellung substituiert ist durch (a) einen Phenylrest, (b) einen durch einen Alkoxy-, Alkyl-, Hydroxy-, Nitro-, Aminorest oder ein Halogenatom substituierten Phenylrest, (c) einen 1,2-Benzisothiazolyl-3-Rest, (d) einen 1,2-Benzisoxazolyl- 3-Rest oder (e) einen pyridyl-2-Rest,
einen Piperidinorest, substituiert in 4-Stellung durch (a) einen Phenylrest, (b) einen durch ein Halogenatom oder einen Hydroxy-, Alkyl- oder Alkoxyrest substituierten Phenylrest, (c) bei Phenylreste, (d) einen Bis(4-fluorphenyl)-methylenrest, (e) einen 4-Fluorbenzoylrest, (f) einen 2-oxo-benzimidazolinyl-1-Rest, (g) einen 2-Oxo-benzimidazolinyl-1-Rest, substituiert in 3-Stellung durch einen Alkylcarbonyl- oder Benzoylrest, (h) einen Hydroxyrest und einen Phenylrest, der gegebenenfalls durch einen Alkyl-, Alkoxy-, Hydroxyrest oder ein Halogenatom substituiert ist, (i) einen Indolyl-3-Rest, (j) einen an dem Stickstoffatom durch einen Alkyl- oder Alkylcarbonylrest und/oder in 5-Stellung durch ein Chloroder Bromatom substituierten Indolyl-3-Rest oder (k) einen (5-Hydroxyindolyl)-3-Rest,
- entweder
R₂ und R₃, die identisch sind, für ein Wasserstoff- oder Halogenatom stehen und R₄ ein Wasserstoffatom bedeutet oder
R₂ und R₄ für ein Wasserstoffatom stehen und R₃ ein Halogenatom oder einen Acetylaminorest bedeutet oder
R₂ und R₃ ein Wasserstoffatom bedeuten und R₄ für ein Halogenatom steht und R₅ für eine Gruppe -CH= steht,
- oder
R₂, R₃ und R₄ ein Wasserstoffatom bedeuten und R₅ für ein Stickstoffatom steht, R₆ für eine Alkylenkette mit 2 bis 4 Kohlenstoffatomen oder eine Propylenkette, welche substituiert ist in 1-oder 3-Stellung durch einen Alkylrest oder in 2-Stellung durch einen Alkyl-, Alkoxy-, Hydroxy-, Dialkylamino-, Piperidino-, Morpholino- oder Thiomorpholinorest, steht, mit der Maßgabe, daß, wenn Rₛ für einen in 2-Stellung durch einen Dialkylamino-, Piperidino-, Morpholino- oder Thiomorpholinorest substituierten Propylenrest steht, R₁ keinen eine Hydroxygruppe enthaltenden Rest bedeuten kann, und wobei die Alkyl- und Alkoxyreste und die Alkyl - und Alkoxyteile 1 bis 4 Kohlenstoffatome in gerader oder verzweigter Kette enthalten,
sowie deren Salze mit Mineral- oder organischen Säuren.

2. Verbindungen der Formel (I) gemäß Anspruch 1, worin die Halogenatome Chlor- oder Bromatome sind.

3. Verbindungen der Formel (I) gemäß Anspruch 1, worin R₁ einen 1,2,3,6-Tetrahydropyridyl-l-Rest, substituiert in 4-Stellung durch einen Halogenphenyl-, Phenyl-oder an dem Stickstoffatom durch einen Alkyl-oder Alkylcarbonylrest substituierten Indolyl-3-.Rest, einen Piperazinyl-1-Rest, substituiert in 4-Stellung durch einen pyridyl-2-, 1,2-Benzisothiazolyl-3- oder Phenylrest, der durch ein Halogenatom oder durch eine Hydroxy-, Amino-oder Alkylgruppe oder durch einen in 4-Stellung durch einen Phenyl- oder N-Alkylindolyl-3 Rest substituierten Piperidinorest substituiert ist, bedeutet.

4. Verfahren zur Herstellung der Verbindungen der Formel (I) gemäß Anspruch 1 mit Ausnahme derjenigen, worin R₆ einen in 2-Stellung durch einen Dialkylamino-, Piperidino-, Morpholino- oder Thiomorpholinorest substituierten Propylenrest steht und/oder R₁ einen 4-Aminophenylpiperazinyl-1-Rest bedeutet, dadurch gekennzeichnet, daß man ein halogeniertes Derivat der Formel 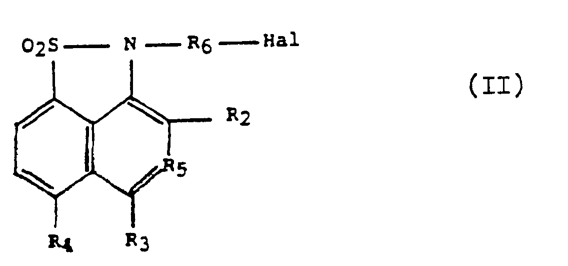 worin Hal für ein Halogenatom steht und R₂, R₃, R₄, R₅ und R₅ die gleichen Bedeutungen wie in Anspruch 1 besitzen, mit Ausnahme dessen, daß R₆ keinen in 2-Stellung durch eine Dialkylamino-, Piperidino-, Morpholino- oder Thiomorpholinogruppe substituierten Propylenrest darstellt, mit einem Derivat der Formel 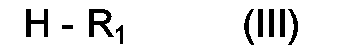 worin R₁ die gleiche Bedeutung wie in Anspruch 1 besitzt mit Ausnahme eines 4-Aminophenylpiperazinyl-1-Restes, umsetzt, das Produkt isoliert und es gegebenenfalls mit einer Mineral- oder organischen Säure in ein Salz überfürt.

5. Verfahren zur Herstellung der Verbindungen der Formel (I) gemäß Anspruch 1, worin
entweder R₅ eine Gruppe =CH- bedeutet, R₂, R₃ und R₄ für ein Wasserstoffatom stehen oder R₂ und R₄ ein Wasserstoffatom bedeuten und R₃ für ein Halogenatom steht oder R₂ und R₃ ein Wasserstoffatom bedeuten und R₄ für ein Halogenatom steht oder R₂ und R₃ ein Halogenatom bedeuten und R₄ für ein Wasserstoffatom steht,
oder R₅ ein Stickstoffatom bedeutet und R₂, R₃ und R₄ für ein Wasserstoffatom stehen, R₆ für einen Alkylen(C₂₋₄)- oder Propylenrest, welcher in 2-Stellung durch einen Hydroxy-, Alkyl- oderAlkoxyrest substituiert ist, steht, mit Ausnahme derjenigen, worin R₁ einen 4-Aminophenyl-piperazinyl-1-Rest bedeutet, dadurch gekennzeichnet, daß man eine Verbindung der Formel 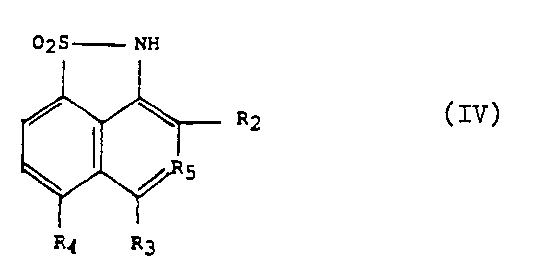 worin R₂, R₃, R₄ und R₅ die vorstehend angegebenen Bedeutungen besitzen, mit einem Derivat der Formel 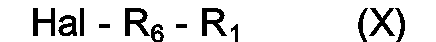 worin R₁ und R₆ die vorstehend angegebenen Bedeutungen besitzen und Hal für ein Halogenatom steht, umsetzt, das Produkt isoliert und es gegebenenfalls mit einer Mineral-oder organischen Säure in ein Salz überführt.

6. Verfahren zur Herstellung der Verbindungen der Formel (I) gemäß Anspruch 1, worin R₆ einen in 2-Stellung mit einem Alkoxyrest substituierten Propylenrest bedeutet, mit Ausnahme derjenigen, worin R₁ einen 1,2,3,6-Tetrahydropyridyl-1-Rest, der in 4-Stellung durch einen Indolyl-3-Rest , einen in 5-Stellung durch einen Hydroxy-oder Hydroxyphenylrest substituierten Indolyl-3-Rest substituiert ist, einen Piperazinyl-1-Rest, der in 4-Stellung durch einen Amino- oder Hydroxyphenylrest substituiert ist, oder einen Piperidinorest, Cer in 4-Stellung durch einen 2-Oxobenzimidazolinyl-1-, Hydroxyphenyl-, Hydroxy- und gegebenenfalls durch einen Hydroxyrest substituierten Phenyl- oder gegebenenfalls in 5-Stellung durch einen Hydroxyrest substituierten Indolyl-3 -Rest substituiert ist, wiedergibt, dadurch gekennzeichnet, daß man eine entsprechende Verbindung der Formel (l), für die R₆ einen in 2-Stellung durch einen Hydroxyrest substituierten Propylenrest bedeutet, alkyliert, das Produkt isoliert und es gegebenenfalls mit einer Mineral- oder organischen Säure in ein Salz überführt.

7. Verfahren zur Herstellung der Verbindungen der Formel (I) gemäß Anspruch 1, worin R₆ einen in 2-Stellung durch einen Dialkylamino-, Piperidino-, Morpholino-oder Thiomorpholinorest substituierten Propylenrest bedeutet, dadurch gekennzeichnet, daß man ein Derivat der Formel 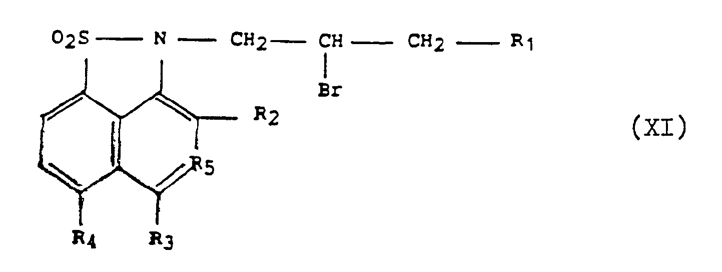 worin R₁, R₂, R₃, R₄ und R₅ die in Anspruch 1 angegebenen Bedeutungen besitzen, mit einem Derivat der Formel 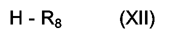 worin R₈ für einen Dialkylamino-, Piperidino-, Morpholino- oder Thiomorpholinorest steht, umsetzt, das Produkt isoliert und es gegebenefalls mit einer Mineral- oder organischen Säure in ein Salz überführt.

8. Verfahren zur Herstellung der Verbindungen der Formel (I) gemäß Anspruch 1, worin R₆ einen in 3-Stellung mit einer Methylgruppe substituierten Propylenrest bedeutet, mit Ausnahme der Verbindungen, worin R₃ für einen Acetylaminorest steht und/oder R₁ einen eine Hydroxygruppe aufweisenden Substituenten wiedergibt, dadurch gekennzeichnet, daß man ein Derivat der Formel 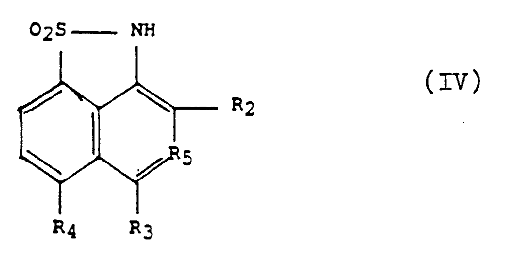 worin R₂, R₃, R₄ und R₅ die in Anspruch 1 angegebenen Bedeutungen besitzen, wobei jedoch R₃ keinen Acetylaminorest bedeuten kann, mit einem Derivat der Formel 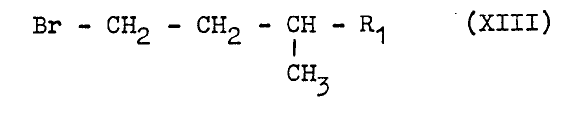 worin R₁ die in Anspruch 1 angegebenen Bedeutungen besitzt, wobei es jedoch keine Hydroxygruppe enthalten kann, umsetzt, das Produkt isoliert und es gegebenenfalls mit einer Mineral- oder organischen Säure in ein Salz überführt;

9. Verfahren zur Herstellung der Verbindungen der Formel (I) gemäß Anspruch 1,
worin R₁ einen 1,2,3,6-Tetrahydro-pyridyl-l-Rest, substituiert in 4-Stellung durch einen an dem Stickstoffatom durch einen Alkyl- oder Alkylcarbonylrest substituierten Indolyl-3-Rest oder einen Piperidinorest, substituiert in 4-Stellung durch einen 2-Oxo-benzimidazolinyl1-Rest, welcher in 3-Stellung durch einen Alkylcarbonyloder Benzoylrest substituiert ist, oder einen an dem Stickstoffatom durch einen Alkyl- oder Alkylcarbonylrest substituierten Indolyl-3-Rest bedeutet, mit Ausnahme derjenigen, worin R₆ für eine in 2-Stellung durch einen Hydroxyrest substituierte Propylengruppe steht,dadurch gekennzeichnet, daß man ein Derivat der Formel 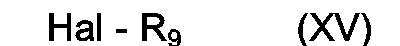
worin Hal für ein Halogenatom steht und Rg einen Alkyl-, Alkylcarbonyl- oder Benzoylrest bedeutet, mit einem entsprechenden Derivat der Formel (I) umsetzt, worin R₁ einen 1,2,3,6-Tetrahydro-pyridyl-1-Rest, der in 4-Stellung durch einen Indolyl-3-Rest substituiert ist, oder einen Piperidinorest, der in 4-Stellung durch einen 2-Oxo-benzimidazolinyl-1- oder Indolyl-3-Rest substituiert ist, bedeutet, das Produkt isoliert und es gegebenenfalls mit einer Mineral- oder organischen Säure in ein Salz überführt.

10. Verfahren zur Herstellung der Verbindungen der Formel (I) gemäß Anspruch 1, worin R₁ einen 1,2,3,6-Tetrahydro-pyridyl-1-Rest, der in 4-Stellung durch einen Halogenphenylrest substituiert ist, bedeutet,dadurch gekennzeichnet, daß man eine entsprechende Verbindung der Formel (I), worin R₁ einen Piperidinorest bedeutet, der in 4-Stellung durch einen Hydroxyrest
und einen durch ein Halogenatom substituierten Phenylrest substituiert ist, dehydratisiert, das Produkt isoliert und es gegebenefalls mit einer Mineral- oder organischen Säure in ein Salz überführt.

11. Verfahren zur Herstellung der Verbindungen der Formel (I) gemäß Anspruch 1, worin R₁ einen 4-Aminophenyl-piperazinyl-1-Rest bedeutet, dadurch gekennzeichnet, daß man eine entsprechende Verbindung der Formel (I), worin R₁ für einen 4-Nitrophenyl-piperazinyl-1-Rest steht, reduziert, das Produkt isoliert und es gegebenenfalls mit einer Mineral- oder organischen Säure in ein Salz überführt.

12. Arzneimittel, dadurch gekennzeichnet, daß sie als Wirkstoff zumindest eine Verbindung gemäßAnspruch 1 oder ein Salz dieser Verbindung mit einer pharmazeutisch annehmbaren Säure enthalten.

13. Arzneimittel, dadurch gekennzeichnet, daß sie als Wirkstoff zumindest eine Verbindung gemäßAnspruch 2 oder ein Salz dieser Verbindung mit einer pharmazeutisch verträglichen Säure enthalten.

14. 2-[3-(4-(4-Fluorphenyl)-piperazinyl-1)-propyl]-naphtho[1,8-cd]-isothiazol-1,1-dioxid und dessen Salze mit einer pharmazeutisch verträglichen Säure.

15. Arzneimittel, dadurch gekennzeichnet, daß sie als Wirkstoff zumindest eine Verbindung gemäßAnspruch 14 oder ein Salz dieser Verbindung mit einer pharmazeutisch verträglichen Säure enthalten.

## Patentansprüche (Patentansprüche für folgende(n) Vertragsstaat(en) : ES, GR)

1. Verfahren zur Herstellung einer Verbindung der Formel 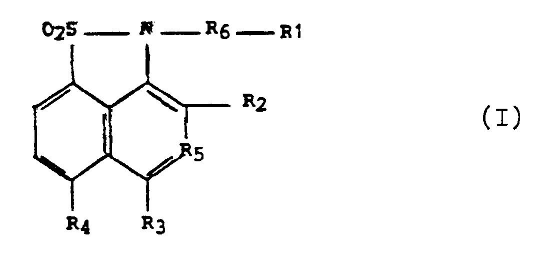 worin
- R₁ bedeutet
einen 1,2,3,6-Tetrahydro-pyridyl-1-Rest, der in 4-Stellung substituiert ist durch (a) einen Phenylrest, (b) einen durch ein Halogenatom oder einen Alkyl-, Hydroxy- oder Alkoxyrest substituierten Phenylrest, (c) einen Indolyl-3-Rest, (d) einen an dem Stickstoffatom durch einen Alkyl- oderAlkylcarbonylrest und/oder in 5-Stellung durch ein Chlor- oder Fluoratom substituierten Indolyl-3-Rest oder (e) einen (5-Hydroxyindolyl)-3-Rest,
einen Piperazinyl-1-Rest, der in 4-Stellung substituiert ist durch (a) einen Phenylrest, (b) einen durch einen Alkoxy-, Alkyl-, Hydroxy-, Nitro-, Aminorest oder ein Halogenatom substituierten Phenylrest, (c) einen 1,2-Benzisothiazolyl-3-Rest, (d) einen 1,2-Benzisoxazolyl-3-Rest oder (e) einen Pyridyl-2-Rest,
einen Piperidinorest, substituiert in 4-Stellung durch (a) einen Phenylrest, (b) einen durch ein Halogenatom oder einen Hydroxy-, Alkyl- oder Alkoxyrest substituierten Phenylrest, (c) zwei Phenylreste, (d) einen Bis-(4-fluorphenyl)-methylenrest, (e) einen 4-Fluorbenzoylrest, (f) einen 2-Oxo-benzimidazolinyl-1-Rest, (g) einen 2-Oxo-benzimidazolinyl-1-Rest, substituiert in 3-Stellung durch einen Alkylcarbonyl- oder Benzoylrest, (h) einen Hydroxyrest und einen Phenylrest, der gegebenenfalls durch einen Alkyl-, Alkoxy-, Hydroxyrest oder ein Halogenatom substituiert ist, (i) einen Indolyl-3-Rest, (j) einen an dem Stickstoffatom durch einen Alkyl- oder Alkylcarbonylrest und/oder in 5-Stellung durch ein Chloroder Bromatom substituierten Indolyl-3-Rest oder (k) einen (5-Hydroxyindolyl)-3-Rest,
- entweder
R₂ und R₃, die identisch sind, für ein Wasserstoff- oder Halogenatom stehen und R₄ ein Wasserstoffatom bedeutet oder
R₂ und R₄ für ein Wasserstoffatom stehen und R₃ ein Halogenatom oder einen Acetylaminorest bedeutet oder
R₂ und R₃ ein Wasserstoffatom bedeuten und R₄ für ein Halogenatom steht und R₅ für eine Gruppe -CH= steht,
- oder
R₂, R₃ und R₄ ein Wasserstoffatom bedeuten und
R₅ für ein Stickstoffatom steht,
R₆ für eine Alkylenkette mit 2 bis 4 Kohlenstoffatomen oder eine Propylenkette, welche substituiert ist in 1-oder 3-Stellung durch einen Alkylrest oder in 2-Stellung durch einen Alkyl-, Alkoxy-, Hydroxy-, Dialkylamino-, Piperidino-, Morpholino- oder Thiomorpholinorest, steht, mit der Maßgabe, daß, wenn Rₛ für einen in 2-Stellung durch einen Dialkylamino-, Piperidino-, Morpholino- oder Thiomorpholinorest substituierten Propylenrest steht, R₁ keinen eine Hydroxygruppe enthaltenden Rest bedeuten kann, und wobei die Alkyl- und Alkoxyreste und die Alkyl- und Alkoxyteile 1 bis 4 Kohlenstoffatome in gerader oder verzweigter Kette enthalten,
sowie von deren Salzen mit Mineral- oder organischen Säuren,
dadurch gekennzeichnet, daß man
(A) zur Herstellung der Verbindungen der Formel (I) mit Ausnahme von denjenigen, worin R₆ für einen in 2-Stellung durch einen Dialkylamino-, Piperidino-, Morpholino- oder Thiomorpholinorest substituierten Propylenrest steht und/oder R₁ einen 4-Aminophenyl-piperazinyl-1-Rest bedeutet, ein halogeniertes Derivat der Formel 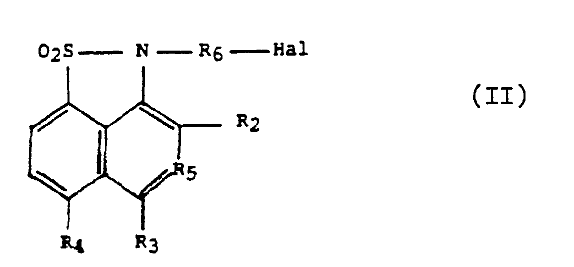 worin Hal für ein Halogenatom steht und R₂, R₃, R₄, R₅ und R₆ die vorstehend angegebenen Bedeutungen besitzen, mit Ausnahme dessen, daß R₆ keinen Dialkylamino-, Piperidino-, Morpholino- oder Thiomorpholinorest darstellt, mit einem Derivat der Formel H - R₁ (lll) worin R₁ die vorstehend angeegebene Bedeutung mit Ausnahme eines 4-Aminophenyl-piperazinyl-1-Restes besitzt, umsetzt, das Produkt isoliert und es gegebenenfalls mit einer Mineral- oder organischen Säure in ein Salz überführt;
(B) zur Herstellung der Verbindungen der Formel (I), worin
entweder R₅ eine Gruppe =CH- bedeutet, R₂, R₃ und R₄ für ein Wasserstoffatom stehen oder R₂ und R₄ ein Wasserstoffatom bedeuten und R₃ für ein Halogenatom steht oder R₂ und R₃ ein Wasserstoffatom bedeuten und R₄ für ein Halogenatom steht oder R₂ und R₃ ein Halogenatom bedeuten und R₄ für ein Wasserstoffatom steht,
oder R₅ ein Stickstoffatom bedeutet und R₂, R₃ und R₄ für ein Wasserstoffatom stehen,
R₆ für einen Alkylen(C₂₋₄)- oder Propylenrest, welcher in 2-Stellung durch einen Hydroxy-, Alkyl- oder Alkoxyrest substituiert ist, steht, mit Ausnahme derjenigen, worin R₁ einen 4-Aminophenyl-piperazinyl-1-Rest bedeutet, eine Verbindung der Formel 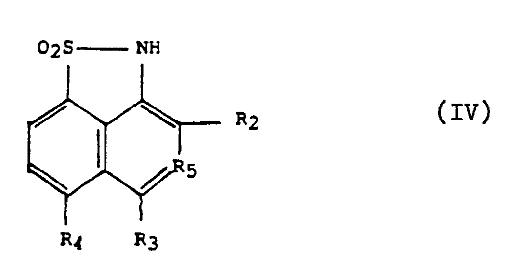 worin R₂, R₃, R₄ und R₅ die vorstehend angegebenen Bedeutungen besitzen, mit einem Derivat der Formel 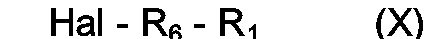 worin R₁ und R₆ die vorstehend angegebenen Bedeutungen besitzen, umsetzt, das Produkt isoliert und es gegebenenfalls mit einer Mineral- oder organischen Säure in ein Salz überführt;
(C) zur Herstellung der Verbindungen der Formel (I), worin R₆ einen in 2-Stellung mit einem Alkoxyrest substituierten Propylenrest bedeutet, mit Ausnahme derjenigen, worin R₁ einen 1,2,3,6-Tetrahydro- pyridyl-1-Rest, der in 4-Stellung substituiert ist durch einen Hydroxyphenyl-, in 5-Stellung durch einen Hydroxyrest substituierten Indolyl-3-Rest, einen Piperazinyl-1-Rest, der substituiert ist in 4-Stellung durch einen Amino- oder Hydroxyphenylrest, oder einen Piperidinorest, der substituiert ist in 4-Stellung durch einen 2-Oxo-benzimidazolinyl-1-, Hydroxyphenyl-, Hydroxy- und gegebenenfalls durch einen Hydroxyrest substituierten Phenyl- oder gegebenenfalls in 5-Stellung durch einen Hydroxyrest substituierten Indolyl-3-Rest, wiedergibt, eine entsprechende Verbindung der Formel (I), für die R₆ einen in 2-Stellung durch einen Hydroxyrest substituierten Propylenrest bedeutet, alkyliert, das Produkt isoliert und es gegebenenfalls mit einer Mineral- oder organischen Säure in ein Salz überführt;
(D) zur Herstellung der Verbindungen der Formel (I), worin R₆ einen in 2-Stellung mit einem Dialkylamino-, Piperidino-, Morpholino- oder Thiomorpholinorest substituierten Propylenrest bedeutet, ein Derivat der Formel 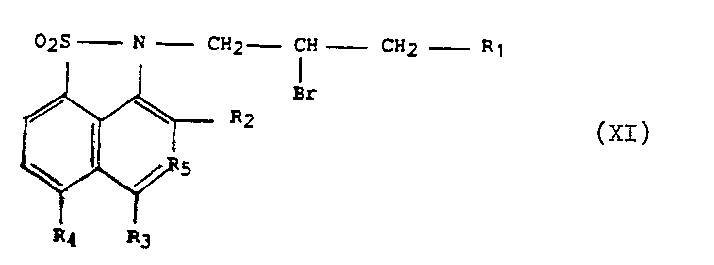 worin Rᵢ, R₂, R₃, R₄ und R₅ die für Formel (I) angegebenen Bedeutungen besitzen, mit einem Derivat der Formel 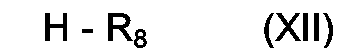 worin R₈ für einen Dialkylamino-, Piperidino-, Morpholino- oder Thiomorpholinorest steht, umsetzt, das Produkt isoliert und es gegebenefalls mit einer Mineral- oder organischen Säure in ein Salz überführt;
(E) zur Herstellung der Verbindungen der Formel (I), worin R₆ einen in 3-Stellung mit einer Methylgruppe substituierten Propylenrest bedeutet, mit Ausnahme der Verbindungen, worin R₃ für einen Acetylaminorest steht und/oder R₁ einen eine Hydroxygruppe aufweisenden Substituenten bedeutet, ein Derivat der Formel 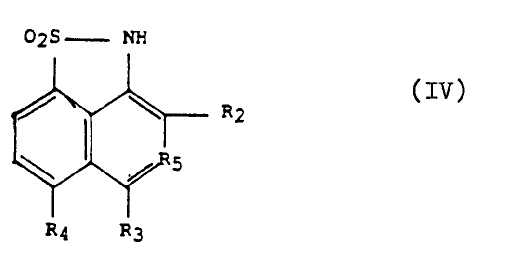 worin R₂, R₃, R₄ und R₅ die für Formel (I) angegebenen Bedeutungen besitzen, wobei jedoch R₃ keinen Acetylaminorest bedeuten kann, mit einem Derivat der Formel 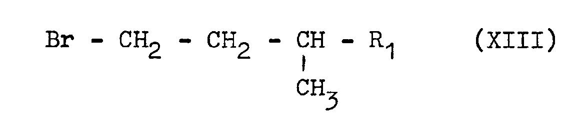 worin R₁ die für Formel (I) angegebenen Bedeutungen besitzt, wobei es jedoch keine Hydroxygruppe enthalten kann, umsetzt, das Produkt isoliert und es gegebenenfalls mit einer Mineral- oder organischen Säure in ein Salz überführt;
(F) zur Herstellung der Verbindungen der Formel (I), worin R₁ einen 1,2,3,6-Tetrahydro-pyridyl-1-Rest, substituiert in 4-Stellung durch einen an dem Stickstoffatom durch einen Alkyl- oder Alkylcarbonylrest substituierten Indolyl-3-Rest oder einen Piperidinorest, substituiert in 4-Stellung durch einen 2-Oxo- benzimidazolinyl-1-Rest, welcher in 3-Stellung durch einen Alkylcarbonyl- oder Benzoylrest substituiert ist, oder einen an dem Stickstoffatom durch einen Alkyl- oder Alkylcarbonylrest substituierten Indolyl-3-Rest bedeutet, mit Ausnahme derjenigen, worin Rs für eine in 2-Stellung durch einen Hydroxyrest substituierte Propylengruppe steht, ein Derivat der Formel 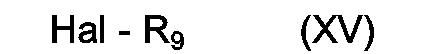 worin Hal für ein Halogenatom steht und Rg einen Alkyl-, Alkylcarbonyl- oder Benzoylrest bedeutet, mit einem entsprechenden Derivat der Formel (I) umsetzt, worin R₁ einen 1,2,3,6-Tetrahydro-pyridyl-l-Rest, der in 4-Stellung durch einen Indolyl-3-Rest substituiert ist, oder einen Piperidinorest, der in 4-Stellung durch einen 2-Oxo-benzimidazolinyl-1- oder Indolyl-3-Rest substituiert ist, bedeutet, das Produkt isoliert und es gegebenenfalls mit einer Mineral- oder organischen Säure in ein Salz überführt;
(G) zur Herstellung der Verbindungen der Formel (I), worin R₁ einen 1,2,3,6-Tetrahydro-pyridyl-1-Rest, der in 4-Stellung durch einen Halogenphenylrest substituiert ist, bedeutet, eine entsprechende Verbindung der Formel (I), worin R₁ einen Piperidinorest bedeutet, der in 4-Stellung durch einen Hydroxyrest und einen durch ein Halogenatom substituierten Phenylrest substituiert ist, dehydratisiert, das Produkt isoliert und es gegebenenfalls mit einer Mineral- oder organischen Säure in ein Salz überführt;
(H) zur Herstellung der Verbindungen der Formel (I), worin R₁ einen 4-Aminophenyl-piperazinyl-1-Rest bedeutet, eine entsprechende Verbindung der Formel (I), worin R₁ für einen 4-Nitrophenyl-piperazinyl-1-Rest steht, reduziert, das Produkt isoliert und es gegebenenfalls mit einer Mineral- oder organischen Säure in ein Salz überführt.

2. Verfahren gemäß Anspruch 1, worin die Halogenatome die Chlor- oder Bromatome sind.

3. Verfahren gemäß Anspruch 1 zur Herstellung der Verbindungen der Formel (I), worin R₁ für einen in 4-Stellung durch einen Halogenphenylrest substituierten 1,2,3,6-Tetrahydro-pyridyl-1-Rest, einen Piperazinyl-1-Rest, substituiert in 4-Stellung durch einen Pyridyl-2-, 1,2-Benzisothiazolyl-3- oder durch einen Phenylrest, der durch ein Halogenatom oder eine Hydroxy-, Amino- oder Alkylgruppe oder durch einen in 4-Stellung durch einen Phenyl- oder N-Alkylindolyl-3-Rest substituierten Piperidinorest substituiert ist, steht.

4. Verfahren gemäß Anspruch 1 zur Herstellung von 2-[3-(4-(4-Fluorphenyl)-piperazinyl-1)-propyl]-naptho-[1,8-cd]-isothiazol-1,1-dioxid und von dessen Salzen mit einer pharmazeutisch verträglichen Säure.

## Claims (Claims for the following Contracting State(s) : AT, BE, CH, DE, FR, GB, IT, Ll, LU, NL, SE)

1. Compounds of formula: 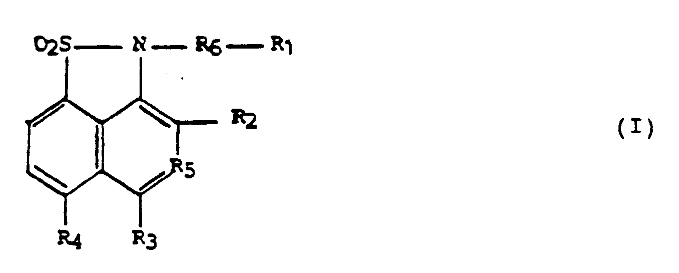
- R₁ represents
. a 1,2,3,6-tetrahydro- 1-pyridyl radical substituted at the 4-position by (a) a phenyl radical, (b) a phenyl radical substituted by a halogen atom or an alkyl, hydroxyl or alkoxy radical, (c) a 3-indolyl radical, (d) a 3-indolyl radical substituted on the nitrogen atom by an alkyl or alkylcarbonyl radical and/or at the 5-position by a chlorine or fluorine atom or (e) a 3-(5-hydroxyindolyl) radical,
. a 1-piperazinyl radical substituted at the 4-position by (a) a phenyl radical, (b) a phenyl radical substituted by an alkoxy, alkyl, hydroxyl, nitro or amino radical or a halogen atom, (c) a 1,2-benzisothiazol-3-yl radical, (d) a 1,2-benzisoxazol-3-yl radical or (e) a 2-pyridyl radical,
. a piperidino radical substituted at the 4-position by (a) a phenyl radical, (b) a phenyl radical substituted by a halogen atom or a hydroxyl, alkyl or alkoxy radical, (c) two phenyl radicals, (d) a bis(4-fluorophenyl)- methylene radical, (e) a 4-fluorobenzoyl radical, (f) a 2-oxo-1-benzimidazolinyl radical, (g) a 2-oxo-1-benzimidazolinyl radical substituted at the 3-position by an alkylcarbonyl or benzoyl radical, (h) a hydroxyl radical and a phenyl radical which is optionally substituted by an alkyl, alkoxy or hydroxyl radical or a halogen atom, (i) a 3-indolyl radical, (j) a 3-indolyl radical substituted on the nitrogen atom by an alkyl or alkylcarbonyl radical and/or at the 5-position by a chlorine or fluorine atom or (k) a 3-(5-hydroxyindolyl) radical,
- either:
. R₂ and R₃ are identical and represent a hydrogen or halogen atom and R₄ represents a hydrogen atom or
. R₂ and R₄ represent a hydrogen atom and R₃ represents a halogen atom oran acetylamino radical or
. R₂ and R₃ represent a hydrogen atom and R₄ represents a halogen atom and R₅ represents a group -CH=,
- or R₂, R₃ and R₄ represent a hydrogen atom and R₅ represents a nitrogen atom, and
R₆ represents an alkylene chain containing 2 to 4 carbon atoms or a propylene chain substituted at the 1- or 3-position by an alkyl radical or at the 2-position by an alkyl, alkoxy, hydroxyl, dialkylamino, piperidino, morpholino orthiomorpholino radical, with the proviso that when R₆ represents a propylene radical substituted at the 2-position by a dialkylamino, piperidino, morpholino orthiomorpholino radical, R₁ cannot be a radical containing a hydroxyl radical, and it being understood that the alkyl and alkoxy radicals and the alkyl and alkoxy portions contain 1 to 4 straight- or branched-chain carbon atoms, as well as their salts with inorganic or organic acids.

2. Compounds of formula (I) according to claim 1 for which the halogen atoms are chlorine or bromine atoms.

3. Compounds of formula (I) according to claim 1 for which R₁ represents a 1,2,3,6-tetrahydro-t-pyridyi radical substituted at the 4-position by a halophenyl, phenyl or 3-indolyl radical substituted on the nitrogen atom by an alkyl or alkylcarbonyl radical, a 1-piperazinyl radical substituted at the 4-position by a 2-pyridyl radical, 1,2-benzisothiazol-3-yl radical or phenyl radical substituted by a halogen atom or a hydroxyl, amino or alkyl radical or a piperidino radical substituted at the 4-position by a phenyl or N-alkyl-3-indolyl radical.

4. Method of preparing the compounds of formula (I) according to claim 1, with the exception of those for which R₆ represents a propylene radical substituted at the 2-position by a dialkylamino, piperidino, morpholino or thiomorpholino radical and/or R₁ represents a 4-aminophenyl-1-piperazinyl radical, characterised in that a halogenated derivative of formula: 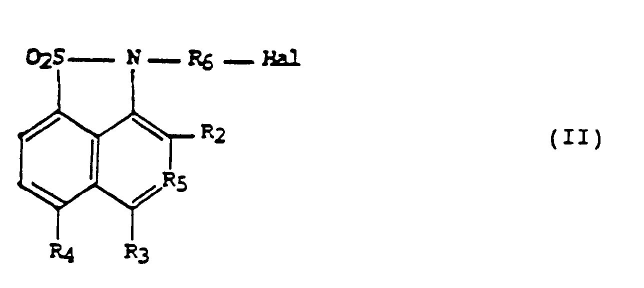 in which Hal represents a halogen atom and R₂, R₃, R₄, R₅ and R₆ have the same meanings as in claim 1 except that R₆ cannot represent a propylene radical substituted at the 2-position by dialkylamino, piperidino, morpholino or thiomorpholino, is reacted with a derivative of formula: 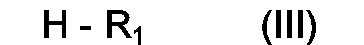 in which R₁ has the same meanings as in claim 1 with the exception of being a 4-aminophenyl-1-piperazinyl radical, the product is isolated and is optionally converted to a salt with an inorganic or organic acid.

5. Method of preparing the compounds of formula (I) according to claim 1 for which
- either R₅ represents a group CH-, R₂, R₃ and R₄ represent a hydrogen atom or R₂ and R₄ represent a hydrogen atom and R₃ represents a halogen atom or R₂ and R₃ represent a hydrogen atom and R₄ represents a halogen atom or R₂ and R₃ represent a halogen atom and R₄ represents a hydrogen atom,
- or R₅ represents a nitrogen atom and R₂, R₃ and R₄ represent a hydrogen atom, and
R₆ represents an alkylene (2-4 C) radical or propylene radical substituted in the 2-position by a hydroxyl, alkyl or alkoxy radical, with the exception of those for which R₁ is a 4-aminophenyl-1-piperazinyl radical, characterised in that a compound of formula: 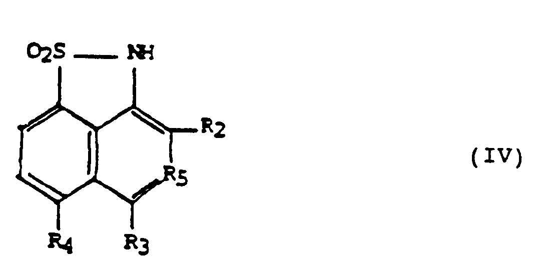 in which R₂, R₃, R₄ and R₅ have the meanings mentioned above, is reacted with a derivative of formula: 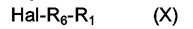 in which R₆ and R₁ have the same meanings as above and Hal represents a halogen atom, the product is isolated and is optionally converted to a salt with an inorganic or organic acid.

6. Method of preparing the compounds of formula (l) according to claim 1 for which R₆ represents a propylene radical substituted at the 2-position by an alkoxy radical, with the exception of those for which R₁ represents a 1,2,3,6-tetrahydro-1-pyridyl radical substituted at the 4-position by a 3-indolyl radical, a 3-indolyl radical substituted at the 5-position by a hydroxyl or hydroxyphenyl radical, a 1-piperazinyl radical substituted at the 4-position by an amino or hydroxyphenyl radical or a piperidino radical substituted at the 4- position by a 2-oxo-1-benzimidazolinyl radical, a hydroxyphenyl radical, a hydroxyl radical and a phenyl radical optionally substituted by a hydroxyl radical or a 3-indolyl radical optionally substituted at the 5-position by a hydroxyl radical, characterised in that a corresponding compound of formula (l) for which R₆ represents a propylene radical substituted at the 2-position by a hydroxyl radical is alkylated, the product is isolated and is optionally converted to a salt with an inorganic or organic acid.

7. Method of preparing the compounds of formula (l) according to claim 1 for which R₆ represents a propylene radical substituted at the 2-position by a dialkylamino, piperidino, morpholino or thiomorpholino radical, characterised in that a derivative of formula: 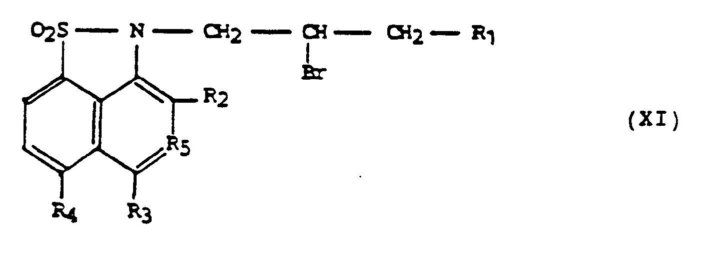 in which R₁, R₂, R₃, R₄ and R₅ have the same meanings as in claim 1 is reacted with a derivative of formula: 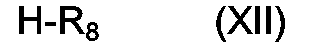 in which R₈ represents a dialkylamino, piperidino, morpholino orthiomorpholino radical, the product is isolated and is optionally converted to a salt with an inorganic or organic acid.

8. Method of preparing the compounds of formula (l) according to claim 1 for which R₆ represents a propylene radical substituted at the 3-position by a methyl radical, with the exception of the compounds for which R₃ represents an acetylamino radical and/or R₁ represents a substituent containing a hydroxyl radical, characterised in that a derivative of formula: 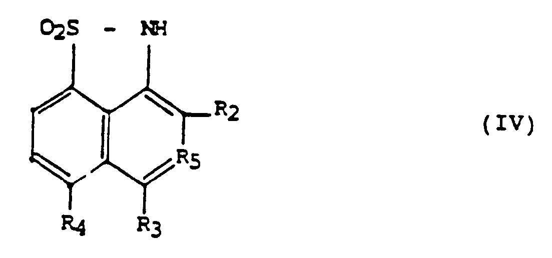 in which R₂, R₃, R₄ and R₅ have the same meanings as in claim 1, except that R₃ is not an acetylamino radical, is reacted with a derivative of formula: 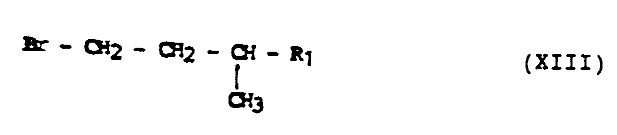 in which R₁ has the same meanings as in claim 1, with the proviso that it does not contain a hydroxyl radical, the product is isolated and is optionally converted to a salt with an inorganic or organic acid.

9. Method of preparing the compounds of formula (l) according to claim 1 for which R₁ represents a 1,2,3,6-tetrahydro-1-pyridyl radical substituted at the 4-position by a 3-indolyl radical substituted on the nitrogen atom by an alkyl or alkylcarbonyl radical or a piperidino radical substituted at the 4-position by a 2-oxo-1-benzimidazolinyl radical substituted at the 3-position by an alkylcarbonyl or benzoyl radical or a 3-indolyl radical substituted on the nitrogen atom by an alkyl or alkylcarbonyl radical, with the exception of those for which R₆ represents a propylene radical substituted at the 2-position by a hydroxyl radical, characterised in that a derivative of formula: 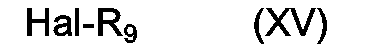 in which Hal represents a halogen atom and Rg represents an alkyl, alkylcarbonyl or benzoyl radical, is reacted with a corresponding derivative of formula (I) for which R₁ represents a 1,2,3,6-tetrahydro-t-pyridyl radical substituted at the 4-position by a 3-indolyl radical or a piperidino radical substituted at the 4- position by a 2-oxo-1-benzimidazolinyl or 3-indolyl radical, the product is isolated and is optionally converted to a salt with an inorganic or organic acid.

10. Method of preparing the compounds of formula (I) according to claim 1 for which R₁ represents a 1,2,3,6-tetrahydro-1-pyridyl radical substituted at the 4-position by a halophenyl radical, characterised in that a corresponding compound of formula (I) for which R₁ represents a piperidino radical substituted at the 4- position by a hydroxyl radical and a phenyl radical substituted by a halogen atom is dehydrated, the product is isolated and is optionally converted to a salt with an inorganic or organic acid.

11. Method of preparing the compounds of formula (I) according to claim 1 for which R₁ represents a 4-aminophenyl-1-piperazinyl radical, characterised in that a corresponding compound of formula (I) for which R₁ represents a 4-nitrophenyl-1-piperazinyl radical is reduced, the product is isolated and is optionally converted to a salt with an inorganic or organic acid.

12. Medicinal products, characterised in that they contain as active principle at least one compound according to claim 1 or a salt of this compound with a pharmaceutically acceptable acid.

13. Medicinal products, characterised in that they contain as active principle at least one compound according to claim 2 or a salt of this compound with a pharmaceutically acceptable acid.

14. 2-[3-(4-(4-Fluorophenyl)-1-piperazinyl)propyl]naphtho[1,8-cd]isothiazole-1,1-dioxide and its salts with a pharmaceutically acceptable acid.

15. Medicinal products, characterised in that they contain as active principle at least one compound according to claim 14 or a salt of this compound with a pharmaceutically acceptable acid.

## Claims (Claims for the following Contracting State(s) : ES, GR)

1. Method of preparing a compound of formula: 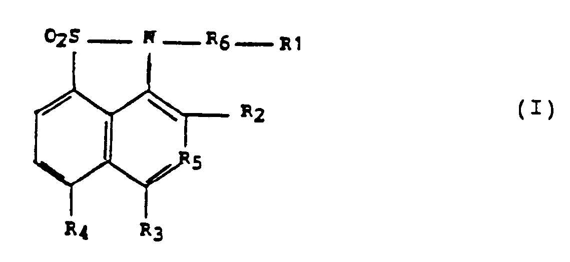 in which
- R₁ represents
. a 1,2,3,6-tetrahydro- 1-pyridyl radical substituted at the 4-position by (a) a phenyl radical, (b) a phenyl radical substituted by a halogen atom or an alkyl, hydroxyl or alkoxy radical, (c) a 3-indolyl radical, (d) a 3-indolyl radical substituted on the nitrogen atom by an alkyl or alkylcarbonyl radical and/or at the 5-position by a chlorine or fluorine atom or (e) a 3-(5-hydroxyindolyl) radical,
. a 1-piperazinyl radical substituted at the 4-position by (a) a phenyl radical, (b) a phenyl radical substituted by an alkoxy, alkyl, hydroxyl, nitro or amino radical or a halogen atom, (c) a 1,2-benzisothiazol-3-yl radical, (d) a 1,2-benzisoxazol-3-yl radical or (e) a 2-pyridyl radical,
. a piperidino radical substituted at the 4-position by (a) a phenyl radical, (b) a phenyl radical substituted by a halogen atom or a hydroxyl, alkyl or alkoxy radical, (c) two phenyl radicals, (d) a bis(4-fluorophenyl)-methylene radical, (e) a 4-fluorobenzoyl radical, (f) a 2-oxo-1-benzimidazolinyl radical, (g) a 2-oxo-1-benzimidazolinyl radical substituted at the 3-position by an alkylcarbonyl or benzoyl radical, (h) a hydroxyl radical and a phenyl radical which is optionally substituted by an alkyl, alkoxy or hydroxyl radical or a halogen atom, (i) a 3-indolyl radical, (j) a 3-indolyl radical substituted on the nitrogen atom by an alkyl or alkylcarbonyl radical and/or at the 5-position by a chlorine or bromine atom or (k) a 3-(5-hydroxyindolyl) radical,
- either:
. R₂ and R₃ are identical and represent a hydrogen or halogen atom and R₄ represents a hydrogen atom or
. R₂ and R₄ represent a hydrogen atom and R₃ represents a halogen atom oran acetylamino radical or
. R₂ and R₃ represent a hydrogen atom and R₄ represents a halogen atom and R₅ represents a group -CH=,
- or R₂, R₃ and R₄ represent a hydrogen atom and R₅ represents a nitrogen atom, and
R₆ represents an alkylene chain containing 2 to 4 carbon atoms or a propylene chain substituted at the 1-or3-position by an alkyl radical or at the 2-position by an alkyl, alkoxy, hydroxyl, dialkylamino, piperidino, morpholino or thiomorpholino radical, with the proviso that when R₆ represents a propylene radical substituted at the 2-position by a dialkylamino, piperidino, morpholino or thiomorpholino radical, R₁ cannot be a radical containing a hydroxyl radical, and it being understood that the alkyl and alkoxy radicals and the alkyl and alkoxy portions contain 1 to 4 straight-or branched-chain carbon atoms, as well as their salts with inorganic or organic acids,
characterised in that:
A - for the preparation of the compounds of formula (I) with the exception of those for which R₆ represents a propylene radical substituted at the 2-position by a dialkylamino, piperidino, morpholino or thiomorpholino radical and/or R₁ represents a 4-aminophenyl-1-piperazinyl radical, a halogenated derivative of formula: 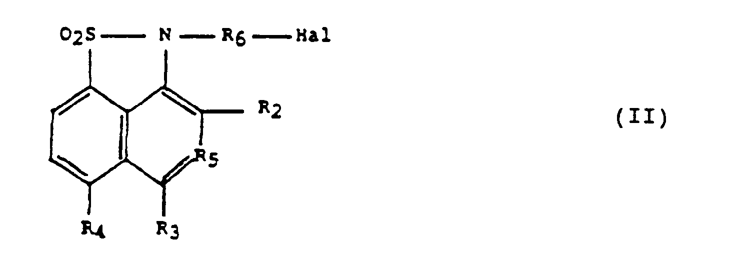 in which Hal represents a halogen atom and R₂, R₃, R₄, R₅ and R₆ have the same meanings as previously except that R₆ is not a dialkylamino, piperidino, morpholino or thiomorpholino radical, is reacted with a derivative of formula: 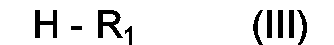 in which R₁ has the same meanings as previously with the exception of being a 4-aminophenyl-1-piperazinyl radical, the product is isolated and is optionally converted to a salt with an inorganic or organic acid,
B - for the preparation of the compounds of formula (I) for which
. either R₅ represents a group =CH-, R₂, R₃ and R₄ represent a hydrogen atom or R₂ and R₄ represent a hydrogen atom and R₃ represents a halogen atom or R₂ and R₃ represent a hydrogen atom and R₄ represents a halogen atom or R₂ and R₃ represent a halogen atom and R₄ represents a hydrogen atom,
. or R₅ represents a nitrogen atom and R₂, R₃ and R₄ represent a hydrogen atom,
R₆ represents an alkylene (2-4 C) radical or propylene radical substituted in the 2-position by a hydroxyl, alkyl or alkoxy radical, with the exception of those for which R₁ represents a 4-aminophenyl-1-piperazinyl radical, a compound of formula: 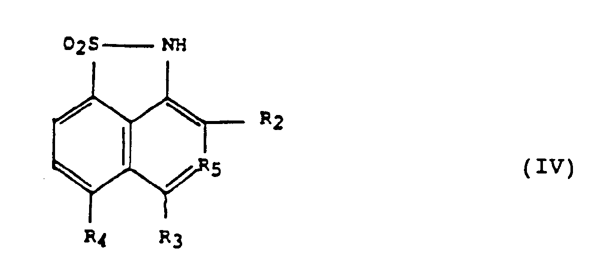 in which R₂, R₃, R₄ and R₅ have the meanings mentioned above is reacted with a derivative of formula: Hal-R₆-R₁ (X) in which R₁ and R₆ have the same meanings as above, the product is isolated and is optionally converted to a salt with an inorganic or organic acid,
C - for the preparation of compounds of formula (I) for which R₆ represents a propylene radical substituted at the 2-position by an alkoxy radical, with the exception of those forwhich R₁ represents a 1,2,3,6-tetrahydro-1-pyridyl radical substituted at the 4-position by a hydroxyphenyl radical, a 3-indolyl radical substituted at the 5-position by a hydroxyl radical, a 1-piperazinyl radical substituted at the 4-position by an amino or hydroxyphenyl radical or a piperidino radical substituted at the 4-position by a 2-oxo-1-benzimidazolinyl radical, a hydroxyphenyl radical, a hydroxyl radical and a phenyl radical optionally substituted by a hydroxyl radical or a 3-indolyl radical optionally substituted at the 5-position by a hydroxyl radical, a corresponding compound of formula (I) for which R₆ represents a propylene radical substituted at the 2-position by a hydroxyl radical is alkylated, the product is isolated and is optionally converted to a salt with an inorganic or organic acid,
D - for the preparation of the compounds of formula (I) for which R₆ represents a propylene radical substituted at the 2-position by a dialkylamino, piperidino, morpholino orthiomorpholino radical, a derivative of formula: 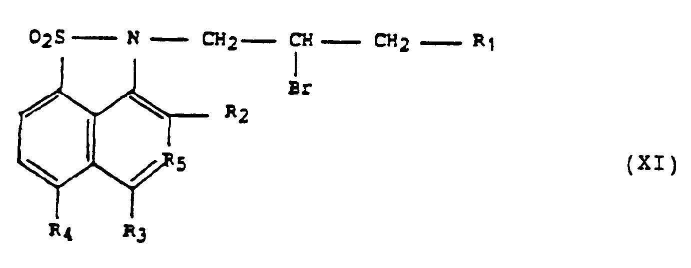 in which R₁, R₂, R₃, R₄ and R₅ have the same meanings as in formula (I) is reacted with a derivative of formula: 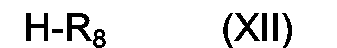 in which R₈ represents a dialkylamino, piperidino, morpholino or thiomorpholino radical, the product is isolated and is optionally converted to a salt with an inorganic or organic acid,
E - for the preparation of the compounds of formula (I) for which R₆ represents a propylene radical substituted at the 3-position by a methyl radical, with the exception of the compounds for which R₃ represents an acetylamino radical and/or R₁ represents a substituent containing a hydroxyl radical, a derivative of formula: 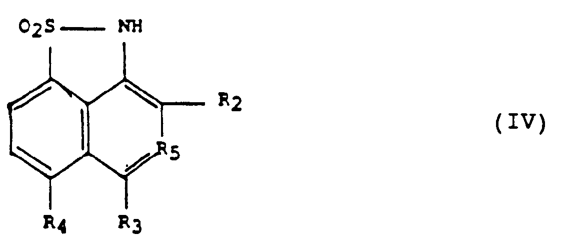 in which R₂, R₃, R₄ and R₅ have the same meanings as in formula (I), except that R₃ cannot be an acetylamino radical, is reacted with a derivative of formula: 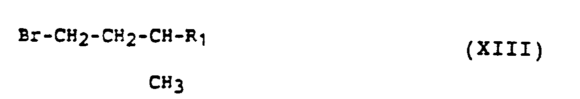 in which R₁ has the same meanings as in formula (I), with the proviso that it does not contain a hydroxyl radical, the product is isolated and is optionally converted to a salt with an inorganic or organic acid, F - for the preparation of the compounds of formula (I) for which R₁ represents a 1,2,3,6-tetrahydro-1-pyridyl radical substituted at the 4-position by a 3-indolyl radical substituted on the nitrogen atom by an alkyl or alkylcarbonyl radical or a piperidino radical substituted at the 4-position by a 2-oxo-1-benzimidazolinyl radical substituted at the 3-position by an alkylcarbonyl or benzoyl radical or a 3-indolyl radical substituted on the nitrogen atom by an alkyl or alkylcarbonyl radical, with the exception of those for which R₆ represents a propylene radical substituted at the 2-position by a hydroxyl radical, a derivative of formula: 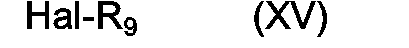 in which Hal represents a halogen atom and Rg represents an alkyl, alkylcarbonyl or benzoyl radical, is reacted with a corresponding derivative of formula (I) for which R₁ represents a 1,2,3,6-tetrahydro-1-pyridyl radical substituted at the 4-position by a 3-indolyl radical or a piperidino radical substituted at the 4-position by a 2-oxo-1-benzimidazolinyl or 3-indolyl radical, the product is isolated and is optionally converted to a salt with an inorganic or organic acid,
G - for the preparation of the compounds of formula (I) for which R₁ represents a 1,2,3,6-tetrahydro-1-pyridyl radical substituted at the 4-position by a halophenyl radical, a corresponding compound of formula (I) for which R₁ represents a piperidino radical substituted at the 4-position by a hydroxyl radical and a phenyl radical substituted by a halogen atom is dehydrated, the product is isolated and is optionally converted to a salt with an inorganic or organic acid,
H - for the preparation of the compounds of formula (I) for which R₁ represents a 4-aminophenyl-1-piperazinyl radical, a corresponding compound of formula (I) for which R₁ represents a 4-nitrophenyl-1-piperazinyl radical is reduced, the product is isolated and is optionally converted to a salt with an inorganic or organic acid.

2. Method according to claim 1, in which the halogen atoms are chlorine or bromine atoms.

3. Method according to claim 1 for the preparation of the compounds of formula (I) for which R₁ represents a 1,2,3,6-tetrahydro-l-pyridyi radical substituted at the 4-position by a halophenyl radical, a 1-piperazinyl radical substituted at the 4-position by a 2-pyridyl radical, a 1,2-benzisothiazol-3-yl radical or a phenyl radical substituted by a halogen atom or a hydroxyl, amino or alkyl radical or a piperidino radical substituted at the 4-position by a phenyl or N-alkyl-3-indolyl radical.

4. Method according to claim 1 for the preparation of 2-[3-(4-(4-fluorophenyl)-1-piperazinyl)propyl]naph- tho[1,8-cd]isothiazole-1,1-dioxide and of its salts with a pharmaceutically acceptable acid.
